(19) Europäisches Patentamt
European Patent Office
Office européen des brevets

(11) EP 0 556 119 B1

(12) **FASCICULE DE BREVET EUROPEEN**

(45) Date de publication et mention de la délivrance du brevet: **14.05.1997 Bulletin 1997/20**

(51) Int. Cl.[6]: **C07D 207/08**, C07D 207/48, C07D 401/04, C07D 401/06, C07D 401/12, A61K 31/40

(21) Numéro de dépôt: **93400340.1**

(22) Date de dépôt: **11.02.1993**

(54) **Dérivés de pyrrolidine portant en position 3 un groupe acide gras saturé ou non-saturé, procédé pour leur préparation et compositions pharmaceutiques ayant une activité d'inhibiteurs de thromboxane-A2 les contenant**

In der 3-Position durch einen gesättigten oder ungesättigten Fettsäurerest substituierte Pyrrolidinderivate, Verfahren zu ihrer Herstellung und diese enthaltende Arzneimittel mit Thromboxan-A2 inhibierender Aktivität

Pyrrolidine derivatives substituted in the 3-position with a saturated or insaturated fatty acid group, process for their preparation and pharmaceuticals with thromboxane A2 inhibiting activity containing them

(84) Etats contractants désignés:
**AT BE CH DE DK ES FR GB GR IE IT LI LU NL PT SE**

(30) Priorité: **12.02.1992 FR 9201523**

(43) Date de publication de la demande:
**18.08.1993 Bulletin 1993/33**

(73) Titulaire: **ADIR ET COMPAGNIE**
**92415 Courbevoie Cédex (FR)**

(72) Inventeurs:
- **Lavielle, Gilbert**
  **F-78170 La Celle Saint Cloud (FR)**
- **Dubuffet, Thierry**
  **F-94240 L'Hay les Roses (FR)**
- **Muller, Olivier**
  **F-95800 Emmery (FR)**
- **Laubie, Michel**
  **F-92420 Vaucresson (FR)**
- **Verbeuren, Tony**
  **F-78540 Vernouillet (FR)**

(56) Documents cités:
**EP-A- 0 289 911** **EP-A- 0 367 130**

- **JOURNAL OF MEDICINAL CHEMISTRY. vol. 34, no. 4, 1991, WASHINGTON US pages 1511 - 1514 MAIS D. E. ET. AL. 'Novel Synthesis and Biochemical Properties of an [125I]-labeled Photoaffinity Probe for Thromboxane A2/Prostaglandin H2 Receptors'**
- **JOURNAL OF MEDICINAL CHEMISTRY. vol. 34, no. 9, 1991, WASHINGTON US pages 2882 - 2891 MISRA R. N. ET. AL. 'Interphenylene 7-Oxabicyclo[2.2.1]heptane Thromboxane A2 Antagonists'**
- **JOURNAL OF MEDICINAL CHEMISTRY. vol. 34, no. 6, 1991, WASHINGTON US pages 1790 - 1797 BHAGWAT S. S. ET. AL. 'Thromboxane Receptor Antagonism Combined with Thromboxane Synthase Inhibition.'**
- **HETEROCYCLES vol. 28, no. 2, 1989, pages 573 - 578 KAWADA K. ET. AL. 'Synthesis of monocyclic analogs of a potent thromboxane receptor antagonist, (.+-.)-(5Z)-7-[3-endo-[(phenyl sulfonyl)amino]bicyclo[2.2.1]hept-2-exo-yl ]heptenoic acid.'**
- **PATENT ABSTRACTS OF JAPAN vol. 13, no. 101 (C-574)9 Mars 1989 & JP-A-63 277 655**
- **TETRAHEDRON LETTERS. vol. 49, 1976, OXFORD GB pages 4463 - 4466 K. KUEHLEIN ET. AL. 'Synthese von 10-Aza-Dihydro-A-prostaglandinen'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.14.3/3.4

EP 0 556 119 B1

## Description

La présente invention concerne des dérivés de la pyrrolidine, leur procédé de préparation et les compositions pharmaceutiques qui les contiennent.

Plus particulièrement, les composés décrits dans la présente invention possèdent des propriétés antithromboxane $A_2$ aussi bien en tant qu'antagonistes des récepteurs au thromboxane $A_2$ ($TXA_2$) qu'en tant qu'inhibiteurs de l'activité de l'enzyme responsable de la synthèse du thromboxane $A_2$ : la thromboxane $A_2$-synthase.

Le thromboxane $A_2$ est un métabolite de l'acide arachidonique produit par les plaquettes sanguines qui provoque une constriction importante des vaisseaux sanguins et induit l'agrégation des plaquettes. La production du thromboxane $A_2$ est augmentée dans des maladies comme l'angine de poitrine ou l'accident vasculaire cérébral et il joue un rôle très important dans tous les processus conduisant aux maladies thrombotiques.

Il était donc particulièrement intéressant de synthétiser des substances susceptibles d'inhiber les activités proagrégantes et vasoconstrictives du thromboxane $A_2$ soit en tant qu'antagonistes des récepteurs au thromboxane $A_2$, soit en tant qu'inhibiteur de la thromboxane $A_2$-synthase.

Des dérivés de pyrrolidine possédant des propriétés antithrombotiques ont été décrits dans la littérature. C'est le cas, en particulier, des composés décrits dans les brevets EP 289 911 et EP 367 130.

Les composés décrits dans la présente invention possèdent des propriétés pharmacologiques nettement plus intenses que celles des autres composés décrits dans l'Art Antérieur.

Ils sont donc utiles en tant qu'antagonistes au thromboxane $A_2$ et en tant qu'inhibiteurs de la thromboxane $A_2$-synthase dans le traitement ou la prévention des maladies thrombotiques comme l'accident vasculaire cérébral, l'angine de poitrine, l'infarctus du myocarde, l'insuffisance circulatoire périphérique, les maladies liées à la formation de thrombus...

Plus spécifiquement, la présente invention concerne les composés de formule (I) :

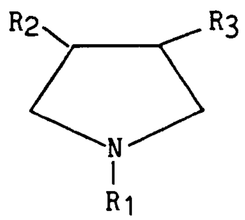

(I)

dans laquelle :

$R_1$ représente :

- un groupement alkyl ($C_1$-$C_6$) linéaire ou ramifié substitué ou non par un groupement pyridin-2-yl, pyridin-3-yl, phényl (lui-même éventuellement substitué par un ou plusieurs atomes d'halogène ou groupements alkyle ($C_1$-$C_6$) linéaire ou ramifié, alkoxy ($C_1$-$C_6$) linéaire ou ramifié, trihalogénométhyle),

- un groupement phényle substitué ou non par un ou plusieurs atomes d'halogène ou groupements alkyle ($C_1$-$C_6$) linéaire ou ramifié, alkoxy ($C_1$-$C_6$) linéaire ou ramifié, trihalogénométhyle,

- un groupement pyridyle,

- un groupement phénylsulfonyl (substitué ou non sur le noyau phényle par un ou plusieurs atomes d'halogène ou groupements alkyle ($C_1$-$C_6$) linéaire ou ramifié, alkoxy ($C_1$-$C_6$) linéaire ou ramifié, trihalogénométhyle), naphtylsulfonyl, quinolylsulfonyl ou isoquinolylsulfonyl,

- un groupement acyle ($C_1$-$C_6$) linéaire ou ramifié,

- un groupement benzoyle ou pyridylcarbonyl, substitué ou non sur le noyau aromatique par un ou plusieurs atomes d'halogène ou groupements alkyle ($C_1$-$C_6$) linéaire ou ramifié, alkoxy ($C_1$-$C_6$) linéaire ou ramifié, trihalogénométhyle,

- un groupement alkylaminocarbonyle ou phénylaminocarbonyle,

- un groupement acylamino ou benzoylamino,

$R_2$ représente :

- un atome d'hydrogène,
- un groupement phényle substitué ou non par un ou plusieurs atomes d'halogène ou groupements alkyle ($C_1$-$C_6$) linéaire ou ramifié, alkoxy ($C_1$-$C_6$) linéaire ou ramifié, hydroxy, trihalogénométhyle,
- un groupement pyridin-3-yle ou pyridin-2-yle, substitué ou non sur le noyau pyridine par un ou plusieurs atomes d'halogène ou groupements alkyle ($C_1$-$C_6$) linéaire ou ramifié, alkoxy ($C_1$-$C_6$) linéaire ou ramifié, trihalogénométhyle,

$R_3$ représente l'un quelconque des groupements suivants :

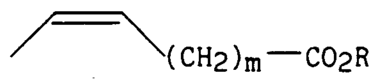

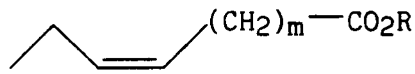

$$-(CH_2)_n-CO_2R$$

dans lesquels

m est égal à 2, 3 ou 4,
n est égal à 4, 5, 6 ou 7,
et R représente un atome d'hydrogène ou un groupement alkyle ($C_1$-$C_6$) linéaire ou ramifié,

leurs énantiomères, diastéréoisomères et épimères ainsi que leurs sels d'addition à un acide ou à une base pharmaceutiquement acceptable.

Parmi les acides pharmaceutiquement acceptables, on peut citer à titre non limitatif les acides chlorhydrique, sulfurique, tartrique, maléique, fumarique, méthane sulfonique, camphorique, etc...

Parmi les bases pharmaceutiquement acceptables, on peut citer à titre non limitatif l'hydroxyde de sodium, l'hydroxyde de potassium, la tertbutylamine, la diéthylamine, l'éthylènediamine.

L'invention s'étend également au procédé de préparation des composés de formule (I) caractérisé en ce que l'on utilise comme produit de départ une pyrrolidine de formule (II) :

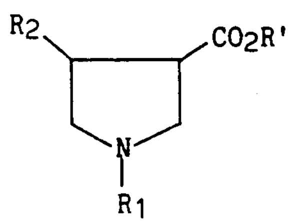

(II)

dans laquelle $R_1$ et $R_2$ ont la même signification que dans la formule (I) et R' représente un groupement alkyle ($C_1$-$C_6$) linéaire ou ramifié, composé de formule (II), sous forme racémique ou isomérique, qui, lorsqu'il est sous forme d'un couple d'énantiomères, peut être transformé, en son autre couple d'énantiomères, par épimérisation dans un alcoolate de sodium,
que l'on fait réagir :

**1** soit avec de l'hydrure de lithium aluminium dans un solvant inerte, pour conduire à la pyrrolidine de formule (III)

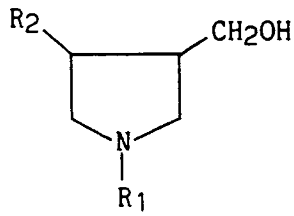

(III)

dans laquelle $R_1$ et $R_2$ ont la même signification que dans la formule (I), que l'on met en réaction :

- ou bien avec de l'acide chlorhydrique gazeux en présence de chl+orure de thionyle en milieu chloroformique, pour conduire à la pyrrolidine de formule (IV) :

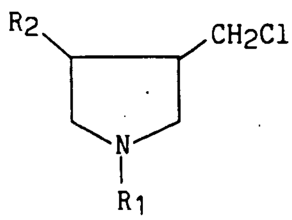

(IV)

dans laquelle $R_1$ et $R_2$ ont la même signification que dans la formule (I), que l'on transforme en pyrrolidine de formule (V) en présence de cyanure de terbutylammonium, dans du diméthylformamide :

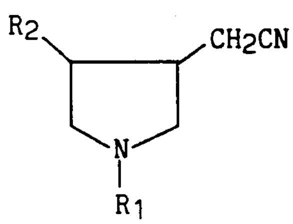

(V)

dans laquelle $R_1$ et $R_2$ ont la même signification que dans la formule (I),

- ou bien avec du chlorure de paratoluène sulfonyle dans de la pyridine pour conduire à la pyrrolidine de formule (VI) :

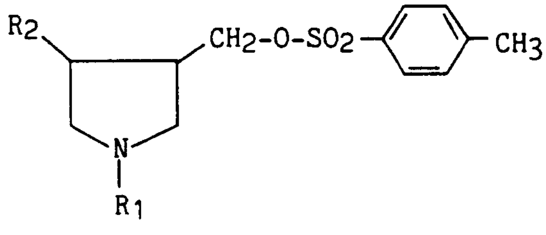

(VI)

dans laquelle $R_1$ et $R_2$ ont la même signification que dans la formule (I), que l'on transforme en pyrrolidine de formule (V) décrite précédemment, en présence de cyanure de potassium dans du diméthylsulfoxyde, dérivé de formule (V) que l'on réduit à l'aide d'hydrure de diisobutylaluminium,
pour conduire à l'aldéhyde de formule (VII) :

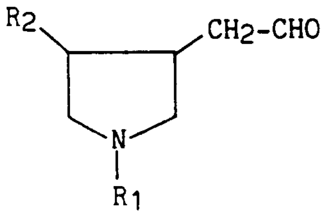

(VII)

dans lequel $R_1$ et $R_2$ ont la même signification que dans la formule (I),

**2** soit avec de l'hydrure de diisobutylaluminium, pour conduire à l'aldéhyde de formule (VIII) :

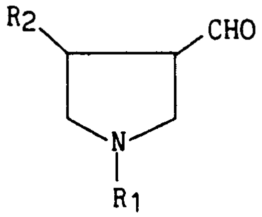

(VIII)

dans lequel $R_1$ et $R_2$ ont la même signification que dans la formule (I) qui est, si on le désire, mis en réaction avec l'ylure de phosphore de formule (IX), préparé par réaction du sel de phosphonium correspondant en présence de terbutanolate de potassium dans du tétrahydrofurane,

$$(C_6H_5)_3\ P{=}CHOCH_3 \qquad (IX)$$

pour conduire au composé de formule (X)

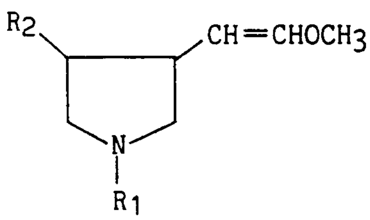

(X)

dans lequel $R_1$ et $R_2$ ont la même signification que dans la formule (I), que l'on traite par de l'acide trifluoroacétique pour conduire à l'aldéhyde de formule (VII) décrit précédemment,
composés de formule (VII) ou (VIII) que l'on fait réagir avec un ylure de phosphore de formule (XI), préparé par réaction du sel de phosphonium correspondant, en présence de terbutanolate de potassium dans du tétrahydrofurane,

$$(C_6H_5)_3\ P{=}CH{-}(CH_2)_m{-}CO_2H \qquad (XI)$$

dans laquelle m a la même signification que dans la formule (I), pour conduire respectivement aux composés de formule (I/a) ou (I/b), cas particulier des composés de formule (I) :

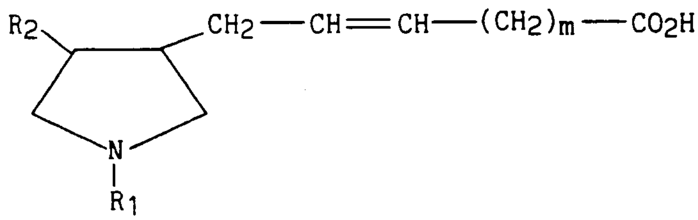

(I/a)

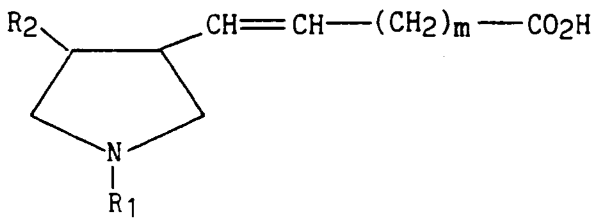

(I/b)

dans lesquelles $R_1$, $R_2$ et m ont la même signification que dans la formule (I),
composés de formule (I/a) ou (I/b)

- que l'on estérifie, si on le souhaite, pour conduire aux composés de formule (I/$a_1$) et (I/$b_1$) correspondants :

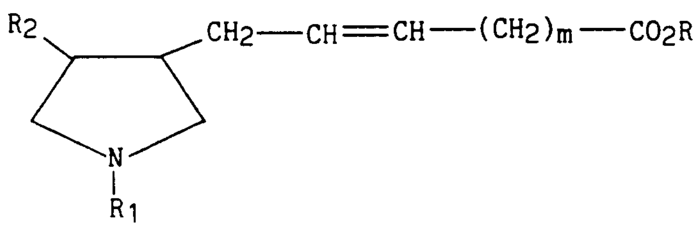

(I/$a_1$)

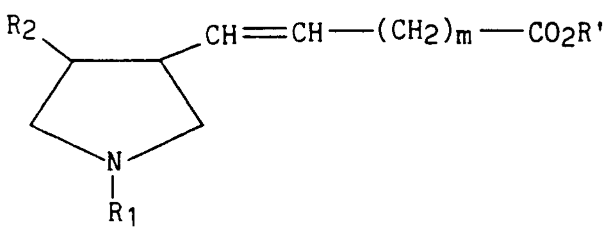

(I/$b_1$)

dans lesquelles $R_1$ et $R_2$ ont la même signification que dans la formule (I) et R' représente un groupement alkyle ($C_1$-$C_6$) linéaire ou ramifié,

- dont on réduit éventuellement la double liaison par hydrogénation catalytique pour conduire au composé de formule (I/c), cas particulier des composés de formule (I),

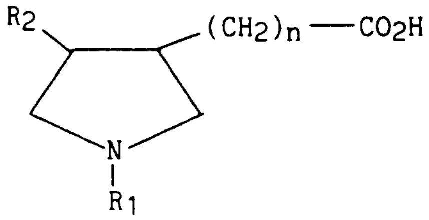

(I/c)

dans laquelle $R_1$, $R_2$ et n ont la même signification que dans la formule (I) et que l'on transforme, si on le souhaite, en ester correspondant de formule ($I/c_1$) :

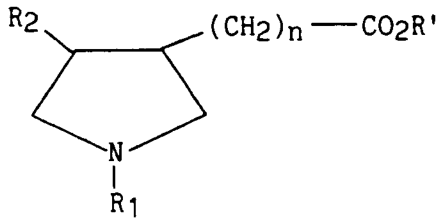

($I/c_1$)

dans laquelle $R_1$ et $R_2$ ont la même signification que dans la formule (I) et R' représente un groupement alkyle ($C_1$-$C_6$) linéaire ou ramifié,
composés de formule (I/a), ($I/a_1$), (I/b), ($I/b_1$), (I/c), ($I/c_1$) qui constituent l'ensemble des composés de formule (I),

- qui, lorsque $R_2$ représente un noyau phényle substitué par un groupement alkoxy ($C_1$-$C_6$) linéaire ou ramifié, peuvent être transformés, si on le désire, en composés de formule (I) dans lesquels $R_2$ représente un noyau phényle substitué par un groupement hydroxy,
- que l'on purifie, le cas échéant, selon une technique classique de purification,
- dont on sépare éventuellement les isomères selon une technique classique de purification,
- et que l'on transforme, si on le souhaite, en leurs sels d'addition à un acide ou à une base pharmaceutiquement acceptable.

De plus, les composés répondant plus spécifiquement à la formule (I'), cas particulier des composés de formule (I) :

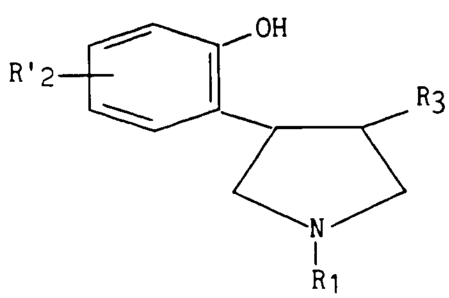

(I')

dans laquelle $R_1$ et $R_3$ ont la même signification que dans la formule (I), $R'_2$ représente un atome d'hydrogène, d'halogène ou un groupement alkyle ($C_1$-$C_6$) linéaire ou ramifié, alkoxy ($C_1$-$C_6$) linéaire ou ramifié ou trihalogénométhyle,
sont également obtenus en utilisant le procédé caractérisé en ce que l'on utilise comme produit de départ le composé de formule (XII) :

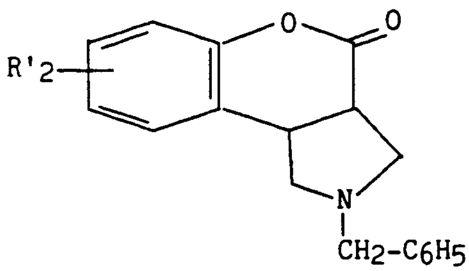

(XII)

dans laquelle R'$_2$ a la même signification que dans la formule (I'), que l'on réduit à l'aide d'hydrure de diisobutylaluminium,
pour conduire au composé de formule (XIII) :

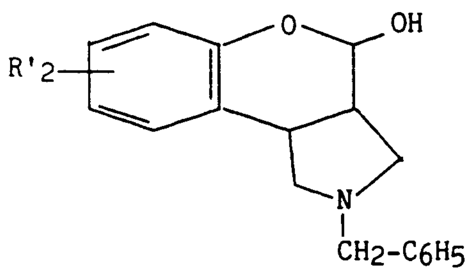

(XIII)

dans laquelle R'$_2$ a la même signification que précédemment, qui est :

a soit :

- ou bien transformé en composé de formule (XIV), par hydrogénation catalytique (Pd/c) dans du dioxane :

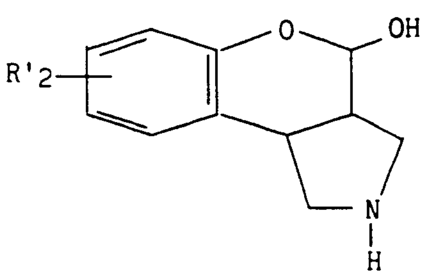

(XIV)

que l'on traite par un dérivé halogéné de formule R$_1$X dans laquelle R$_1$ a la même signification que dans la formule (I) et X représente un atome d'halogène, dans du chloroforme en présence de triéthylamine,
pour conduire au composé de formule (XV) :

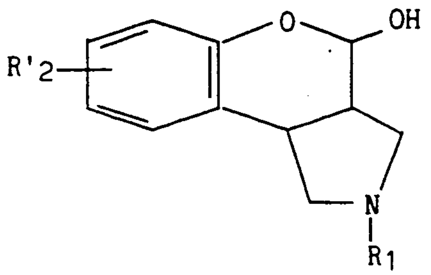

(XV)

dans laquelle R'$_2$ et R$_1$ ont la même signification que précédemment,

- ou bien transformé en composé de formule (XVI), par hydrogénation catalytique (Pd/c) dans un milieu acide chlorhydrique/éthanol :

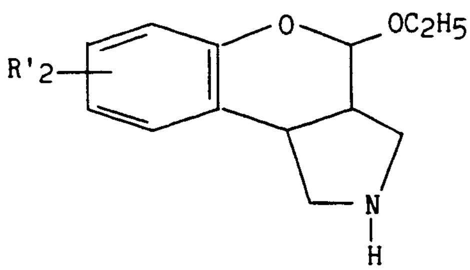

(XVI)

dans laquelle R'$_2$ a la même signification que précédemment,
que l'on traite par un dérivé halogéné de formule R$_1$X, décrit précédemment, pour conduire au composé de formule (XVII) :

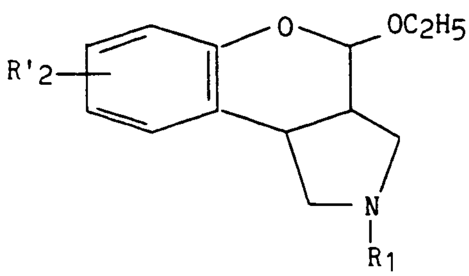

(XVII)

dans laquelle R'$_2$ et R$_1$ ont la même signification que précédemment,
qui est traité par de l'acide trifluoroacétique, pour conduire au composé de formule (XV) décrit précédemment, dérivé de formule (XV) qui est alors mis en réaction avec l'ylure de phosphore de formule (IX) préparé par réaction du sel de phosphonium correspondant,
en présence de terbutanolate de potassium dans du tétrahydrofurane,

$$(C_6H_5)_3\ P{=}CH_2OCH_3 \qquad (IX)$$

pour conduire au composé de formule (XVIII) :

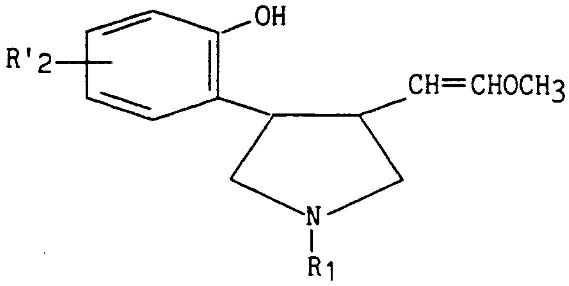

(XVIII)

dans laquelle $R_1$ et $R'_2$ ont la même signification que précédemment, dont on sépare éventuellement les diastéréoisomères selon une technique classique de séparation, que l'on traite par de l'acide trifluoroacétique pour conduire à l'aldéhyde de formule (XIX) :

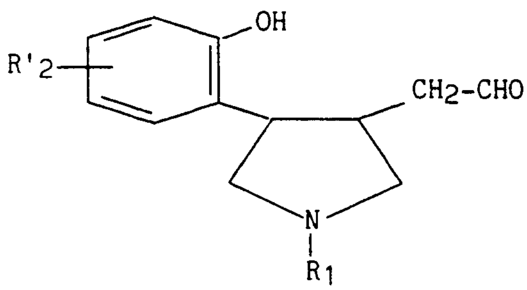

(XIX)

dans laquelle $R_1$ et $R'_2$ ont la même signification que précédemment, dont on sépare éventuellement les diastéréoisomères selon une technique classique de séparation, b soit mis en réaction avec l'ylure de phosphore de formule (IX) décrit précédemment, pour conduire au composé de formule (XX) :

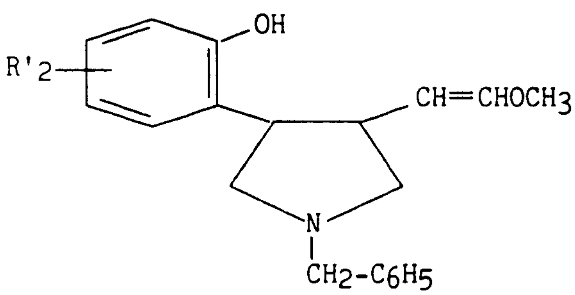

(XX)

dans laquelle $R'_2$ a la même signification que précédemment, dont on sépare éventuellement les diastéréoisomères selon une technique classique de séparation, que l'on traite par de l'acide trifluoroacétique, pour conduire à l'aldéhyde de formule (XIX/a), cas particulier des composés de formule (XIX) :

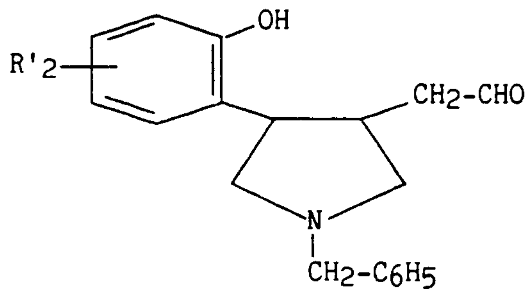

(XIX/a)

dans laquelle R'$_2$ a la même signification que précédemment,
dont on sépare éventuellement les diastéréoisomères selon une technique classique de séparation,
composés de formule (XIII) ou (XIX), que l'on fait réagir avec un ylure de phosphore de formule (X) préparé par réaction du sel de phosphonium correspondant en présence de terbutanolate de potassium dans du tétrahydrofurane,

$$(C_6H_5)_3\ P{=}CH\text{-}(CH_2)_m\text{-}CO_2H \qquad (X)$$

dans laquelle m a la même signification que dans la formule (I),
pour conduire respectivement aux composés de formules (I/d) et (I/e), cas particulier des composés de formule (I) :

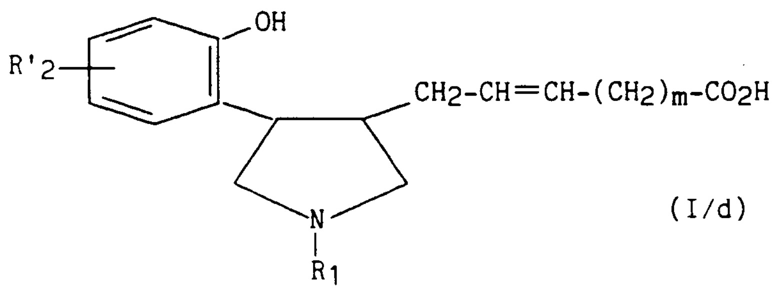

(I/d)

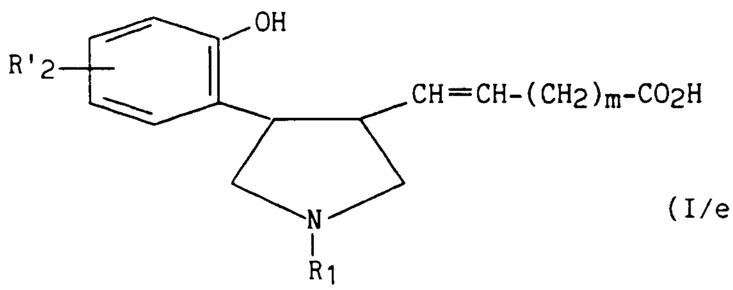

(I/e)

dans lesquelles R$_1$ et R'$_2$ ont la même signification que précédemment,

- que l'on estérifie, si on le souhaite, pour conduire aux composés de formules (I/d$_1$) et (I/e$_1$) correspondants :

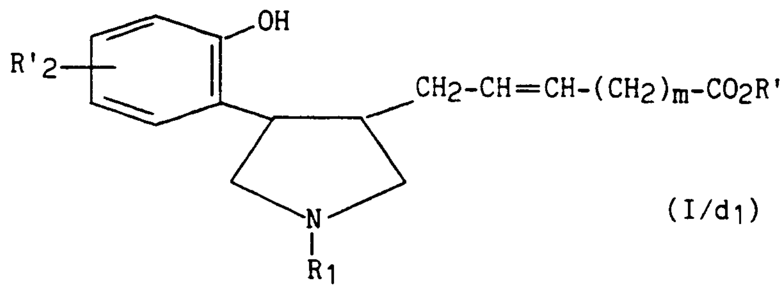

(I/d1)

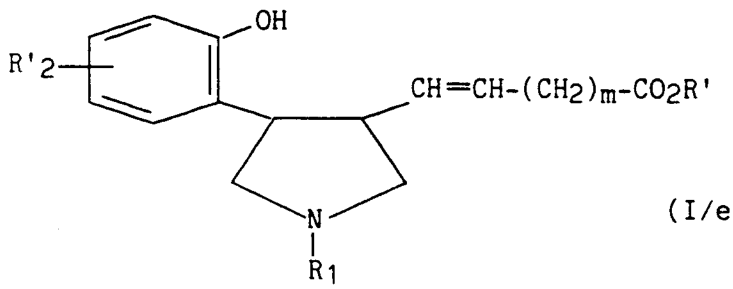

(I/e1)

dans lesquelles $R_1$ et $R'_2$ ont la même signification que précédemment et R' représente un groupement alkyle ($C_1$-$C_6$) linéaire ou ramifié,

- dont on réduit éventuellement la double liaison par hydrogénation catalytique pour conduire au composé de formule (I/f), cas particulier des composés de formule (I) :

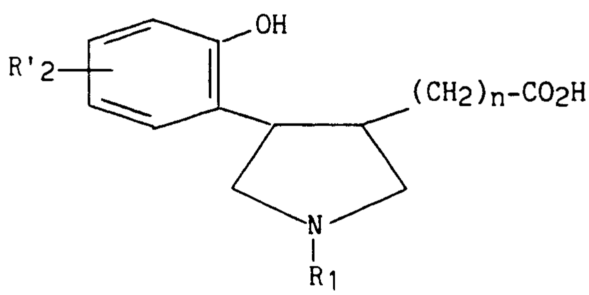

(I/f)

dans laquelle $R_1$ et $R'_2$ ont la même signification que précédemment,
et que l'on transforme, si on le souhaite, en ester correspondant de formule (I/$f_1$) :

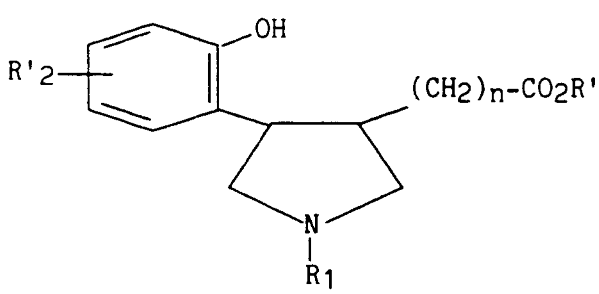

(I/f1)

dans laquelle $R_1$, $R'_2$ et R' ont la même signification que précédemment,
composés de formule (I/d), (I/$d_1$), (I/e), (I/$e_1$), (I/f) et (I/$f_1$) qui constituent l'ensemble des composés de formule (I'),

- qui, lorsque $R'_2$ représente un groupement alkoxy ($C_1$-$C_6$) linéaire ou ramifié, peuvent être transformés, si on

le désire, en composés de formule (I') dans lesquels $R'_2$ représente un groupement hydroxy,

- que l'on purifie, le cas échéant, selon une technique classique de purification,
- dont on sépare éventuellement les isomères selon une technique classique de purification,
- et que l'on transforme, si on le souhaite, en leurs sels d'addition à un acide ou à une base pharmaceutiquement acceptable.

Les composés de formule (II), utilisés comme produits de départ sont préparés selon le procédé décrit dans J. Chem. Soc., Chem. Comm., 1566, 1985 ou selon le procédé décrit dans Chem. Pharm. Bull, 33, 2762, 1985 et Organic Synthesis, 67, 133, 1988.

Les composés de formule (XII), utilisés comme produit de départ sont préparés en utilisant le procédé décrit dans Chem. Pharm. Bull, 33, 2762, 1985 et Organic Synthesis, 67, 133, 1988.

Les composés de formule (I) possèdent des propriétés pharmacologiques très intéressantes. En particulier, ils sont capables d'inhiber l'agrégation plaquétaire induite par le U46619 (9,11-didéoxy-11α,9α-époxyméthanoprostaglandine $F_{2\alpha}$), agoniste des récepteurs $TXA_2$, d'inhiber les contractions provoquées par le U46619 sur la trachée de cobaye et de prévenir in vivo les bronchoconstrictions induites par le U46619 chez le cobaye. En plus, les composés inhibent la synthèse de $TXA_2$ dans le sang du lapin.

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 10 et 200 mg pour un traitement en 1 à 3 prises par 24 heures.

Les exemples suivants illustrent l'invention.

Les produits de départ utilisés sont des produits connus ou préparés selon des modes opératoires connus.

Les lettres α et β signifient que les hydrogènes de la pyrrolidine sont en cis l'un par rapport à l'autre dans le cas de (3α,4α) et en trans l'un par rapport à l'autre dans le cas de (3α,4β).

**EXEMPLE 1 : Acide (4Z)-5-[(3α,4β)-1-Méthyl-4-(2-méthoxyphényl)pyrrolidin-3-yl]pent-4-ènoïque, sel de sodium**

Stade A : [(3α,4β)-1-Méthyl-4-(2-méthoxyphényl)pyrrolidin-3-yl] formaldéhyde

A 20,8 mmoles de [(3α,4β)-1-méthyl-4-(2-méthoxyphényl)pyrrolidin-3-yl] carboxylate de méthyle (préparé selon le procédé décrit dans J. Chem. Soc., Chem. Comm., 1566, 1985) dans 160 ml de toluène et 40 ml de pentane, sont ajoutés à -78°C, 41,6 ml d'une solution 1M d'hydrure de diisobutylaluminium dans l'hexane. Le milieu réactionnel est agité une heure à -78°C, puis traité par un mélange méthanol/eau (1/1). Après évaporation, les sels sont filtrés et lavés par de l'acétonitrile. Les filtrats sont alors évaporés. Le produit attendu est obtenu après purification sur colonne de silice en utilisant comme solvant d'élution un mélange dichlorométhane/ méthanol (90/10).

Rendement : 61 %

| Microanalyse élémentaire : | | | |
|---|---|---|---|
| | C % | H % | N % |
| calculé | 71,21 | 7,81 | 6,39 |
| trouvé | 70,94 | 7,60 | 6,22 |

Résonance magnétique nucléaire du proton ($CDCl_3$/TMS) :

La constante de couplage J entre les deux protons géminés 3α et 4β de la pyrrolidine est égale à 7,4 Hz.

Stade B : Acide (4Z)-5-[(3α,4β)-1-Méthyl-4-(2-méthoxyphényl)pyrrolidin-3-yl]pent-4-ènoïque, sel de sodium

A 5,5 mmoles de bromure de 3-carboxypropyltriphényl phosphonium dans 15 ml de tétrahydrofurane sont ajoutés lentement à température ambiante 11 ml d'une solution 1M de terbutylate de potassium dans le tétrahydrofurane. Le milieu réactionnel est agité 1 heure. 5,5 mmoles du composé obtenu au stade précédent en solution dans 30 ml de THF sont alors ajoutées et l'ensemble est agité 2 heures. Le milieu réactionnel est alors traité par une solution saturée de chlorure d'ammonium. Après évaporation, une solution aqueuse 1N d'hydroxyde de sodium est ajoutée au résidu. Cette phase aqueuse est lavée par de l'éther et acidifiée à l'aide d'acide chlorhydrique 1N. Après extraction au dichlorométhane, séchage et évaporation, le produit attendu est purifié par chromatographie sur colonne de silice en utilisant comme solvant d'élution un mélange dichlorométhane/méthanol (95/5). L'acide est alors transformé en sel de sodium correspondant.

| Microanalyse élémentaire : | | | |
|---|---|---|---|
| | C % | H % | N % |
| calculé | 65,58 | 7,12 | 4,50 |
| trouvé | 65,27 | 6,77 | 4,50 |

Les exemples 2 à 5 ont été obtenus en utilisant le même procédé que celui décrit pour l'exemple 1, en utilisant les produits de départ appropriés.

**EXEMPLE 2 : Acide (4Z)-5-[(3α,4β)-1-Méthyl-4-(4-fluorophényl)pyrrolidin-3-yl]pent-4-ènoïque, sel de sodium**

| Microanalyse élémentaire : | | | |
|---|---|---|---|
| | C % | H % | N % |
| calculé | 64,20 | 6,40 | 4,68 |
| trouvé | 64,29 | 6,16 | 4,52 |

**EXEMPLE 3 : Acide (4Z)-5-[(3α,4β)-1-Méthyl-4-phényl-pyrrolidin-3-yl]pent-4-ènoïque, sel de sodium**

| Microanalyse élémentaire : | | | |
|---|---|---|---|
| | C % | H % | N % |
| calculé | 68,31 | 7,17 | 4,98 |
| trouvé | 68,02 | 6,84 | 5,09 |

**EXEMPLE 4 : Acide (5Z)-6-[(3α,4β)-1-Méthyl-4-(4-fluorophényl)pyrrolidin-3-yl]hex-5-ènoïque, sel de sodium**

| Microanalyse élémentaire : | | | |
|---|---|---|---|
| | C % | H % | N % |
| calculé | 65,16 | 6,76 | 4,47 |
| trouvé | 65,29 | 6,90 | 4,09 |

**<u>EXEMPLE 5</u> : Acide (5Z)-6-[(3α,4β)-1-Méthyl-4-phényl-pyrrolidin-3-yl]hex-5-ènoïque, sel de sodium**

| Microanalyse élémentaire : | | | |
|---|---|---|---|
| | C % | H % | N % |
| calculé | 69,13 | 7,51 | 4,74 |
| trouvé | 68,74 | 7,10 | 4,97 |

**<u>EXEMPLE 6</u> : Acide (5Z)-7-[(3α,4α)-1-Méthyl-4-(4-fluorophényl)pyrrolidin-3-yl]hept-5-ènoïque, sel de sodium**

<u>Stade A</u> : (3α,4α)-1-Méthyl-3-hydroxyméthyl-4-(4-fluorophényl) pyrrolidine

A une suspension contenant 300 mmoles d'hydrure de lithium aluminium dans 800 ml de tétrahydrofurane et 200 ml d'éther éthylique sont ajoutées, à 10°C, 200 mmoles de [(3α,4α)-1-méthyl-4-(4-fluorophényl)pyrrolidin-3-yl] carboxylate d'éthyle (préparé selon le procédé décrit dans J. Chem. Soc., Chem. Comm., 1566, 1985) dans 100 ml de THF. L'ensemble est maintenu 30 minutes à 10°C puis traité successivement par 100 ml d'acétate d'éthyle puis 1 litre d'eau.

Après extraction par de l'éther, séchage et évaporation, le produit attendu est purifié par chromatographie sur colonne de silice en utilisant comme éluant un mélange dichlorométhane/méthanol (80/20).

<u>Rendement</u> : 90 %

| Microanalyse élémentaire : | | | |
|---|---|---|---|
| | C % | H % | N % |
| calculé | 68,87 | 7,70 | 6,69 |
| trouvé | 68,61 | 7,63 | 6,56 |

<u>Résonance magnétique nucléaire du proton</u> ($CDCl_3$/TMS) :

La constante de couplage J entre les deux protons géminés 3α et 4α de la pyrrolidine est égale à 7,5 Hz.

<u>Stade B</u> : (3α,4α)-1-Méthyl-3-chlorométhyl-4-(4-fluorophényl)pyrrolidine

Une solution contenant 95 mmoles du produit obtenu au stade précédent dans 300 ml de chloroforme est saturée par de l'acide chlorhydrique gazeux et portée 30 minutes à reflux. Après refroidissement à 30°C, une solution contenant 191 mmoles de chlorure de thionyle dans 90 ml de chloroforme est ajoutée lentement. L'ensemble est porté 3 heures à reflux. Le solvant est alors évaporé et le produit cristallise sous forme de chlorhydrate et est déplacé de son sel par de la soude et extrait au dichlorométhane.

<u>Rendement</u> : 95 % en chlorhydrate

| Microanalyse élémentaire (chlorhydrate) : | | | | |
|---|---|---|---|---|
| | C % | H % | N % | Cl % |
| calculé | 54,56 | 6,10 | 5,30 | 26,84 |
| trouvé | 54,34 | 5,91 | 5,27 | 26,60 |

Stade C : (3α,4α)-1-Méthyl-3-cyanométhyl-4-(4-fluorophényl)pyrrolidine

A 85 mmoles du produit obtenu au stade précédent dans 400 ml de diméthylformamide sont ajoutées 153 mmoles de cyanure de tétrabutylammonium. L'ensemble est porté 8 heures à 75°C puis hydrolysé. Après extraction au dichlorométhane, le produit attendu est obtenu après purification par chromatographie sur colonne de silice en utilisant comme éluant un mélange dichlorométhane/méthanol (95/5).

Rendement : 80 %

| Microanalyse élémentaire : | | | |
|---|---|---|---|
| | C % | H % | N % |
| calculé | 71,47 | 6,87 | 12,83 |
| trouvé | 71,09 | 6,82 | 12,70 |

Résonance magnétique nucléaire du proton ($CDCl_3$/TMS) :

La constante de couplage J entre les deux protons géminés 3α et 4α de la pyrrolidine est égale à 7,5 Hz.

Stade D : [(3α,4α)-1-méthyl-4-(4-fluorophényl)pyrrolidin-3-yl]acétaldéhyde

Le produit attendu est obtenu en utilisant le même procédé que celui décrit au stade A de l'exemple 1.

Rendement : 60 %

| Microanalyse élémentaire : | | | |
|---|---|---|---|
| | C % | H % | N % |
| calculé | 70,57 | 7,29 | 6,33 |
| trouvé | 70,64 | 7,48 | 6,20 |

Résonance magnétique nucléaire du proton ($CDCl_3$/TMS) :

La constante de couplage J entre les deux protons géminés 3α et 4α de la pyrrolidine est égale à 7,5 Hz.

Stade E : Acide (5Z)-7-[(3α,4α)-1-méthyl-4-(4-fluorophényl)pyrrolidin-3-yl]hept-5-ènoïque

Le produit attendu est obtenu selon le même procédé que celui décrit au stade B de l'exemple 1 en utilisant les produits correspondants.

| Microanalyse élémentaire : | | | |
|---|---|---|---|
| | C % | H % | N % |
| calculé | 70,79 | 7,92 | 4,59 |
| trouvé | 70,40 | 7,88 | 4,29 |

**EXEMPLE 6 BIS : Acide (5Z)-7-[(3α,4α)-1-Méthyl-4-(4-fluorophényl) pyrrolidin-3-yl]hept-5-ènoïque, sel de sodium**

L'exemple 6 bis est le même que l'exemple 6 mais il est obtenu en utilisant un mode opératoire différent.

Stade A : (3α,4α)-1-Méthyl-3-hydroxyméthyl-4-(4-fluorophényl)pyrrolidine

Ce stade est identique au stade A de l'exemple 6.

Stade B : (3α,4α)-1-Méthyl-3-(paratoluène-sulfonyloxy)méthyl-4-(4-fluorophényl)pyrrolidine

A 95 mmoles du composé obtenu au stade précédent dans 300 ml de pyridine sont ajoutées à +10°C 104 mmoles de chlorure de paratoluène-sulfonyle. L'ensemble est agité une nuit à température ambiante puis hydrolysé par 1,2 l d'eau glacée. Après extraction au dichlorométhane, séchage et évaporation, le produit attendu est recristallisé dans un mélange éther isopropylique/éther éthylique.

Rendement : 75 %

| Microanalyse élémentaire : | | | |
|---|---|---|---|
| | C % | H % | N % |
| calculé | 62,74 | 6,05 | 3,85 |
| trouvé | 62,41 | 5,97 | 3,72 |

Stade C : (3α,4α)-1-Méthyl-3-cyanométhyl-4-(4-fluorophényl)pyrrolidine

Dans 20 ml de diméthyl sulfoxyde sont ajoutées 13 mmoles du produit obtenu au stade précédent et 13 mmoles de cyanure de potassium. L'ensemble est chauffé à 110°C pendant 15 heures. Après évaporation, le résidu est repris par de l'eau et extrait au dichlorométhane. Le produit attendu est alors purifié par chromatographie sur gel de silice en utilisant comme éluant un mélange dichlorométhane/méthanol (95/5).

Stades D et E :

Ces stades sont identiques aux stades D et E de l'exemple 6 et conduisent aux mêmes composés.

Les exemples 7 à 12 ont été obtenus selon le procédé décrit dans les exemples 6 ou 6 bis en utilisant les produits de départ correspondants.

**EXEMPLE 7 : Acide (4Z)-6-[(3α,4α)-1-Méthyl-4-(4-fluorophényl)pyrrolidin-3-yl]hex-4-ènoïque**

| Microanalyse élémentaire : | | | |
|---|---|---|---|
| | C % | H % | N % |
| calculé | 70,08 | 7,61 | 4,81 |
| trouvé | 69,87 | 8,12 | 5,03 |

**EXEMPLE 8 : Acide (4Z)-6-[(3α,4α)-1-Méthyl-4-(2-méthoxyphényl)pyrrolidin-3-yl]hex-4-ènoïque**

**EXEMPLE 9 : Acide (4Z)-6-[(3α,4α)-1-Benzyl-4-(2-méthoxyphényl)pyrrolidin-3-yl]hex-4-ènoïque**

**EXEMPLE 10 : Acide (4Z)-6-[(3α,4β)-1-Méthyl-4-phényl-pyrrolidin-3-yl]hex-4-ènoïque, sel de sodium**

| Microanalyse élémentaire : | | | |
|---|---|---|---|
| | C % | H % | N % |
| calculé | 67,02 | 7,56 | 4,59 |
| trouvé | 66,88 | 6,88 | 4,50 |

**EXEMPLE 11 : Acide (5Z)-7-[(3α,4β)-1-Méthyl-4-(4-fluorophényl)pyrrolidin-3-yl]hept-5-ènoïque**

| Microanalyse élémentaire : | | | |
|---|---|---|---|
| | C % | H % | N % |
| calculé | 70,79 | 7,92 | 4,59 |
| trouvé | 70,40 | 7,88 | 4,29 |

**EXEMPLE 12 : Acide (4Z)-6-[(3α,4β)-1-Méthyl-4-(2-méthoxyphényl)pyrrolidin-3-yl]hex-4-ènoïque**

**EXEMPLE 13 : Acide (5Z)-6-[(3α,4α)-1-Benzyl-4-(2-hydroxyphényl)pyrrolidin-3-yl]hex-5-ènoïque**

Ce composé est obtenu en utilisant le même procédé que celui décrit pour l'exemple 1.

| Microanalyse élémentaire : | | | |
|---|---|---|---|
| | C % | H % | N % |
| calculé | 75,59 | 7,45 | 3,83 |
| trouvé | 75,22 | 7,19 | 3,72 |

**EXEMPLE 14 : Acide (5Z)-7-[(3α,4α)-1-Benzyl-4-(2-hydroxyphényl)pyrrolidin-3-yl]hept-5-ènoïque**

Stade A : (3aα,9bα)-2-Benzyl-4-hydroxy-1,2,3,3a,4,9b-hexahydrobenzopyrano[3,4-c]pyrrole

A une solution contenant 100 mmoles de 2-benzyl-4-oxoperhydrobenzopyrano[3,4-c]pyrrolidine (préparé selon le procédé décrit dans Chem. Pharm. Bull., 33, 2762, 1985 et Organic Synthesis, 67, 133, 1988) dans 800 ml de toluène sont ajoutées, à -75°C, 200 mmoles d'hydrure de diisobutylaluminium. L'ensemble est maintenu 45 minutes à -75°C puis traité pendant 30 minutes par une solution contenant 75 ml d'isopropanol et 225 ml de toluène. On ajoute alors 300 ml d'eau et l'agitation est maintenue 15 heures. Le milieu biphasique est décanté et filtré. Le produit attendu est extrait du brut par cristallisation dans de l'éther éthylique.

Rendement : 65 %
Point de fusion : 138°C

| Microanalyse élémentaire : | | | |
|---|---|---|---|
| | C % | H % | N % |
| calculé | 76,84 | 6,81 | 4,98 |
| trouvé | 76,78 | 6,95 | 5,06 |

Stade B : (3α,4α)-1-Benzyl-3-méthoxyvinyl-4-(2-hydroxyphényl) pyrrolidine

A 49,6 mmoles de chlorure de méthoxyméthyltriphényl phosphonium dans 160 ml de tétrahydrofurane sont additionnées lentement 49,6 ml d'une solution 1M de tertiobutylate de potassium. Après 1 heure d'agitation à température ambiante, 16,5 mmoles du produit obtenu au stade précédent sont ajoutées. L'ensemble est abandonné 2 heures à température ambiante et 2 heures à +40°C puis traité par une solution saturée de chlorure d'ammonium. Après extraction à l'éther, séchage et évaporation, le produit attendu est purifié par chromatographie sur gel de silice en utilisant comme solvant d'élution le dichlorométhane.

Rendement : 62 %

Stade C : [(3α,4α)-1-Benzyl-4-(2-hydroxyphényl)pyrrolidin-3-yl] acétaldéhyde

A 20,4 mmoles du composé obtenu au stade précédent sont ajoutés 6 ml d'eau, 12 ml d'acétonitrile et 6 ml d'acide trifluoroacétique. Après un reflux de 2 heures, les solvants sont évaporés. On additionne 5 ml d'eau au résidu puis de la soude jusqu'à pH 10 et l'ensemble est maintenu 30 minutes sous agitation. Après extraction au dichlorométhane, séchage et évaporation, le produit attendu est obtenu après chromatographie sur gel de silice en utilisant comme solvant d'élution un mélange dichlorométhane/méthanol (98/2).

Rendement : 62 %

Stade D : Acide (5Z)-7-[(3α,4α)-1-Benzyl-4-(2-hydroxyphényl)pyrrolidin-3-yl]hept-5-ènoïque

Le produit attendu est obtenu en utilisant le même procédé que celui décrit au stade B de l'exemple 1.

| Microanalyse élémentaire : | | | |
|---|---|---|---|
| | C % | H % | N % |
| calculé | 75,96 | 7,70 | 3,69 |
| trouvé | 75,49 | 7,54 | 3,49 |

**EXEMPLE 15 : Acide (4Z)-6-[(3α,4α)-1-Benzyl-4-(2-hydroxyphényl)pyrrolidin-3-yl]hex-4-ènoïque**

Ce composé est obtenu selon le même procédé que celui décrit dans l'exemple 14.

| Microanalyse élémentaire : | | | |
|---|---|---|---|
| | C % | H % | N % |
| calculé | 75,59 | 7,45 | 3,83 |
| trouvé | 75,24 | 7,43 | 4,02 |

**EXEMPLE 16 : Acide (4Z)-6-[(3α,4α)-1-nicotinoyl-4-(2-hydroxyphényl) pyrrolidin-3-yl]hex-4-ènoïque**

Stade A : (3aα,9bα)-2-Benzyl-4-hydroxy-1,2,3,3a,4,9b-hexahydrobenzyopyrano[3,4-c]pyrrole

Ce stade est identique au stade A de l'exemple 14.

Stade B : (3aα,9bα)-4-hydroxy-1,2,3,3a,4,9b-hexahydro-benzopyrano [3,4-c]pyrrole, chlorhydrate

La débenzylation du composé obtenu au stade précédent est réalisée dans le dioxane, à 60°C et pression atmosphérique, en présence d'acide chlorhydrique gazeux et de 10 % en masse de palladium sur charbon à 10 %. Le catalyseur est alors filtré et lavé par du diméthylformamide. Le filtrat est évaporé. Le produit attendu est obtenu par transformation de l'huile ainsi obtenue en chlorhydrate dans du dioxane.

Rendement : 90 %
Point de fusion : 168°C

Stade C : (3aα,9bα)-2-Nicotinoyl-4-hydroxy-1,2,3,3a,4,9b-hexahydrobenzopyrano[3,4-c]pyrrole

A 176 mmoles de chlorure de l'acide nicotinique en solution dans le chloroforme à 0°C, on ajoute 176 mmoles du composé obtenu au stade précédent en solution dans le chloroforme en présence de 352 mmoles de triéthylamine. Après 3 heures d'agitation à température ambiante, 200 ml d'eau sont ajoutés. La phase chloroformique est décantée, lavée à l'eau acidulée, séchée et évaporée. Le produit attendu est obtenu après purification de l'huile par chromatographie sur silice en utilisant comme éluant l'éther éthylique.

Rendement : 85 %

Stade D : (3α,4α)-1-Nicotinoyl-3-méthoxyvinyl-4-(2-hydroxyphényl) pyrrolidine

Le produit attendu est obtenu selon le même procédé que celui décrit au stade B de l'exemple 14.

Rendement : 65 %

Stade E : [(3α,4α)-1-Nicotinoyl-4-(2-hydroxyphényl)pyrrolidinyl-3-yl]acétaldéhyde

Le produit attendu est obtenu selon le même procédé que celui décrit au stade C de l'exemple 14.

Rendement : 80 %

Stade F : Acide (4Z)-6-[(3α,4α)-1-nicotinoyl-4-(2-hydroxyphényl) pyrrolidin-3-yl]hex-4-ènoïque

Le produit attendu est obtenu selon le même procédé que celui décrit au stade B de l'exemple 1.

| Microanalyse élémentaire : | | | |
|---|---|---|---|
| | C % | H % | N % |
| calculé | 69,47 | 6,31 | 7,37 |
| trouvé | 69,64 | 6,46 | 7,15 |

**<u>EXEMPLES 17 ET 18</u> :**

**<u>EXEMPLE 17</u> : Acide (4Z)-6-[(3α,4α)-1-(4-chlorophénylsulfonyl)-4-(2-hydroxyphényl)pyrrolidin-3-yl]hex-4-ènoïque**

<u>Stade A</u> : (3aα,9bα)-2-Benzyl-4-hydroxy-1,2,3,3a,4,9b-hexahydro-benzopyrano[3,4-c]pyrrole

Ce stade est identique au stade A de l'exemple 14.

<u>Stade B</u> : (3aα,9bα)-4-Ethoxy-1,2,3,3a,4,9b-hexahydro-benzopyrano [3,4-c]pyrrole, chlorhydrate

Ce stade est identique au stade B de l'exemple 16 mais est effectué dans l'éthanol et la précipitation du chlorhydrate est réalisée en milieu dioxane/éther éthylique.

<u>Rendement</u> : 95 %

<u>Stade C</u> : (3aα,9bα)-4-Chlorophénylsulfonyl-4-éthoxy-1,2,3,3a,4,9b-benzopyrano[3,4-c]pyrrole

Le produit attendu est obtenu en utilisant le même procédé que celui décrit au stade C de l'exemple 16.

<u>Rendement</u> : 85 %

<u>Stade D</u> : (3aα,9bα)-2-(4-Chlorophénylsulfonyl)-4-hydroxy-1,2,3,3a,4,9b-benzopyrano[3,4-c]pyrrole

45 mmoles du produit obtenu au stade précédent sont hydrolysées dans 40 ml d'eau en présence de 40 ml d'acide trifluoroacétique et 70 ml d'acétonitrile au reflux pendant 5 heures.

<u>Rendement</u> : 90 %
<u>Point de fusion</u> : 156-158°C

<u>Stade E</u> : (3α,4α)-1-(4-Chlorophénylsulfonyl)-3-méthoxyvinyl-4-(2-hydroxyphényl)pyrrolidine et (3α,4β)-1-(4-chlorophénylsulfonyl)-3-méthoxyvinyl-4-(2-hydroxyphényl)pyrrolidine

Les produits attendus sont obtenus sous forme de mélange de diastéréoisomères en utilisant le même procédé que celui décrit au stade B de l'exemple 14.

<u>Rendement</u> : 90 %

<u>Stade F</u> : [(3α,4α)-1-(4-Chlorophénylsulfonyl)-4-(2-hydroxyphényl) pyrrolidin-3-yl]acétaldéhyde et [(3α,4β)-1-(4-chlorophénylsulfonyl)-4-(2-hydroxyphényl) pyrrolidin-3-yl]acétaldéhyde

Les produits attendus sont obtenus en utilisant le même procédé que celui décrit au stade C de l'exemple 14, en partant du mélange précédent.

<u>Rendement</u> : 90 %

Stade G : Acide (4Z)-6-[(3α,4α)-1-(4-chlorophénylsulfonyl)-4-(2-hydroxyphényl)pyrrolidin-3-yl]hex-4-ènoïque

Le produit attendu est obtenu en utilisant le même procédé que celui décrit au stade B de l'exemple 1, suivi d'une séparation HPLC dans les conditions suivantes : colonne : Lichrosorb RP18 ; éluant d'élution : méthanol/eau/acide perchlorique : 55/45/0,05.

Rendement : 35 %

| Microanalyse élémentaire : | | | | | |
|---|---|---|---|---|---|
| | C % | H % | N % | Cl % | S % |
| calculé | 58,73 | 5,38 | 3,11 | 7,88 | 7,13 |
| trouvé | 58,62 | 5,82 | 3,16 | 8,11 | 6,85 |

**EXEMPLE 18 : Acide (4Z)-6-[(3α,4β)-1-(4-chlorophénylsulfonyl)-4-(2-hydroxyphényl)pyrrolidin-3-yl]hex-4-ènoïque**

Le produit attendu est obtenu lors de la séparation par HPLC décrite au stade G de l'exemple 17.

**EXEMPLES 19 ET 20 :**

**EXEMPLE 19 : Acide (4Z)-6-[(3α,4α)-1-isobutyroyl-4-(2-hydroxyphényl) pyrrolidin-3-yl)]hex-4-ènoïque**

Stade A : (3aα,9bα)-2-benzyl-4-hydroxy-1,2,3,3a,4,9b-hexahydrobenzopyrano[3,4-c]pyrrole

Le produit attendu est obtenu selon le même procédé que celui décrit au stade A de l'exemple 14.

Stade B : (3aα,9bα)-4-hydroxy-1,2,3,3a,4,9b-hexahydrobenzopyrano [3,4-c]pyrrole, chlorhydrate

Le produit attendu est obtenu selon le même procédé que celui décrit au stade B de l'exemple 16.

Stade C : (3aα,9bα)-2-isobutyroyl-4-hydroxy-1,2,3,3a,4,9b-hexahydrobenzopyrano[3,4-c]pyrrole

Le produit attendu est obtenu selon le même procédé que celui décrit au stade C de l'exemple 16, en remplaçant le chlorure de l'acide nicotinique par le chlorure de l'acide isobutyrique.

Rendement : 90 %

Stade D : (3α,4α)-1-isobutyroyl-3-méthoxyvinyl-4-(2-hydroxyphényl) pyrrolidine et (3α,4β)-1-isobutyroyl-3-méthoxyvinyl-4-(2-hydroxyphényl)pyrrolidine

Les produits attendus sont obtenus sous forme de mélange de diastéréoisomères en utilisant le même procédé que celui décrit au stade B de l'exemple 14.

Rendement : 75 %

Stade E : [(3α,4α)-1-isobutyroyl-4-(2-hydroxyphényl)pyrrolidinyl-3-yl] acétaldéhyde et [(3α,4β)-1-isobutyroyl-4-(2-hydroxyphényl) pyrrolidinyl-3-yl]acétaldéhyde

Les produits attendus sont obtenus en utilisant le même procédé que celui décrit au stade C de l'exemple 14, en partant du mélange précédent.

Rendement : 85 %

Stade F : Acide (4Z)-6-[(3α,4α)-1-isobutyroyl-4-(2-hydroxyphényl) pyrrolidin-3-yl)]hex-4-ènoïque

Le produit attendu est obtenu selon le même procédé que celui décrit au stade B de l'exemple 1 et est purifié par HPLC (phase inverse C18) en utilisant comme éluant un mélange méthanol/eau/acide perchlorique (55/45/0,05).

| Microanalyse élémentaire : | | | |
|---|---|---|---|
| | C % | H % | N % |
| calculé | 69,54 | 7,88 | 4,05 |
| trouvé | 69,01 | 7,55 | 3,94 |

**EXEMPLE 20 : Acide (4Z)-6-[(3α,4β)-1-isobutyroyl-4-(2-hydroxyphényl) pyrrolidin-3-yl]hex-4-ènoïque**

Le produit attendu est obtenu lors de la séparation par HPLC décrite au stade F de l'exemple 19.

Résonance magnétique nucléaire du proton (pyridine/TMS) :

La constante de couplage J entre les deux protons éthylèniques est égale à 10,8 Hz.

**EXEMPLE 21 : Acide (4Z)-6-[(3α,4α)-4-(2-hydroxyphényl)-1-[(pyridin-3-yl) méthyl]pyrrolidin-3-yl]hex-4-ènoïque**

Stade A : (3aα,4bα)-2-[(pyridin-3-yl)méthyl]4-oxoperhydrobenzopyrano [3,4-c]pyrrolidine

Ce composé est préparé selon le procédé décrit dans Chem. Pharm. Bull., 33, 2762, 1985 et Organic Synthesis, 67, 133, 1988 en remplaçant la benzylamine par de la 3-aminométhylpyridine.

Rendement : 65 %
Point de fusion (base) : 70-72°C

| Microanalyse élémentaire : | | | |
|---|---|---|---|
| | C % | H % | N % |
| calculé | 72,84 | 5,75 | 9,99 |
| trouvé | 72,10 | 5,49 | 10,03 |

Stade B : (3aα,9bα)-2-[(pyridin-3-yl)méthyl]4-hydroxy-1,2,3,3a,4,9b-hexahydrobenzopyrano[3,4-c]pyrrole

Le produit attendu est obtenu selon le même procédé que celui décrit au stade A de l'exemple 14.

Rendement : 80 %

| Microanalyse élémentaire : | | | |
|---|---|---|---|
| | C % | H % | N % |
| calculé | 72,32 | 6,43 | 9,92 |
| trouvé | 72,91 | 6,81 | 9,56 |

Stade C : (3α,4α)-1-(pyridin-3-yl)méthyl-3-méthoxyvinyl-4-(2-hydroxyphényl)pyrrolidine

Le produit attendu est obtenu selon le même procédé que celui décrit au stade B de l'exemple 14.

Rendement : 70 %

Stade D : [(3α,4α)-1-(pyridin-3-yl)méthyl-4-(2-hydroxyphényl) pyrrolidinyl-3-yl]acétaldéhyde

Le produit attendu est obtenu selon le même procédé que celui décrit au stade C de l'exemple 14.

Rendement : 45 %

Stade E : Acide (4Z)-6-[(3α,4β)-4-(2-hydroxyphényl-1-[(pyridin-3-yl) méthyl]pyrrolidin-3-yl]hex-4-ènoïque

Le produit attendu est obtenu selon le même procédé que celui décrit au stade B de l'exemple 1.

Rendement : 40 %

| Microanalyse élémentaire : | | | |
|---|---|---|---|
| | C % | H % | N % |
| calculé | 72,11 | 7,15 | 7,64 |
| trouvé | 72,28 | 6,83 | 7,84 |

**EXEMPLE 22 : Acide (4Z)-6-[(3α,4α)-1-phénylaminocarbonyl-4-(2-hydroxyphényl)pyrrolidin-3-yl]hex-4-ènoïque**

Stade A : (3aα,9bβ)-2-benzyl-4-hydroxy-1,2,3,3a,4,9b-hexahydrobenzo-pyrano[3,4-c]pyrrole

Le produit attendu est obtenu selon le même procédé que celui décrit au stade A de l'exemple 14.

Stade B : (3aα,9bβ)-4-hydroxy-1,2,3,3a,4,9b-hexahydrobenzopyrano[3,4-c] pyrrole, chlorhydrate

Le produit attendu est obtenu selon le même procédé que celui décrit au stade B de l'exemple 16.

Stade C : (3aα,9bα)-4-phénylaminocarbonyl-4-éthoxy-1,2,3,3a,4,9b-hexahydrobenzopyrano[3,4-c]pyrrole

Le produit attendu est obtenu en utilisant le même procédé que celui décrit au stade C de l'exemple 16.

Rendement : 60 %

Stade D : (3aα,9bα)-4-phénylaminocarbonyl-4-hydroxy-1,2,3,3a,4,9b-hexahydrobenzopyrano[3,4-c]pyrrole

Le produit attendu est obtenu en utilisant le même procédé que celui décrit au stade D de l'exemple 17.

Rendement : 60 %
Point de fusion : 238-240°C

Stade E : (3α,4α)-1-phénylaminocarbonyl-3-méthoxyvinyl-4-(2-hydroxyphényl)pyrrolidine

Le produit attendu est obtenu en utilisant le même procédé que celui décrit au stade B de l'exemple 14.

Rendement : 75 %

Stade F : [(3α,4α)-1-phénylaminocarbonyl-4-(2-hydroxyphényl) pyrrolidinyl-3-yl]acétaldéhyde

Le produit attendu est obtenu en utilisant le même procédé que celui décrit au stade C de l'exemple 14.

Rendement : 95 %

Stade G : Acide (4Z)-6-[(3α,4α)-1-phénylaminocarbonyl-4-(2-hydroxyphényl)pyrrolidin-3-yl]hex-4-ènoïque

Le produit attendu est obtenu en utilisant le même procédé que celui décrit au stade B de l'exemple 1.

Rendement : 55 %

| Microanalyse élémentaire : | | | |
|---|---|---|---|
| | C % | H % | N % |
| calculé | 69,85 | 6,88 | 7,08 |
| trouvé | 69,75 | 6,89 | 7,10 |

**EXEMPLE 23 : Acide (4Z)-6-[(3α,4α)-1-heptanoyl-4-(2-hydroxyphényl) pyrrolidin-3-yl)]hex-4-ènoïque**

Ce composé est obtenu selon le même procédé que celui décrit dans l'exemple 16.

Rendement : 36 %

**EXEMPLES 24 ET 25 :**

**EXEMPLE 24 : Acide (4Z)-6-[(3α,4α)-1-[(isoquinol-5-yl)sulfonyl]-4-(2-hydroxyphényl)pyrrolidin-3-yl]hex-4-ènoïque**

Ce composé est obtenu selon le même procédé que celui décrit dans l'exemple 19.

Rendement : 25 %

| Microanalyse élémentaire : | | | | |
|---|---|---|---|---|
| | C % | H % | N % | S % |
| calculé | 64,36 | 5,62 | 6,00 | 6,87 |
| trouvé | 64,11 | 5,54 | 6,11 | 6,75 |

**EXEMPLE 25 : Acide (4Z)-6-[(3α,4β)-1-[(isoquinol-5-yl)sulfonyl]-4-(2-hydroxyphényl)pyrrolidin-3-yl]hex-4-ènoïque**

Le produit attendu est obtenu lors de la séparation par HPLC du composé de l'exemple 24.

Rendement : 20 %

**<u>EXEMPLE 26</u> : Acide 4Z-6-[1-(4-chlorophénylsulfonyl)pyrrolidin-3-yl] hex-4-ènoïque**

<u>Stade A</u> : Chlorhydrate de 3-cyanométhyl pyrrolidine

La débenzylation catalytique en présence d'hydrogène et de charbon palladié du chlorhydrate de la 3-cyanométhyl 1-benzyl pyrrolidine, dans l'éthanol, conduit à l'obtention du composé désiré.

<u>Rendement</u> : 95 %

<u>Stade B</u> : 3-cyanométhyl 1-(4-chlorophénylsulfonyl) pyrrolidine

L'acylation dans le chloroforme de 36,6 g (0,25 mole) de chlorhydrate de 3-cyanométhyl pyrrolidine par 52,7 g (0,25 mole) de chlorure de l'acide parachlorophényl sulfonique en présence de 50,5 g (0,5 mole) de triéthylamine conduit à l'obtention de 72 g d'un solide qui est purifié par chromatographie sur silice Merck 70-230 mesh en utilisant comme éluant un mélange dichlorométhane-méthanol 99/1 v/v.

<u>Rendement</u> : 65 %

<u>Stade C</u> : [1-(4-chlorophénylsulfonyl)pyrrolidin-3-yl]acétaldéhyde

10 g (0,035 mole) du composé obtenu au stade précédent sont mis en solution dans 150 ml de dichlorométhane refroidit à -60°C. Additionner à cette solution 159 ml d'hydrure de diisobutylaluminium 1M dans l'hexane, puis hydrolyser 2 heures plus tard par 67 ml de méthanol, 1 l d'éther éthylique, 67 ml de solution saturée de chlorure de sodium et 48 g de sulfate de sodium. Après filtration et concentration, on obtient 10 g de l'aldéhyde désire.

<u>Rendement</u> : 100 %

<u>Stade D</u> : Acide (R,S)-4Z-6-[1-(4-chlorophénylsulfonyl)pyrrolidin-3-yl] hex-4-ènoïque

Le produit attendu est obtenu selon le même procédé que celui décrit au stade B de l'exemple 1 mais en utilisant 2 moles de bromure de 3-carboxypropyltriphényl phosphonium et 4 moles de terbutylate de potassium pour 1 mole du composé du stade précédent.

| Microanalyse élémentaire : | | | | | |
|---|---|---|---|---|---|
| | C % | H % | N % | Cl % | S % |
| calculé | 53,70 | 5,63 | 3,91 | 9,91 | 8,96 |
| trouvé | 53,99 | 5,74 | 4,22 | 9,62 | 9,01 |

**<u>EXEMPLE 27</u> : Acide (4Z)-6-[(3α,4α)-1-[(quinol-8-yl)sulfonyl]-4-(2-hydroxyphényl)pyrrolidin-3-yl]hex-4-ènoïque**

Ce composé est obtenu selon les mêmes procédés que ceux décrits dans l'exemple 28.

<u>Rendement</u> : 30 %

| Microanalyse élémentaire : | | | | |
|---|---|---|---|---|
| | C % | H % | N % | S % |
| calculé | 64,36 | 5,62 | 6,00 | 6,87 |
| trovué | 64,22 | 5,88 | 6,12 | 6,62 |

**EXEMPLE 28 : Acide (4Z)-6-[(3α,4α)-1-(napht-1-yl-sulfonyl)-4-(2-hydroxyphényl)pyrrolidin-3-yl)]hex-4-ènoïque**

Stade A : (3α,4α)-1-benzyl-3-hydroxyméthyl-4-(2-méthoxyphényl) pyrrolidine

Le produit attendu est obtenu selon le même procédé que celui décrit au stade A de l'exemple 6 mais en utilisant le [(3α,4α)-1-benzyl-4-(2-méthoxyphényl)pyrrolidin-3-yl]carboxylate de méthyle (préparé selon le procédé décrit dans J. Chem. Soc., Chem. Comm., 1566, 1985) comme matière première.

Rendement : 93 %
Point de fusion : 75 %

| Microanalyse élémentaire : | | | |
|---|---|---|---|
| | C % | H % | N % |
| calculé | 76,74 | 7,80 | 4,71 |
| trouvé | 76,78 | 7,82 | 5,07 |

Stade B : (3α,4α)-1-benzyl-3-chlorométhyl-4-(2-méthoxyphényl)pyrrolidine

Le produit attendu est obtenu selon le même procédé que celui décrit au stade B de l'exemple 6.

Rendement : 93 %
Point de fusion : 207-209°C (chlorhydrate)

| Microanalyse élémentaire (chlorhydrate) : | | | | | |
|---|---|---|---|---|---|
| | C % | H % | N % | Cl % | Cl % |
| calculé | 64,78 | 6,58 | 3,98 | 20,13 | 10,06 |
| trouvé | 64,59 | 6,46 | 4,01 | 20,21 | 10,02 |

Stade C : (3α,4α)-1-benzyl-3-cyanométhyl-4-(2-méthoxyphényl)pyrrolidine

Le produit attendu est obtenu selon le même procédé que celui décrit au stade C de l'exemple 6.

Rendement : 90 %

| Microanalyse élémentaire : | | | |
|---|---|---|---|
| | C % | H % | N % |
| calculé | 78,40 | 7,24 | 9,14 |
| trouvé | 78,45 | 7,17 | 9,18 |

Stade D : (3α,4α)-3-cyanométhyl-4-(2-méthoxyphényl)pyrrolidine

La débenzylation du composé obtenu au stade précédent est réalisée dans l'éthanol, à 30°C et pression atmosphérique, en présence d'acide chlorhydrique gazeux et de 10 % en masse de charbon palladié à 10 %. Le catalyseur est alors filtré, le filtrat évaporé et les cristaux sont filtrés.

Rendement : 98 %

Stade E : (3α,4α)-1-(napht-1-yl-sulfonyl)-3-cyanométhyl-4-(2-méthoxyphényl)pyrrolidine

Le produit attendu est obtenu selon le même procédé que celui décrit au stade C de l'exemple 16.

Rendement : 85 %

Stade F : (3α,4α)-1-(napht-1-yl-sulfonyl)-3-cyanométhyl-4-(2-hydroxyphényl)pyrrolidine

Additionner à -30°C, 240 ml (0,24 mole) d'une solution 1M de tribromure de bore dans le dichlorométhane à une solution chloroformique de 20,5 g (0,0475 mole) du composé obtenu dans l'étape précédente. Après 12 heures à température ambiante ajouter 100 ml d'eau puis 90 ml de lessive de soude à 10°C. Décanter la phase organique à pH 7 puis l'évaporer après séchage sur sulfate de sodium. Le produit est purifié par chromatographie sur silice en utilisant comme éluant un mélange dichlorométhane-méthanol 98/2 v/v.

Rendement : 45 %

Stade G : [(3α,4α)-1-(napht-1-yl-sulfonyl)-4-(2-hydroxyphényl) pyrrolidin-3-yl]acétaldéhyde

Le produit attendu est obtenu selon le même procédé que celui décrit au stade A de l'exemple 1.

Rendement : 75 %

Stade H : Acide (4Z)-6-[(3α,4α)-1-(napht-1-yl-sulfonyl)-4-(2-hydroxyphényl)pyrrolidin-3-yl)]hex-4-ènoïque

Le produit attendu est obtenu selon le même procédé que celui décrit au stade B de l'exemple 1.

Rendement : 25 %

| Microanalyse élémentaire : | | | | |
|---|---|---|---|---|
| | C % | H % | N % | S % |
| calculé | 67,08 | 5,85 | 3,01 | 6,89 |
| trouvé | 66,80 | 6,10 | 2,89 | 6,58 |

**EXEMPLE 29 : Acide (4Z)-6-[(3α,4α)-1-(napht-2-yl-sulfonyl)-4-(2-hydroxyphényl)pyrrolidin-3-yl)]hex-4-ènoïque**

Ce composé est obtenu selon les mêmes procédés que ceux décrits dans l'exemple 28.

Rendement : 25 %

| Microanalyse élémentaire : | | | | |
|---|---|---|---|---|
| | C % | H % | N % | S % |
| calculé | 67,08 | 5,85 | 3,01 | 6,89 |
| trouvé | 66,81 | 6,01 | 3,14 | 7,21 |

**<u>EXEMPLE 30</u> : Acide (5Z)-7-[(3α,4α)-1-[(isoquinolyl-5-yl)sulfonyl]-4-(2-hydroxyphényl)pyrrolidin-3-yl)]hept-5-ènoïque**

Ce composé est obtenu selon les mêmes procédés que ceux décrits dans l'exemple 28.

<u>Rendement</u> : 20 %

| Microanalyse élémentaire : | | | | |
|---|---|---|---|---|
| | C % | H % | N % | S % |
| calculé | 64,98 | 5,87 | 5,83 | 6,67 |
| trouvé | 64,66 | 6,15 | 5,68 | 6,67 |

**<u>EXEMPLE 31</u> : Acide (4Z)-6-[(3α,4α)-1-(napht-1-yl-sulfonyl)-4-(2-hydroxy-5-méthylphényl)pyrrolidin-3-yl]hex-4-ènoïque**

<u>Stade A</u> : (3α,4α)-1-benzyl-3-hydroxy-méthyl-4-(2-méthoxy-5-méthylphényl) pyrrolidine

Le produit attendu est obtenu par le même procédé que celui décrit au stade A de l'exemple 6, le produit de départ étant la [(3α,4α)-1-benzyl-4-(2-méthoxy-5-méthylphényl)pyrrolidin-3-yl]carboxylate de méthyle (préparé selon le même procédé décrit dans J. Chem. Soc., Chem. Comm., 1566, 1985).

<u>Rendement</u> : 93 %

| Microanalyse élémentaire : | | | |
|---|---|---|---|
| | C % | H % | N % |
| calculé | 77,14 | 8,09 | 4,50 |
| trouvé | 76,86 | 7,99 | 4,32 |

<u>Stade B</u> : (3α,4α)-1-benzyl-3-chlorométhyl-4-(2-méthoxy-5-méthylphényl) pyrrolidine

Le produit attendu est obtenu selon le même procédé que celui décrit au stade B de l'exemple 6.

<u>Rendement</u> : 82 %

| Microanalyse élémentaire : | | | | |
|---|---|---|---|---|
| | C % | H % | N % | Cl % |
| calculé | 72,82 | 7,33 | 4,25 | 10,75 |
| trouvé | 72,62 | 7,10 | 4,36 | 11,02 |

Stade C : (3α,4α)-1-benzyl-3-cyanométhyl-4-(2-méthoxy-5-méthylphényl) pyrrolidine

Le produit attendu est obtenu selon le même procédé que celui décrit au stade C de l'exemple 6.

Rendement : 63 %

| Microanalyse élémentaire : | | | | |
|---|---|---|---|---|
| | C % | H % | N % | S % |
| calculé | 68,55 | 5,75 | 6,66 | 7,62 |
| trouvé | 68,28 | 5,82 | 6,42 | 7,73 |

Stade D : (3α,4α)-3-cyanométhyl-4-(2-méthoxy-5-méthylphényl)pyrrolidine

Le produit attendu est obtenu selon le même procédé que celui décrit au stade D de l'exemple 28.

Stade E : (3α,4α)-1-(napht-1-yl-sulfonyl)-3-cyanométhyl-4-(2-méthoxy-5-méthylphényl)pyrrolidine

Le produit attendu est obtenu selon le même procédé que celui décrit au stade C de l'exemple 16.

Rendement : 78 %

Stade F : (3α,4α)-1-(napht-1-yl-sulfonyl)-3-cyanométhyl-4-(2-hydroxy-5-méthylphényl)pyrrolidine

Le produit attendu est obtenu selon le même procédé que celui décrit au stade F de l'exemple 28.

Rendement : 62 %

| Microanalyse élémentaire : | | | | |
|---|---|---|---|---|
| | C % | H % | N % | S % |
| calculé | 67,96 | 5,45 | 6,89 | 7,88 |
| trouvé | 67,72 | 5,58 | 6,71 | 7,63 |

Stade G : [(3α,4α)-1-(napht-1-yl-sulfonyl)-4-(2-hydroxy-5-méthylphényl) pyrrolidin-3-yl]acétaldéhyde

Le produit attendu est obtenu selon le même procédé que celui décrit au stade A de l'exemple 1.

Rendement : 78 %

Stade H : Acide (4Z)-6-[(3α,4α)-1-(napht-1-yl-sulfonyl)-4-(2-hydroxy-5-méthylphényl)pyrrolidin-3-yl]hex-4-ènoïque

Le produit attendu est obtenu selon le même procédé que celui décrit au stade B de l'exemple 1.

Rendement : 38 %

| Microanalyse élémentaire : | | | | |
|---|---|---|---|---|
| | C % | H % | N % | S % |
| calculé | 67,62 | 6,09 | 2,92 | 6,68 |
| trouvé | 67,35 | 5,89 | 3,07 | 6,74 |

**EXEMPLE 32 : Acide (4Z)-6-[(3α,4α)-1-(napht-1-yl-sulfonyl)-4-(2-méthoxy-5-méthylphényl)pyrrolidin-3-yl]hex-4-ènoïque**

Les stades A, B, C, D et E sont identiques aux stades A, B, C, D et E de l'exemple 31.

Stade F : [(3α,4α)-1-(napht-1-yl-sulfonyl)-4-(2-méthoxy-5-méthylphényl)pyrrolidin-3-yl]acétaldéhyde

Le produit attendu est obtenu selon le même procédé que celui décrit au stade A de l'exemple 1.

Rendement : 83 %

Stade G : Acide (4Z)-6-[(3α,4α)-1-(napht-1-yl-sulfonyl)-4-(2-méthoxy-5-méthylphényl)pyrrolidin-3-yl]hex-4-ènoïque

Le produit attendu est obtenu selon le même procédé que celui décrit au stade B de l'exemple 1.

Rendement : 36 %

| Microanalyse élémentaire : | | | | |
|---|---|---|---|---|
| | C % | H % | N % | S % |
| calculé | 68,13 | 6,33 | 2,84 | 6,49 |
| trouvé | 68,02 | 6,41 | 2,68 | 6,55 |

ETUDE PHARMACOLOGIQUE DES DERIVES DE L'INVENTION

**EXEMPLE 33 : Agrégation plaquettaire**

Les lapins (2-3 kg) sont anesthésiés avec du sodium pentobarbital (30 mg/kg i.v.). Après cannulation de l'artère carotide gauche, le sang est prélevé sur citrate de sodium (0.109 M) (1 vol. de citrate pour 9 vol. de sang).

Le plasma riche en plaquettes (PRP) est obtenu par centrifugation (20°C) à 250 g pendant 20 minutes et le plasma pauvre en plaquettes (PPP) par centrifugation à 1000 g (10 min). Le nombre des plaquettes (PL) dans le PRP est ajusté entre 300-350000 pl/$mm^3$ par dilution avec du PPP autologue. Le PRP est conservé à la température de la pièce jusqu'au moment du test et est utilisé dans les 4 heures qui suivent le prélèvement.

L'agrégation plaquettaire est réalisée à 37°C dans des tubes en verre siliconés à l'aide d'un agrégomètre. Le PRP et les PL sont agités à 1000 rpm (révolution par minute). Afin d'étudier l'activité des antagonistes du thromboxane, le PRP est incubé 1 min à 37°C, puis l'antagoniste est ajouté pour une durée de 3 min avant l'addition de l'agoniste U46619 (1.2 µM). Le volume final de la cuve est alors de 250 µl. L'intensité de l'agrégation plaquettaire est établie en prenant l'amplitude maximale de tracés agrégants et est exprimée en pourcentage de transmission lumineuse (% T). L'activité des antagonistes est exprimée en $IC_{50}$, c'est-à-dire la concentration de la substance qui induit 50 % d'inhibition de la réponse agrégante induite par le U46619.

Lors de ce test, l'$IC_{50}$ des composés des exemples 18, 22, 24, 25, 28 et 30 sont les suivants :

| | |
|---|---|
| exemple 18 | 5,4 $10^{-6}$M |
| exemple 22 | 4 $10^{-6}$M |
| exemple 24 | 0,8 $10^{-6}$M |
| exemple 25 | 5 $10^{-6}$M |
| exemple 28 | 0,5 $10^{-6}$M |
| exemple 30 | 0,5 $10^{-6}$M |

**EXEMPLE 34 : Détermination des valeurs des $pA_2$ sur la trachée de cobaye**

Des cobayes mâles albinos de 400-500 grammes ont été sacrifiés par un coup sur la nuque et par élongation cervicale. La gorge est ouverte et la trachée est rapidement prélevée et puis découpée en anneaux de deux cartilages. Ces anneaux sont montés entre deux crochets dans des cuves thermostatées à 37°C contenant du liquide physiologique (composition en mM:NaCl 118 ; $NaHCO_3$ 25 ; Glucose 10 ; KCl 4.7 ; $CaCl_2$ 1.25 ; $MgSO_4$ 1.19 ; $KH_2PO_4$ 1.14).

La solution physiologique est bullée par un mélange de 95 % $O_2$ - 5 % $CO_2$. Le crochet inférieur constitue le point fixe tandis que le crochet supérieur est relié à un capteur de force isométrique. Les tissus sont mis sous une tension de base de 3,5 grammes. Les substances pharmacologiques étudiées sont préparées immédiatement avant l'usage. Les drogues sont solubilisées dans l'eau ou dans le diméthylsulfoxyde.

Après le montage, les préparations sont laissées en repos pendant 90 minutes, des rinçages étant effectués toutes les 30 minutes. Après réajustement de la tension de base, une contraction provoquée par une dose unique d'agoniste (U46619 ; $10^{-5}$M) est provoquée afin de régulariser les contractions suivantes. Après lavage et retour à la ligne de base, une première courbe effet/concentration est réalisé par adjonction de doses cumulatives d'U46619 ($10^{-9}$M à $10^{-5}$M ; l'espacement entre les doses est d'un demi-log). Cette première expérience permet de calculer la concentration efficace 50 % ($EC_{50}$) "témoin".

Cette $EC_{50}$ est calculée en routine de la manière suivante : les valeurs de tension sont d'abord converties en pourcentages par rapport à l'effet maximum, ces pourcentages sont ensuite reportés sur un graphique avec les pourcentages en ordonnée et les log (concentration) en abscisse. Une régression linéaire est alors effectuée sur les points compris entre 10 % et 90 % (ce qui correspond à la partie linéaire de la courbe sigmoïde). La concentration correspondant au demi effet maximum (50 %) peut être facilement calculée à l'aide des paramètres de la droite.

Après lavage et retour à la ligne de base, l'organe est mis en contact avec l'antagoniste (8 concentrations différentes pour chaque organe) pendant 20 minutes. Une deuxième courbe effet/concentration est alors réalisée en présence de l'antagoniste et l'$EC_{50}$ "traité" peut alors être calculée. On dispose ainsi de tous les éléments permettant le calcul du $pA_2$ (antagonisme compétitif) ou $pD_2$ (antagonisme non compétitif).

Le $pA_2$ (qui représente le logarithme négatif de la concentration d'antagoniste en présence de laquelle il faut 2 fois plus d'agoniste pour obtenir le même effet) est déterminé en reportant sur un graphe les valeurs de log ((L/l)-1) par rapport à log (concentration d'antagoniste) avec L = effet en présence d'antagoniste et l = effet témoin.

Lors de ce test, les valeurs des $pA_2$ des composés des exemples 14 et 18 sont les suivantes :

exemple 14 : 6,55
exemple 18 : 8,10
exemple 19 : 6,7
exemple 21 : 5,8
exemple 23 : 7,7
exemple 24 : 9,5
exemple 28 : 8,8

**EXEMPLE 35 : $IC_{50}$ sur la pression trachéale chez le cobaye**

Des cobayes albinos mâles (350-400 g) soumis à une diète hydrique de 18 heures sont anesthésiés au carbamate d'éthyle (1.25 g/kg i.p.). Un cathéter est introduit dans l'artère carotide afin de mesurer la pression artérielle au moyen d'une cellule de pression. Un deuxième cathéter est introduit dans la veine jugulaire et sert à injecter les substances pharmacologiques. La trachée est cannulée et le cobaye est mis en respiration assitée au moyen d'un respirateur. La température de l'animal est maintenue à 37°C à l'aide d'une couverture thermostatée. Une aiguille piquée dans la cannule trachéale est reliée à une cellule de pression et permet d'enregistrer la pression trachéale.

Les cobayes sont prétraités par la d-tubocurarine (1 mg/kg i.v.) et par l'indométhacine (10 mg/kg i.v.). Injectée à la dose de 2 µg/kg i.v., le U46619 provoque une bronchoconstriction qui entraine une augmentation de la pression trachéale et induit une augmentation de la pression artérielle. Les réponses au U46619 sont réversibles et reproductibles si les injections sont effectuées toutes les 10 minutes.

Les antagonistes des récepteurs au thromboxane sont injectés 5 minutes avant les injections d'U46619. La dose d'antagoniste inhibant de 50 % l'augmentation de la pression trachéale causée par le U46619 est recherchée ($IC_{50}$).

| | |
|---|---|
| exemple 13 | : 2,28 mg/kg |
| exemple 14 | : 1,02 mg/kg |
| exemple 15 | : 0,177 mg/kg |
| exemple 18 | : 0,030 mg/kg |
| exemple 19 | : 0,1 mg/kg |
| exemple 20 | : 1,31 mg/kg |
| exemple 21 | : 1,03 mg/kg |
| exemple 22 | : 1,21 mg/kg |
| exemple 23 | : 0,43 mg/kg |
| exemple 24 | : 0,01 mg/kg |
| exemple 25 | : 0,06 mg/kg |
| exemple 27 | : 0,03 mg/kg |
| exemple 28 | : 0,01 mg/kg |
| exemple 29 | : 0,11 mg/kg |
| exemple 30 | : 0,08 mg/kg |

**EXEMPLE 36 : Composition pharmaceutique**

Formule de préparation pour 1000 comprimés dosés à 10 mg

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

**Revendications**

**Revendications pour les Etats contractants suivants : AT, BE, CH, DE, DK, FR, GB, GR, IE, IT, LI, LU, NL, PT, SE**

1. Composés de formule (I) :

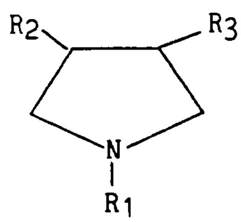

(I)

dans laquelle :

$R_1$ représente :

- un groupement alkyl ($C_1$-$C_6$) linéaire ou ramifié substitué ou non par un groupement pyridin-2-yl, pyridin-3-yl, phényl (lui-même éventuellement substitué par un ou plusieurs atomes d'halogène ou groupements alkyle ($C_1$-$C_6$) linéaire ou ramifié, alkoxy ($C_1$-$C_6$) linéaire ou ramifié, trihalogénométhyle),
- un groupement phényle substitué ou non par un ou plusieurs atomes d'halogène ou groupements alkyle ($C_1$-$C_6$) linéaire ou ramifié, alkoxy ($C_1$-$C_6$) linéaire ou ramifié, trihalogénométhyle,
- un groupement pyridyle,
- un groupement phénylsulfonyl (substitué ou non sur le noyau phényle par un ou plusieurs atomes d'halogène ou groupements alkyle ($C_1$-$C_6$) linéaire ou ramifié, alkoxy ($C_1$-$C_6$) linéaire ou ramifié, trihalogénométhyle), naphtylsulfonyl, quinolylsulfonyl ou isoquinolylsulfonyl,
- un groupement acyle ($C_1$-$C_6$) linéaire ou ramifié,
- un groupement benzoyle ou pyridylcarbonyl, substitué ou non sur le noyau aromatique par un ou plusieurs atomes d'halogène ou groupements alkyle ($C_1$-$C_6$) linéaire ou ramifié, alkoxy ($C_1$-$C_6$) linéaire ou ramifié, trihalogénométhyle,
- un groupement alkylaminocarbonyle ou phénylaminocarbonyle,
- un groupement acylamino ou benzoylamino,

$R_2$ représente :

- un atome d'hydrogène,
- un groupement phényle substitué ou non par un ou plusieurs atomes d'halogène ou groupements alkyle ($C_1$-$C_6$) linéaire ou ramifié, alkoxy ($C_1$-$C_6$) linéaire ou ramifié, hydroxy, trihalogénométhyle,
- un groupement pyridin-3-yle ou pyridin-2-yle, substitué ou non sur le noyau pyridine par un ou plusieurs atomes d'halogène ou groupements alkyle ($C_1$-$C_6$) linéaire ou ramifié, alkoxy ($C_1$-$C_6$) linéaire ou ramifié, trihalogénométhyle,

$R_3$ représente l'un quelconque des groupements suivants :

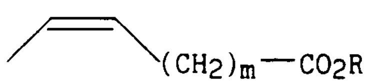

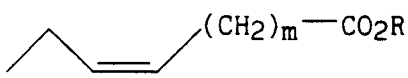

$$-(CH_2)_n-CO_2R$$

dans lesquels

m est égal à 2, 3 ou 4,
n est égal à 4, 5, 6 ou 7, et
R représente un atome d'hydrogène ou un groupement alkyle ($C_1$-$C_6$) linéaire ou ramifié,

leurs énantiomères, diastéréoisomères et épimères ainsi que leurs sels d'addition à un acide ou à une base pharmaceutiquement acceptable.

**2.** Composés de formule (I) selon la revendication 1 dans laquelle $R_1$ représente un groupement phénylsulfonyl substitué ou non, leurs énantiomères, diastéréoisomères et épimères ainsi que leurs sels d'addition à un acide ou à une

base pharmaceutiquement acceptable.

**3.** Composés de formule (I) selon la revendication 1 dans laquelle $R_2$ représente un groupement hydroxyphényle, leurs énantiomères, diastéréoisomères et épimères ainsi que leurs sels d'addition à un acide ou à une base pharmaceutiquement acceptable.

**4.** Composés de formule (I) selon la revendication 1 dans laquelle $R_1$ représente un groupement naphtylsulfonyl, leurs énantiomères, diastéréoisomères et épimères ainsi que leurs sels d'addition à un acide ou à une base pharmaceutiquement acceptable.

**5.** Composés de formule (I) selon la revendication 1 dans laquelle $R_1$ représente un groupement isoquinolylsulfonyl, leurs énantiomères, diastéréoisomères et épimères ainsi que leurs sels d'addition à un acide ou à une base pharmaceutiquement acceptable.

**6.** Composé de formule (I) selon la revendication 1 qui est l'acide (4Z)-6-[(3α,4α)-1-(4-chlorophénylsulfonyl)-4-(2-hydroxyphényl)pyrrolidin-3-yl] hex-4-ènoïque, ainsi que ses sels d'addition à une base pharmaceutiquement acceptable.

**7.** Composé de formule (I) selon la revendication 1 qui est l'acide (4Z)-6-[(3α,4α)-1-[(isoquinol-5-yl)sulfonyl]-4-(2-hydroxyphényl)pyrrolidin-3-yl] hex-4-ènoïque, ainsi que ses sels d'addition à une base pharmaceutiquement acceptable.

**8.** Composé de formule (I) selon la revendication 1 qui est l'acide (4Z)-6-[(3α,4α)-1-(napht-1-yl-sulfonyl)-4-(2-hydroxyphényl)pyrrolidin-3-yl)]hex-4-ènoïque, ainsi que ses sels d'addition à une base pharmaceutiquement acceptable.

**9.** Procédé de préparation des composés de formule (I) selon la revendication 1 caractérisé en ce que l'on utilise comme produit de départ une pyrrolidine de formule (II) :

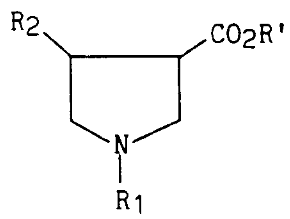

(II)

dans laquelle $R_1$ et $R_2$ ont la même signification que dans la formule (I) et R' représente un groupement alkyle ($C_1$-$C_6$) linéaire ou ramifié,
composé de formule (II), sous forme racémique ou isomérique, qui, lorsqu'il est sous forme d'un couple d'énantiomères, peut être transformé, en son autre couple d'énantiomères, par épimérisation dans un alcoolate de sodium, que l'on fait réagir :

**1** soit avec de l'hydrure de lithium aluminium dans un solvant inerte, pour conduire à la pyrrolidine de formule (III)

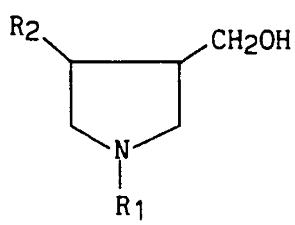

(III)

dans laquelle $R_1$ et $R_2$ ont la même signification que dans la formule (I), que l'on met en réaction :

- ou bien avec de l'acide chlorhydrique gazeux en présence de chlorure de thionyle en milieu chloroformique, pour conduire à la pyrrolidine de formule (IV) :

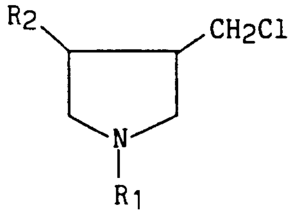

(IV)

dans laquelle $R_1$ et $R_2$ ont la même signification que dans la formule (I), que l'on transforme en pyrrolidine de formule (V) en présence de cyanure de terbutylammonium, dans du diméthylformamide :

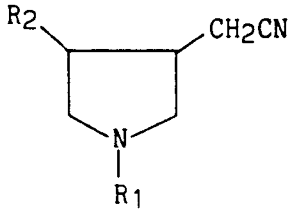

(V)

dans laquelle $R_1$ et $R_2$ ont la même signification que dans la formule (I),

- ou bien avec du chlorure de paratoluène sulfonyle dans de la pyridine pour conduire à la pyrrolidine de formule (VI) :

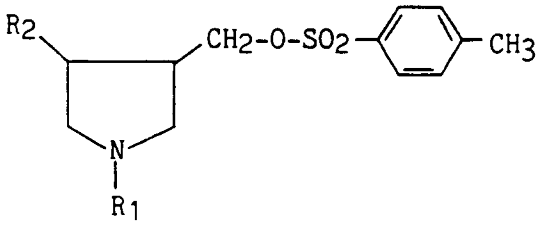

(VI)

dans laquelle $R_1$ et $R_2$ ont la même signification que dans la formule (I), que l'on transforme en pyrrolidine de formule (V) décrite précédemment, en présence de cyanure de potassium dans du diméthylsulfoxyde,
dérivé de formule (V) que l'on réduit à l'aide d'hydrure de diisobutylaluminium,
pour conduire à l'aldéhyde de formule (VII) :

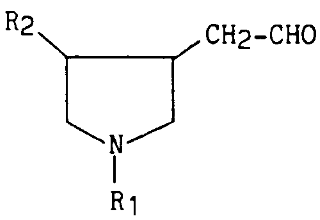

(VII)

dans lequel $R_1$ et $R_2$ ont la même signification que dans la formule (I),

**2** soit avec de l'hydrure de diisobutylaluminium, pour conduire à l'aldéhyde de formule (VIII) :

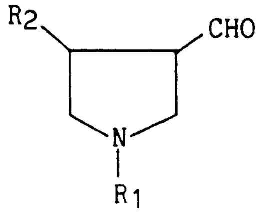

(VIII)

dans lequel $R_1$ et $R_2$ ont la même signification que dans la formule (I) qui est, si on le désire, mis en réaction avec l'ylure de phosphore de formule (IX), préparé par réaction du sel de phosphonium correspondant en présence de terbutanolate de potassium dans du tétrahydrofurane,

$$(C_6H_5)_3\ P{=}CHOCH_3 \qquad (IX)$$

pour conduire au composé de formule (X)

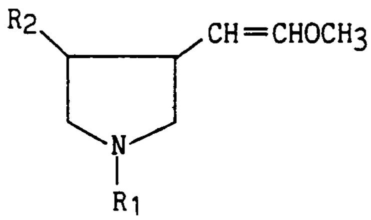

(X)

dans lequel $R_1$ et $R_2$ ont la même signification que dans la formule (I),
que l'on traite par de l'acide trifluoroacétique pour conduire à l'aldéhyde de formule (VII) décrit précédemment, composés de formule (VII) ou (VIII) que l'on fait réagir avec un ylure de phosphore de formule (XI), préparé par réaction du sel de phosphonium correspondant, en présence de terbutanolate de potassium dans du tétrahydrofurane,

$$(C_6H_5)_3\ P{=}CH{-}(CH_2)_m{-}CO_2H \qquad (XI)$$

dans laquelle m a la même signification que dans la formule (I),
pour conduire respectivement aux composés de formule (I/a) ou (I/b), cas particulier des composés de formule (I) :

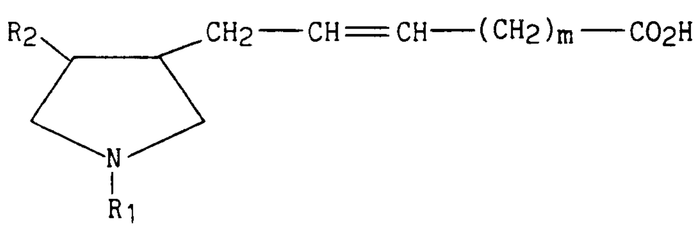

(I/a)

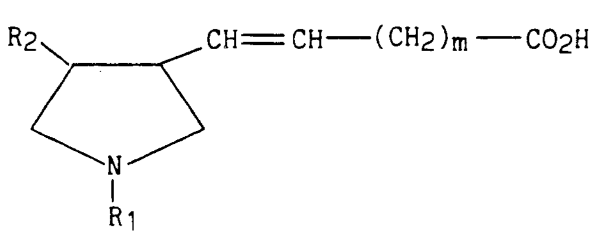

(I/b)

dans lesquelles $R_1$, $R_2$ et m ont la même signification que dans la formule (I),

composés de formule (I/a) ou (I/b)

- que l'on estérifie, si on le souhaite, pour conduire aux composés de formule ($I/a_1$) et ($I/b_1$) correspondants :

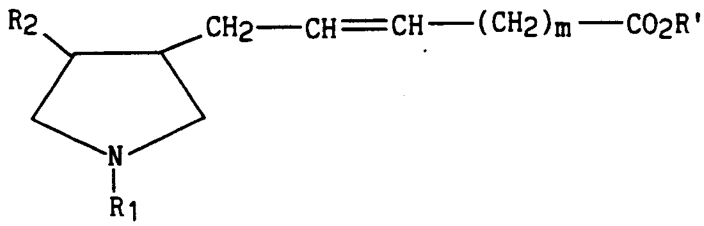

($I/a_1$)

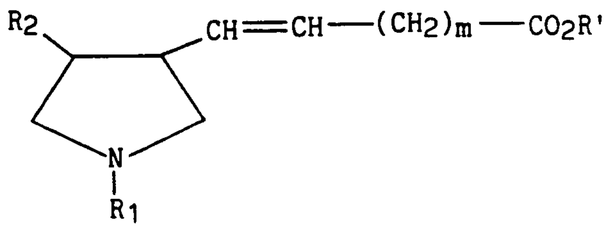

($I/b_1$)

dans lesquelles $R_1$ et $R_2$ ont la même signification que dans la formule (I) et R' représente un groupement alkyle ($C_1$-$C_6$) linéaire ou ramifié,

- dont on réduit éventuellement la double liaison par hydrogénation catalytique pour conduire au composé de formule (I/c), cas particulier des composés de formule (I),

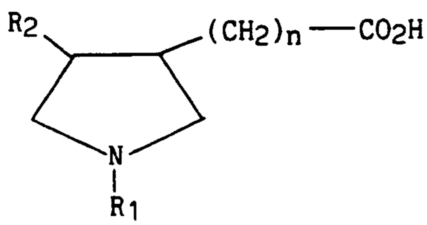

(I/c)

dans laquelle $R_1$, $R_2$ et n ont la même signification que dans la formule (I) et que l'on transforme, si on le souhaite, en ester correspondant de formule ($I/c_1$) :

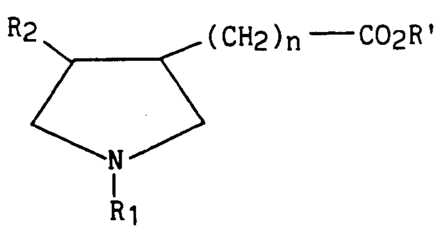

($I/c_1$)

dans laquelle $R_1$ et $R_2$ ont la même signification que dans la formule (I) et R' représente un groupement alkyle ($C_1$-$C_6$) linéaire ou ramifié,
composés de formule (I/a), ($I/a_1$), (I/b), ($I/b_1$), (I/c), ($I/c_1$) qui constituent l'ensemble des composés de formule (I),

- qui, lorsque $R_2$ représente un noyau phényle substitué par un groupement alkoxy ($C_1$-$C_6$) linéaire ou ramifié, peuvent être transformés, si on le désire, en composés de formule (I) dans lesquels $R_2$ représente un noyau phényle substitué par un groupement hydroxy,
- que l'on purifie, le cas échéant, selon une technique classique de purification,
- dont on sépare éventuellement les isomères selon une technique classique de purification,
- et que l'on transforme, si on le souhaite, en leurs sels d'addition à un acide ou à une base pharmaceutiquement acceptable.

**10.** Procédé de préparation des composés de formule (I'), cas particulier des composés de formule (I) selon la revendication 1 :

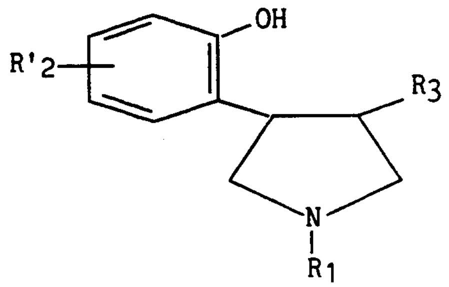

(I')

dans laquelle $R_1$ et $R_3$ ont la même signification que dans la formule (I), $R'_2$ représente un atome d'hydrogène, d'halogène ou un groupement alkyle ($C_1$-$C_6$) linéaire ou ramifié, alkoxy ($C_1$-$C_6$) linéaire ou ramifié ou trihalogénométhyle,
sont également obtenus en utilisant le procédé caractérisé en ce que l'on utilise comme produit de départ le composé de formule (XII) :

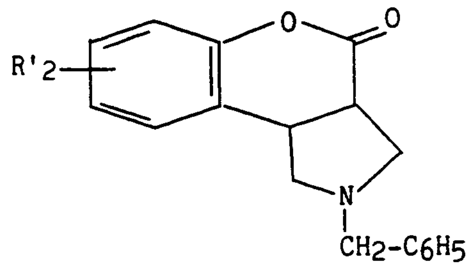

(XII)

dans laquelle $R'_2$ a la même signification que dans la formule (I'), que l'on réduit à l'aide d'hydrure de diisobutylaluminium,
pour conduire au composé de formule (XIII) :

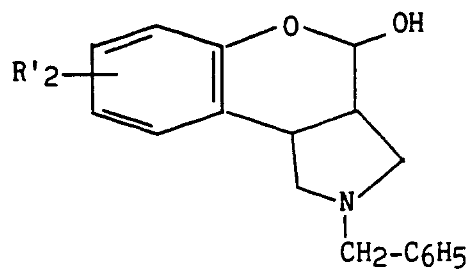

(XIII)

dans laquelle $R'_2$ a la même signification que précédemment, qui est :

a soit :

- ou bien transformé en composé de formule (XIV), par hydrogénation catalytique (Pd/c) dans du dioxane :

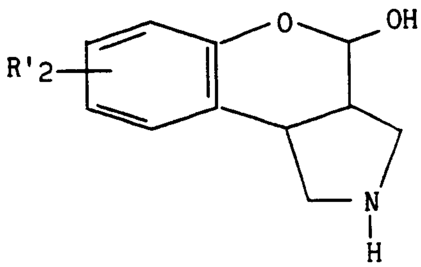

(XIV)

que l'on traite par un dérivé halogéné de formule $R_1X$ dans laquelle $R_1$ a la même signification que dans la formule (I) et X représente un atome d'halogène, dans du chloroforme en présence de triéthylamine, pour conduire au composé de formule (XV) :

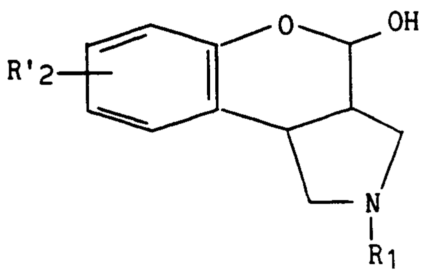

(XV)

dans laquelle $R'_2$ et $R_1$ ont la même signification que précédemment,

- ou bien transformé en composé de formule (XVI), par hydrogénation catalytique (Pd/c) dans un milieu acide chlorhydrique/éthanol :

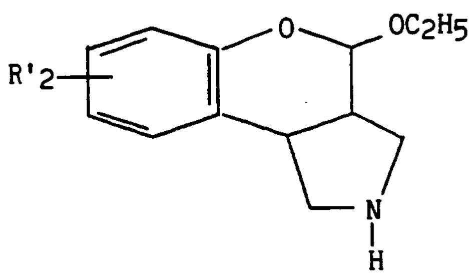

(XVI)

dans laquelle $R'_2$ a la même signification que précédemment, que l'on traite par un dérivé halogéné de formule $R_1X$, décrit précédemment, pour conduire au composé de formule (XVII) :

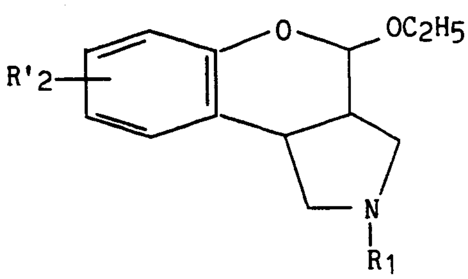

(XVII)

dans laquelle R'$_2$ et $R_1$ ont la même signification que précédemment, qui est traité par de l'acide trifluoroacétique, pour conduire au composé de formule (XV) décrit précédemment,
dérivé de formule (XV) qui est alors mis en réaction avec l'ylure de phosphore de formule (IX) préparé par réaction du sel de phosphonium correspondant,
en présence de terbutanolate de potassium dans du tétrahydrofurane,

$$(C_6H_5)_3\,P{=}CHOCH_3 \qquad (IX)$$

pour conduire au composé de formule (XVIII) :

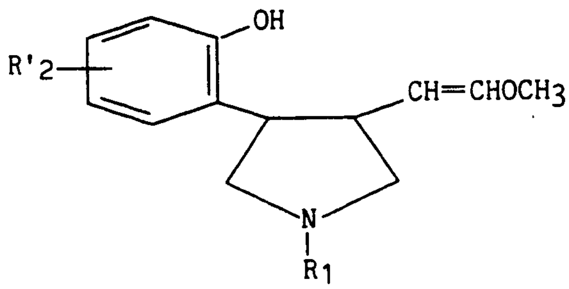

(XVIII)

dans laquelle $R_1$ et R'$_2$ ont la même signification que précédemment, dont on sépare éventuellement les diastéréoisomères selon une technique classique de séparation,
que l'on traite par de l'acide trifluoroacétique pour conduire à l'aldéhyde de formule (XIX) :

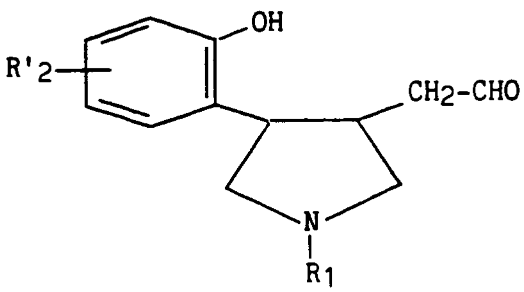

(XIX)

dans laquelle $R_1$ et R'$_2$ ont la même signification que précédemment, dont on sépare éventuellement les diastéréoisomères selon une technique classique de séparation,

b soit mis en réaction avec l'ylure de phosphore de formule (IX) décrit précédemment,

pour conduire au composé de formule (XX) :

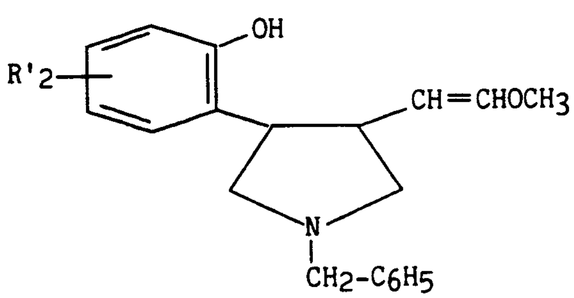

(XX)

dans laquelle R'$_2$ a la même signification que précédemment,
dont on sépare éventuellement les diastéréoisomères selon une technique classique de séparation,
que l'on traite par de l'acide trifluoroacétique,
pour conduire à l'aldéhyde de formule (XIX/a), cas particulier des composés de formule (XIX) :

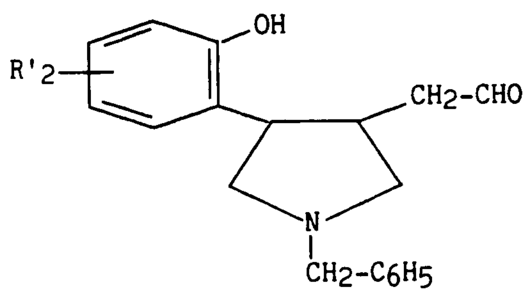

(XIX/a)

dans laquelle R'$_2$ a la même signification que précédemment,
dont on sépare éventuellement les diastéréoisomères selon une technique classique de séparation,
composés de formule (XIII) ou (XIX), que l'on fait réagir avec un ylure de phosphore de formule (X) préparé par réaction du sel de phosphonium correspondant en présence de terbutanolate de potassium dans du tétrahydrofurane,

$$(C_6H_5)_3\ P{=}CH\text{-}(CH_2)_m\text{-}CO_2H \qquad (X)$$

dans laquelle m a la même signification que dans la formule (I),
pour conduire respectivement aux composés de formules (I/d) et (I/e), cas particulier des composés de formule (I) :

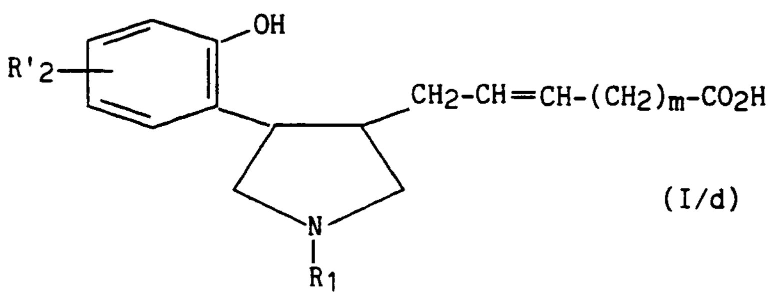

(I/d)

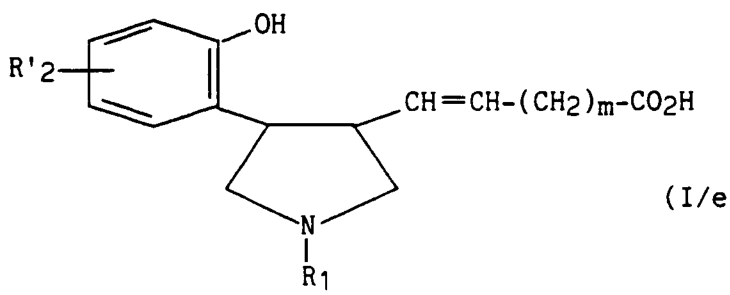

(I/e)

dans lesquelles R$_1$ et R'$_2$ ont la même signification que précédemment,

- que l'on stérifie, si on le souhaite, pour conduire aux composés de formules (I/d$_1$) et (I/e$_1$) correspondants :

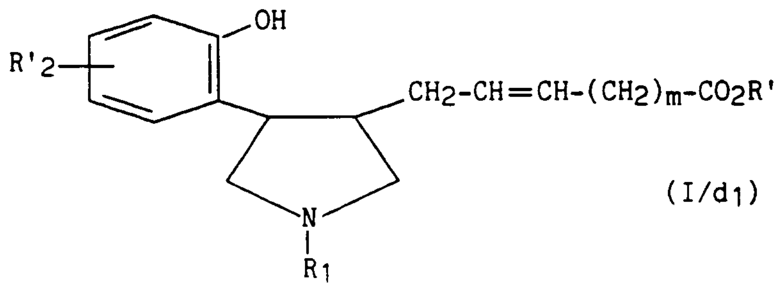

(I/d$_1$)

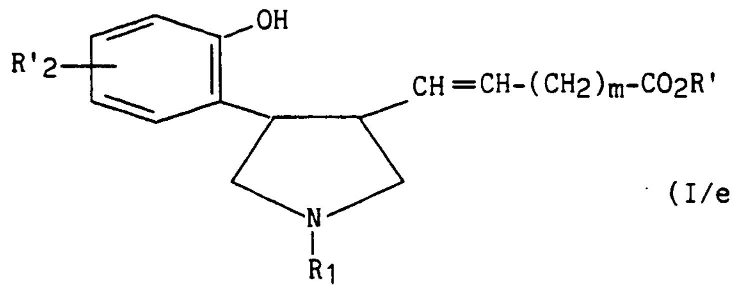

(I/e$_1$)

dans lesquelles $R_1$ et $R'_2$ ont la même signification que précédemment et R' représente un groupement alkyle ($C_1$-$C_6$) linéaire ou ramifié,

- dont on réduit éventuellement la double liaison par hydrogénation catalytique pour conduire au composé de formule (I/f), cas particulier des composés de formule (I) :

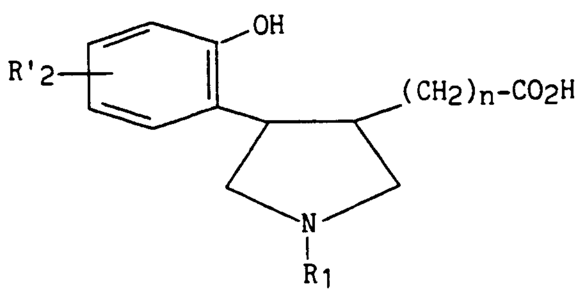

(I/f)

dans laquelle $R_1$ et $R'_2$ ont la même signification que précédemment, et que l'on transforme, si on le souhaite, en ester correspondant de formule (I/$f_1$) :

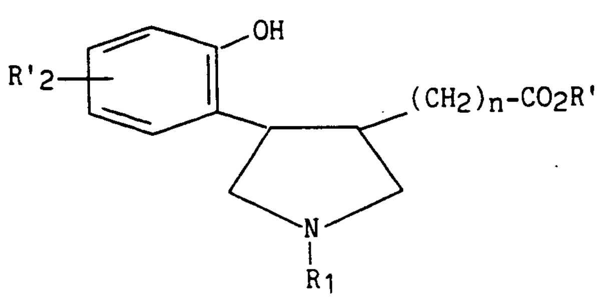

(I/$f_1$)

dans laquelle $R_1$, $R'_2$ et R' ont la même signification que précédemment,
composés de formule (I/d), (I/$d_1$), (I/e), (I/$e_1$), (I/f) et (I/$f_1$) qui constituent l'ensemble des composés de formule (I'),

- qui, lorsque $R'_2$ représente un groupement alkoxy ($C_1$-$C_6$) linéaire ou ramifié, peuvent être transformés, si on

le désire, en composés de formule (I') dans lesquels $R'_2$ représente un groupement hydroxy,

- que l'on purifie, le cas échéant, selon une technique classique de purification,
- dont on sépare éventuellement les isomères selon une technique classique de purification,
- et que l'on transforme, si on le souhaite, en leurs sels d'addition à un acide ou à une base pharmaceutiquement acceptable.

**11.** Compositions pharmaceutiques contenant comme principe actif au moins un composé selon l'une quelconque des revendications 1 à 3 seul ou en combinaison avec un ou plusieurs véhicules inertes non toxiques pharmaceutiquement acceptables.

**12.** Composition pharmaceutique selon la revendication 5 contenant au moins un principe actif selon l'une des revendications 1 à 3 utiles en tant qu'antagonistes des récepteurs au thromboxane $A_2$ ou en tant qu'inhibiteurs de la thromboxane $A_2$-synthase.

**Revendications pour l'Etat contractant suivant : ES**

**1.** Procédé de préparation des composés de formule (I) :

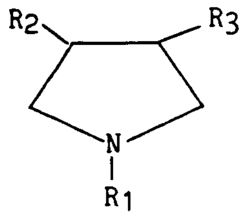

(I)

dans laquelle :

$R_1$ représente :

- un groupement alkyl ($C_1$-$C_6$) linéaire ou ramifié substitué ou non par un groupement pyridin-2-yl, pyridin-3-yl, phényl (lui-même éventuellement substitué par un ou plusieurs atomes d'halogène ou groupements alkyle ($C_1$-$C_6$) linéaire ou ramifié, alkoxy ($C_1$-$C_6$) linéaire ou ramifié, trihalogénométhyle),

- un groupement phényle substitué ou non par un ou plusieurs atomes d'halogène ou groupements alkyle ($C_1$-$C_6$) linéaire ou ramifié, alkoxy ($C_1$-$C_6$) linéaire ou ramifié, trihalogénométhyle,

- un groupement pyridyle,

- un groupement phénylsulfonyl (substitué ou non sur le noyau phényle par un ou plusieurs atomes d'halogène ou groupements alkyle ($C_1$-$C_6$) linéaire ou ramifié, alkoxy ($C_1$-$C_6$) linéaire ou ramifié, trihalogénométhyle), naphtylsulfonyl, quinolylsulfonyl ou isoquinolylsulfonyl,

- un groupement acyle ($C_1$-$C_6$) linéaire ou ramifié,

- un groupement benzoyle ou pyridylcarbonyl, substitué ou non sur le noyau aromatique par un ou plusieurs atomes d'halogène ou groupements alkyle ($C_1$-$C_6$) linéaire ou ramifié, alkoxy ($C_1$-$C_6$) linéaire ou ramifié, trihalogénométhyle,

- un groupement alkylaminocarbonyle ou phénylaminocarbonyle,

- un groupement acylamino ou benzoylamino,

$R_2$ représente :

- un atome d'hydrogène,

- un groupement phényle substitué ou non par un ou plusieurs atomes d'halogène ou groupements alkyle

($C_1$-$C_6$) linéaire ou ramifié, alkoxy ($C_1$-$C_6$) linéaire ou ramifié, hydroxy, trihalogénométhyle,

- un groupement pyridin-3-yle ou pyridin-2-yle, substitué ou non sur le noyau pyridine par un ou plusieurs atomes d'halogène ou groupements alkyle ($C_1$-$C_6$) linéaire ou ramifié, alkoxy ($C_1$-$C_6$) linéaire ou ramifié, trihalogénométhyle,

$R_3$ représente l'un quelconque des groupements suivants :

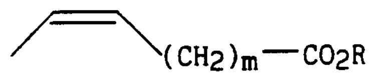

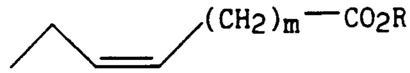

$$-(CH_2)_n-CO_2R$$

dans lesquels

m est égal à 2, 3 ou 4,
n est égal à 4, 5, 6 ou 7, et
R représente un atome d'hydrogène ou un groupement alkyle ($C_1$-$C_6$) linéaire ou ramifié,

leurs énantiomères, diastéréoisomères et épimères ainsi que leurs sels d'addition à un acide ou à une base pharmaceutiquement acceptable, caractérisé en ce que l'on utilise comme produit de départ une pyrrolidine de formule (II) :

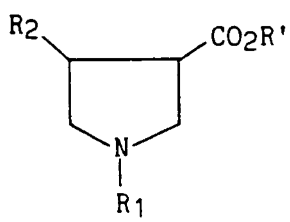

(II)

dans laquelle $R_1$ et $R_2$ ont la même signification que dans la formule (I) et R' représente un groupement alkyle ($C_1$-$C_6$) linéaire ou ramifié, composé de formule (II), sous forme racémique ou isomérique, qui, lorsqu'il est sous forme d'un couple d'énantiomères, peut être transformé, en son autre couple d'énantiomères, par épimérisation dans un alcoolate de sodium, que l'on fait réagir :

**1** soit avec de l'hydrure de lithium aluminium dans un solvant inerte, pour conduire à la pyrrolidine de formule (III)

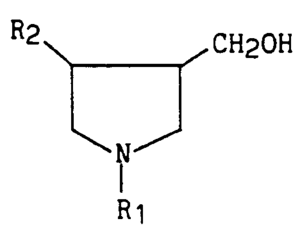

(III)

dans laquelle $R_1$ et $R_2$ ont la même signification que dans la formule (I), que l'on met en réaction :

- ou bien avec de l'acide chlorhydrique gazeux en présence de chlorure de thionyle en milieu chloroformique, pour conduire à la pyrrolidine de formule (IV) :

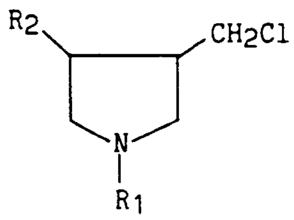

(IV)

dans laquelle $R_1$ et $R_2$ ont la même signification que dans la formule (I), que l'on transforme en pyrrolidine de formule (V) en présence de cyanure de terbutylammonium, dans du diméthylformamide :

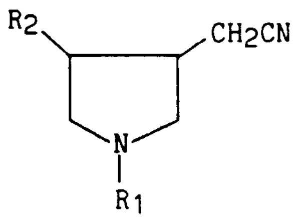

(V)

dans laquelle $R_1$ et $R_2$ ont la même signification que dans la formule (I),

- ou bien avec du chlorure de paratoluène sulfonyle dans de la pyridine pour conduire à la pyrrolidine de formule (VI) :

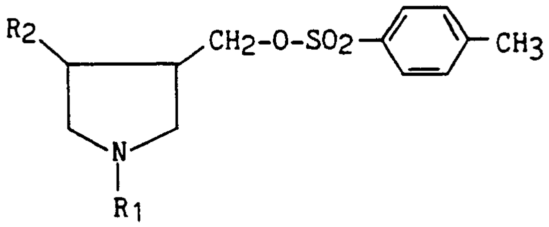

(VI)

dans laquelle $R_1$ et $R_2$ ont la même signification que dans la formule (I),
que l'on transforme en pyrrolidine de formule (V) décrite précédemment, en présence de cyanure de potassium dans du diméthylsulfoxyde,
dérivé de formule (V) que l'on réduit à l'aide d'hydrure de diisobutylaluminium,
pour conduire à l'aldéhyde de formule (VII) :

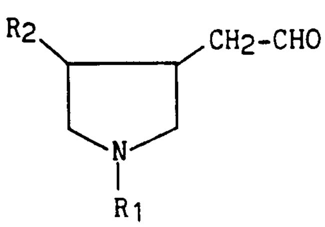

(VII)

dans lequel $R_1$ et $R_2$ ont la même signification que dans la formule (I),

**2** soit avec de l'hydrure de diisobutylaluminium, pour conduire à l'aldéhyde de formule (VIII) :

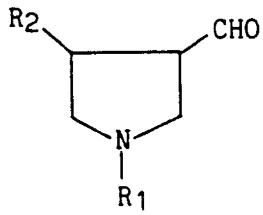

(VIII)

dans lequel $R_1$ et $R_2$ ont la même signification que dans la formule (I) qui est, si on le désire, mis en réaction avec l'ylure de phosphore de formule (IX), préparé par réaction du sel de phosphonium correspondant en présence de terbutanolate de potassium dans du tétrahydrofurane,

$$(C_6H_5)_3\ P{=}CHOCH_3 \qquad (IX)$$

pour conduire au composé de formule (X)

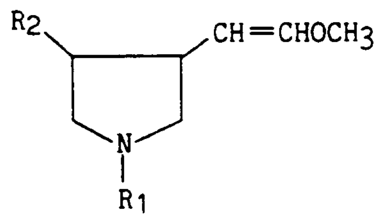

(X)

dans lequel $R_1$ et $R_2$ ont la même signification que dans la formule (I),
que l'on traite par de l'acide trifluoroacétique pour conduire à l'aldéhyde de formule (VII) décrit précédemment, composés de formule (VII) ou (VIII) que l'on fait réagir avec un ylure de phosphore de formule (XI), préparé par réaction du sel de phosphonium correspondant, en présence de terbutanolate de potassium dans du tétrahydrofurane,

$$(C_6H_5)_3\ P{=}CH{-}(CH_2)_m{-}CO_2H \qquad (XI)$$

dans laquelle m a la même signification que dans la formule (I),
pour conduire respectivement aux composés de formule (I/a) ou (I/b), cas particulier des composés de formule (I) :

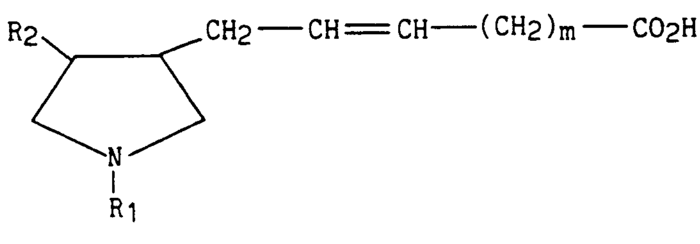

(I/a)

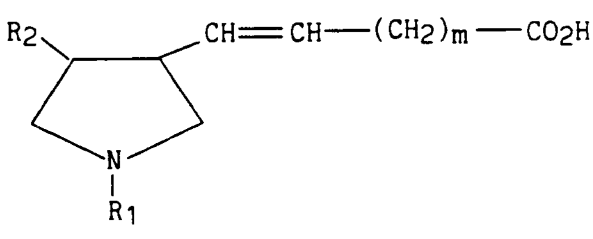

(I/b)

dans lesquelles $R_1$, $R_2$ et m ont la même signification que dans la formule (I), composés de formule (I/a) ou (I/b)

- que l'on estérifie, si on le souhaite, pour conduire aux composés de formule ($I/a_1$) et ($I/b_1$) correspondants :

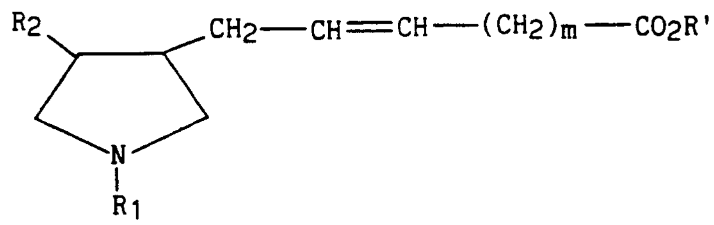

($I/a_1$)

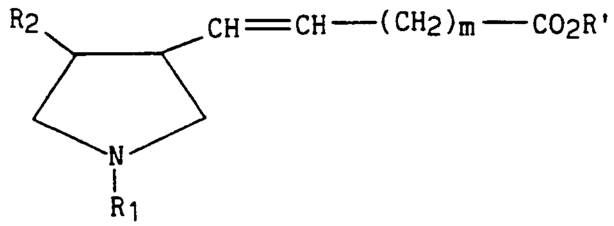

($I/b_1$)

dans lesquelles $R_1$ et $R_2$ ont la même signification que dans la formule (I) et R' représente un groupement alkyle ($C_1$-$C_6$) linéaire ou ramifié,

- dont on réduit éventuellement la double liaison par hydrogénation catalytique pour conduire au composé de formule (I/c), cas particulier des composés de formule (I),

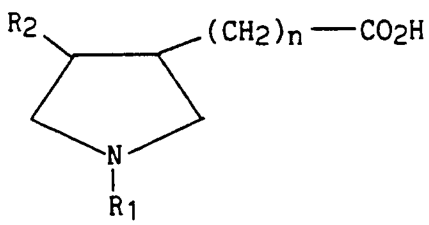

(I/c)

dans laquelle $R_1$, $R_2$ et n ont la même signification que dans la formule (I) et que l'on transforme, si on le souhaite, en ester correspondant de formule ($I/c_1$) :

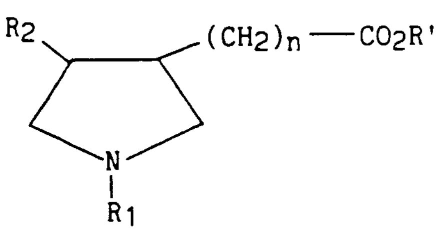

($I/c_1$)

dans laquelle $R_1$ et $R_2$ ont la même signification que dans la formule (I) et R' représente un groupement alkyle ($C_1$-$C_6$) linéaire ou ramifié,
composés de formule (I/a), ($I/a_1$), (I/b), ($I/b_1$), (I/c), ($I/c_1$) qui constituent l'ensemble des composés de formule (I),

- qui, lorsque $R_2$ représente un noyau phényle substitué par un groupement alkoxy ($C_1$-$C_6$) linéaire ou ramifié, peuvent être transformés, si on le désire, en composés de formule (I) dans lesquels $R_2$ représente un noyau phényle substitué par un groupement hydroxy,
- que l'on purifie, le cas échéant, selon une technique classique de purification,
- dont on sépare éventuellement les isomères selon une technique classique de purification,
- et que l'on transforme, si on le souhaite, en leurs sels d'addition à un acide ou à une base pharmaceutiquement acceptable.

2. Procédé de préparation des composés de formule (I) selon la revendication 1 dans laquelle $R_1$ représente un groupement phénylsulfonyl substitué ou non, leurs énantiomères, diastéréoisomères et épimères ainsi que leurs sels d'addition à un acide ou à une base pharmaceutiquement acceptable.

3. Procédé de préparation des composés de formule (I) selon la revendication 1 dans laquelle $R_2$ représente un groupement hydroxyphényle, leurs énantiomères, diastéréoisomères et épimères ainsi que leurs sels d'addition à un acide ou à une base pharmaceutiquement acceptable.

4. Procédé de préparation des composés de formule (I) selon la revendication 1 dans laquelle $R_1$ représente un groupement naphtylsulfonyl, leurs énantiomères, diastéréoisomères et épimères ainsi que leurs sels d'addition à un acide ou à une base pharmaceutiquement acceptable.

5. Procédé de préparation des composés de formule (I) selon la revendication 1 dans laquelle $R_1$ représente un groupement isoquinolylsulfonyl, leurs énantiomères, diastéréoisomères et épimères ainsi que leurs sels d'addition à un acide ou à une base pharmaceutiquement acceptable.

6. Procédé de préparation du composé de formule (I) selon la revendication 1 qui est l'acide (4Z)-6-[(3α,4α)-1-(4-chlorophényl-sulfonyl)-4-(2-hydroxyphényl)pyrrolidin-3-yl] hex-4-ènoïque, ainsi que ses sels d'addition à une base pharmaceutiquement acceptable.

7. Procédé de préparation du composé de formule (I) selon la revendication 1 qui est l'acide (4Z)-6-[(3α,4α)-1-[(isoquinol-5-yl)sulfonyl]-4-(2-hydroxyphényl) pyrrolidin-3-yl] hex-4-ènoïque, ainsi que ses sels d'addition à une base pharmaceutiquement acceptable.

8. Procédé de préparation du composé de formule (I) selon la revendication 1 qui est l'acide (4Z)-6-[(3α,4α)-1-(napht-1-yl-sulfonyl)-4-(2-hydroxyphényl)pyrrolidin-3-yl)]hex-4-ènoïque, ainsi que ses sels d'addition à une base pharmaceutiquement acceptable.

9. Procédé de préparation des composés de formule (I'), cas particulier des composés de formule (I) selon la revendication 1 :

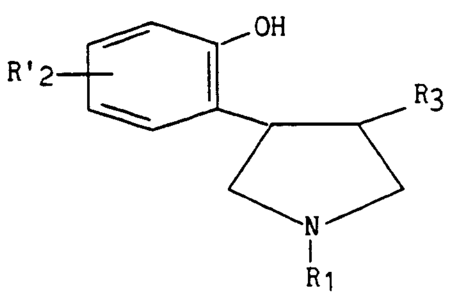

(I')

dans laquelle $R_1$ et $R_3$ ont la même signification que dans la formule (I), $R'_2$ représente un atome d'hydrogène, d'halogène ou un groupement alkyle ($C_1$-$C_6$) linéaire ou ramifié, alkoxy ($C_1$-$C_6$) linéaire ou ramifié ou trihalogénométhyle,
caractérisé en ce que l'on utilise comme produit de départ le composé de formule (XII) :

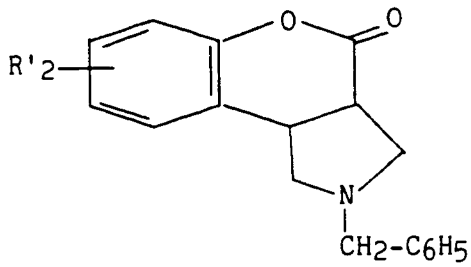

(XII)

dans laquelle R'$_2$ a la même signification que dans la formule (I'), que l'on réduit à l'aide d'hydrure de diisobutylaluminium,
pour conduire au composé de formule (XIII) :

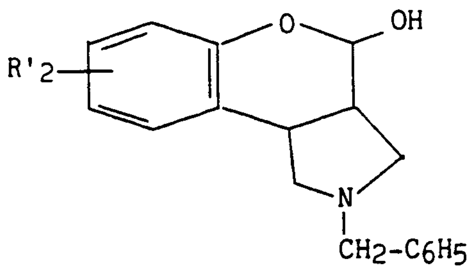

(XIII)

dans laquelle R'$_2$ a la même signification que précédemment, qui est :

a soit :

- ou bien transformé en composé de formule (XIV), par hydrogénation catalytique (Pd/c) dans du dioxane :

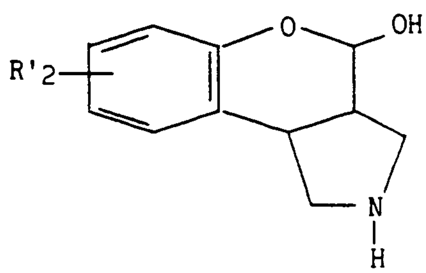

(XIV)

que l'on traite par un dérivé halogéné de formule R$_1$X dans laquelle R$_1$ a la même signification que dans la formule (I) et X représente un atome d'halogène, dans du chloroforme en présence de triéthylamine,
pour conduire au composé de formule (XV) :

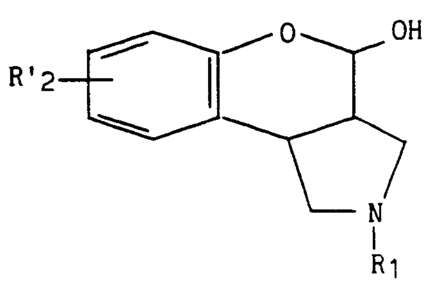

(XV)

dans laquelle R'$_2$ et $R_1$ ont la même signification que précédemment,

- ou bien transformé en composé de formule (XVI), par hydrogénation catalytique (Pd/c) dans un milieu acide chlorhydrique/éthanol :

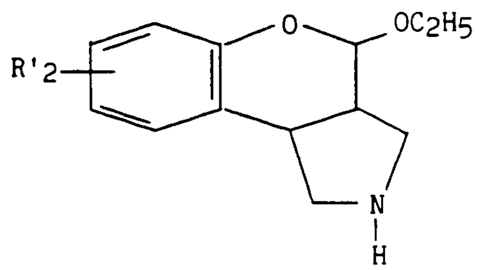

(XVI)

dans laquelle R'$_2$ a la même signification que précédemment,
que l'on traite par un dérivé halogéné de formule $R_1X$, décrit précédemment, pour conduire au composé de formule (XVII) :

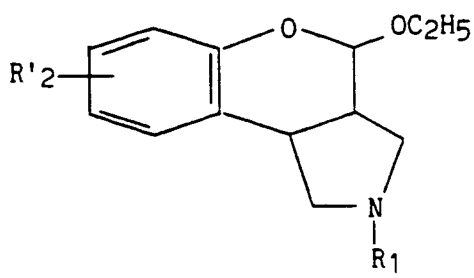

(XVII)

dans laquelle R'$_2$ et $R_1$ ont la même signification que précédemment,
qui est traité par de l'acide trifluoroacétique, pour conduire au composé de formule (XV) décrit précédemment, dérivé de formule (XV) qui est alors mis en réaction avec l'ylure de phosphore de formule (IX) préparé par réaction du sel de phosphonium correspondant,
en présence de terbutanolate de potassium dans du tétrahydrofurane,

$$(C_6H_5)_3\ P{=}CHOCH_3 \qquad (IX)$$

pour conduire au composé de formule (XVIII) :

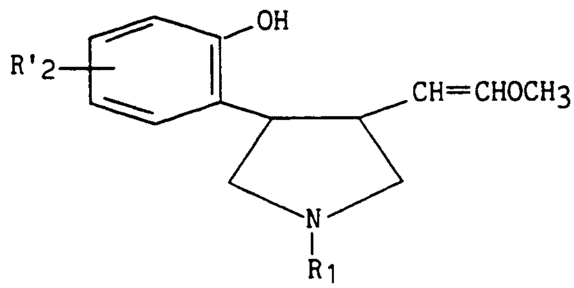

(XVIII)

dans laquelle $R_1$ et R'$_2$ ont la même signification que précédemment,
dont on sépare éventuellement les diastéréoisomères selon une technique classique de séparation,
que l'on traite par de l'acide trifluoroacétique pour conduire à l'aldéhyde de formule (XIX) :

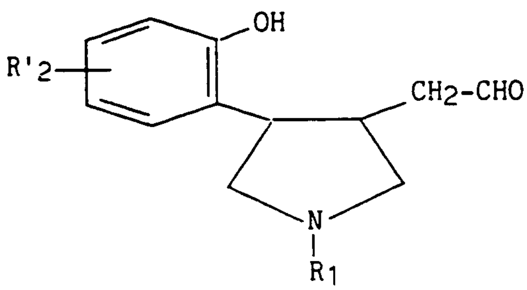

(XIX)

dans laquelle $R_1$ et $R'_2$ ont la même signification que précédemment,
dont on sépare éventuellement les diastéréoisomères selon une technique classique de séparation,

b soit mis en réaction avec l'ylure de phosphore de formule (IX) décrit précédemment,
pour conduire au composé de formule (XX) :

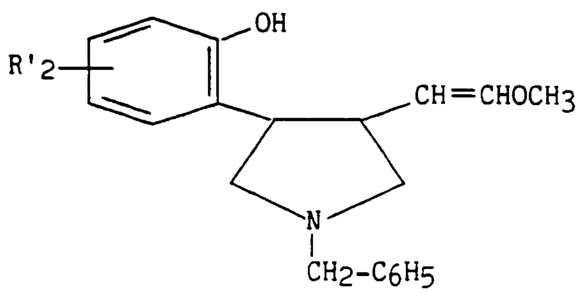

(XX)

dans laquelle $R'_2$ a la même signification que précédemment,
dont on sépare éventuellement les diastéréoisomères selon une technique classique de séparation,
que l'on traite par de l'acide trifluoroacétique,
pour conduire à l'aldéhyde de formule (XIX/a), cas particulier des composés de formule (XIX) :

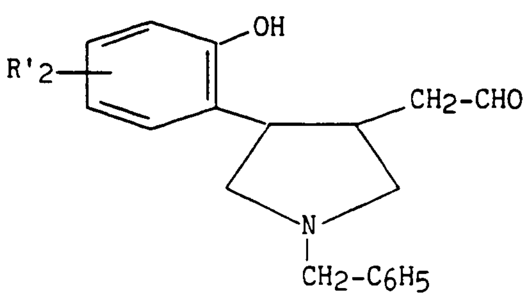

(XIX/a)

dans laquelle $R'_2$ a la même signification que précédemment,
dont on sépare éventuellement les diastéréoisomères selon une technique classique de séparation,
composés de formule (XIII) ou (XIX), que l'on fait réagir avec un ylure de phosphore de formule (X) préparé par réaction du sel de phosphonium correspondant en présence de terbutanolate de potassium dans du tétrahydrofurane,

$$(C_6H_5)_3\,P{=}CH\text{-}(CH_2)_m\text{-}CO_2H \qquad (X)$$

dans laquelle m a la même signification que dans la formule (I), pour conduire respectivement aux composés de formules (I/d) et (I/e), cas particulier des composés de formule (I) :

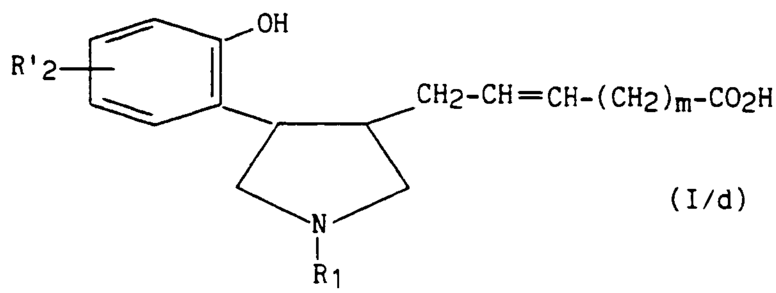

(I/d)

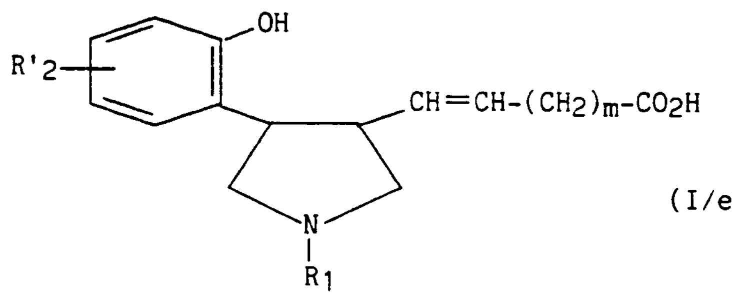

(I/e)

dans lesquelles $R_1$ et $R'_2$ ont la même signification que précedemment,

- que l'on stérifie, si on le souhaite, pour conduire aux composés de formules ($I/d_1$) et ($I/e_1$) correspondants :

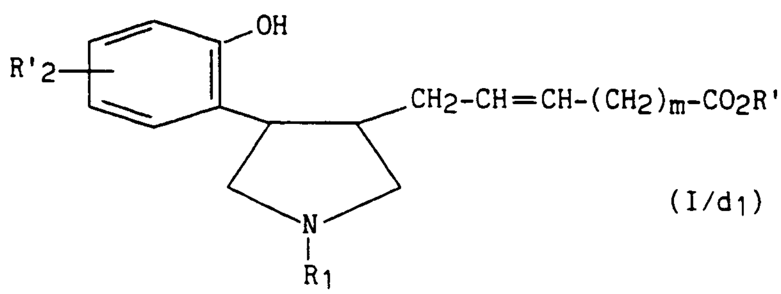

($I/d_1$)

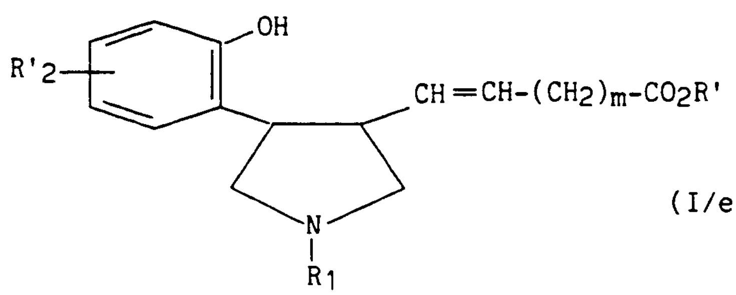

($I/e_1$)

dans lesquelles $R_1$ et $R'_2$ ont la même signification que précédemment et R' représente un groupement alkyle ($C_1$-$C_6$) linéaire ou ramifié,

- dont on réduit éventuellement la double liaison par hydrogénation catalytique pour conduire au composé de formule (I/f), cas particulier des composés de formule (I) :

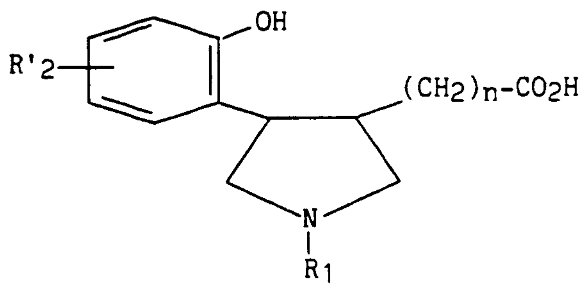

(I/f)

dans laquelle $R_1$ et $R'_2$ ont la même signification que précédemment, et que l'on transforme, si on le souhaite, en ester correspondant de formule ($I/f_1$) :

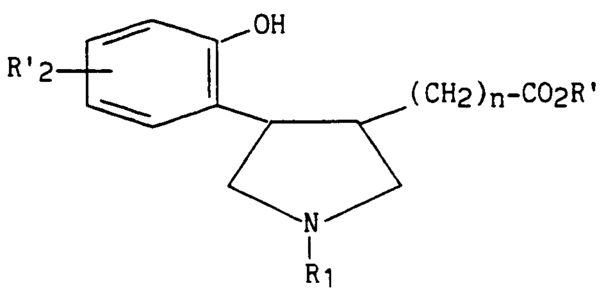

($I/f_1$)

dans laquelle $R_1$, $R'_2$ et R' ont la même signification que précédemment, composés de formule (I/d), ($I/d_1$), (I/e), ($I/e_1$), (I/f) et ($I/f_1$) qui constituent l'ensemble des composés de formule (I'),

- qui, lorsque $R'_2$ représente un groupement alkoxy ($C_1$-$C_6$) linéaire ou ramifié, peuvent être transformés, si on le désire, en composés de formule (I') dans lesquels $R'_2$ représente un groupement hydroxy,
- que l'on purifie, le cas échéant, selon une technique classique de purification,
- dont on sépare éventuellement les isomères selon une technique classique de purification,
- et que l'on transforme, si on le souhaite, en leurs sels d'addition à un acide ou à une base pharmaceutiquement acceptable.

**Claims**

**Claims for the following Contracting States : AT, BE, CH, DE, DK, FR, GB, GR, IE, IT, LI, LU, NL, PT, SE**

**1.** Compounds of formula (I):

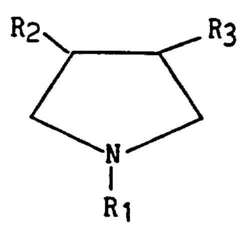

(I)

in which:

$R_1$ represents:

- a linear or branched ($C_1$-$C_6$)alkyl group that is unsubstituted or substituted by a pyridin-2-yl, pyridin-3-yl or phenyl group (which is itself optionally substituted by one or more halogen atoms or linear or branched

($C_1$-$C_6$)alkyl, linear or branched ($C_1$-$C_6$)alkoxy or trihalomethyl groups),

- a phenyl group that is unsubstituted or substituted by one or more halogen atoms or linear or branched ($C_1$-$C_6$)-alkyl, linear or branched ($C_1$-$C_6$)alkoxy or trihalomethyl groups,
- a pyridyl group,
- a phenylsulphonyl group (that is unsubstituted or substituted on the phenyl ring by one or more halogen atoms or linear or branched ($C_1$-$C_6$)alkyl, linear or branched ($C_1$-$C_6$)alkoxy or trihalomethyl groups), a naphthylsulphonyl group, a quinolylsulphonyl group or an isoquinolylsulphonyl group,
- a linear or branched ($C_1$-$C_6$)acyl group,
- a benzoyl or pyridylcarbonyl group that is unsubstituted or substituted on the aromatic ring by one or more halogen atoms or linear or branched ($C_1$-$C_6$)alkyl, linear or branched ($C_1$-$C_6$)alkoxy or trihalomethyl groups,
- an alkylaminocarbonyl or phenylaminocarbonyl group, or
- an acylamino or benzoylamino group;

$R_2$ represents:

- a hydrogen atom,
- a phenyl group that is unsubstituted or substituted by one or more halogen atoms or linear or branched ($C_1$-$C_6$)alkyl, linear or branched ($C_1$-$C_6$)alkoxy, hydroxy or trihalomethyl groups, or
- a pyridin-3-yl or pyridin-2-yl group that is unsubstituted or substituted on the pyridine ring by one or more halogen atoms or linear or branched ($C_1$-$C_6$)alkyl, linear or branched ($C_1$-$C_6$)alkoxy or trihalomethyl groups; and

$R_3$ represents any one of the following groups:

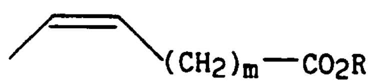

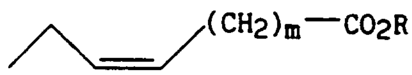

$$-(CH_2)_n-CO_2R$$

in which

m is 2, 3 or 4,
n is 4, 5, 6 or 7, and
R represents a hydrogen atom or a linear or branched ($C_1$-$C_6$)alkyl group;

their enantiomers, diastereoisomers and epimers, as well as addition salts thereof with a pharmaceutically acceptable acid or base.

**2.** Compounds of formula (I) according to claim 1 in which $R_1$ represents an unsubstituted or substituted phenylsulphonyl group, their enantiomers, diastereoisomers and epimers, as well as addition salts thereof with a pharmaceutically acceptable acid or base.

**3.** Compounds of formula (I) according to claim 1 in which $R_2$ represents a hydroxyphenyl group, their enantiomers, diastereoisomers and epimers, as well as addition salts thereof with a pharmaceutically acceptable acid or base.

**4.** Compounds of formula (I) according to claim 1 in which $R_1$ represents a naphthylsulphonyl group, their enantiomers, diastereoisomers and epimers, as well as addition salts thereof with a pharmaceutically acceptable acid or base.

**5.** Compounds of formula (I) according to claim 1 in which $R_1$ represents an isoquinolylsulphonyl group, their enantiomers, diastereoisomers and epimers, as well as addition salts thereof with a pharmaceutically acceptable acid or base.

6. Compound of formula (I) according to claim 1 that is (4Z)-6-[(3α,4α)-1-(4-chlorophenylsulphonyl)-4-(2-hydroxyphenyl)pyrrolidin-3-yl]hex-4-enoic acid, as well as its addition salts with a pharmaceutically acceptable base.

7. Compound of formula (I) according to claim 1 that is (4Z)-6-[(3α,4α)-1-((isoquinol-5-yl)sulphonyl]-4-(2-hydroxyphenyl)pyrrolidin-3-yl)hex-4-enoic acid, as well as its addition salts with a pharmaceutically acceptable base.

8. Compound of formula (I) according to claim 1 that is (4Z)-6-[(3α,4α)-1-(naphth-1-ylsulphonyl)-4-(2-hydroxyphenyl)pyrrolidin-3-yl]hex-4-enoic acid, as well as its addition salts with a pharmaceutically acceptable base.

9. Process for the preparation of compounds of formula (I) according to claim 1, characterised in that there is used as starting material a pyrrolidine of formula (II):

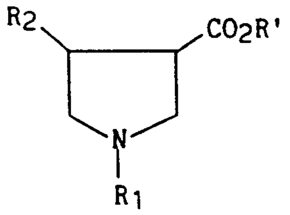 (II),

in which $R_1$ and $R_2$ are as defined in formula (I) and R' represents a linear or branched ($C_1$-$C_6$)alkyl group, which, in racemic or isomeric form, may, when in the form of a pair of enantiomers, be converted into its other pair of enantiomers by epimerisation in a sodium alcoholate,
which compound of formula (II) is reacted:

1) **<u>either</u>** with lithium aluminium hydride in an inert solvent to yield a pyrrolidine of formula (III):

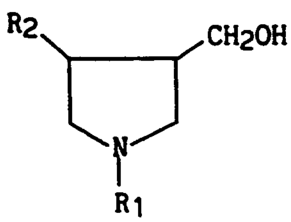 (III)

in which $R_1$ and $R_2$ are as defined in formula (I), which is reacted:

- <u>either</u> with gaseous hydrogen chloride in the presence of thionyl chloride in a chloroform medium to yield a pyrrolidine of formula (IV):

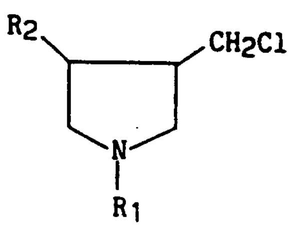 (IV)

in which $R_1$ and $R_2$ are as defined in formula (I),
which is converted in the presence of tert.-butylammonium cyanide in dimethylformamide into a pyrrolidine of formula (V):

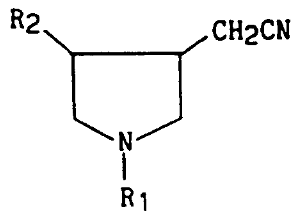

(V)

in which $R_1$ and $R_2$ are as defined in formula (I),

- or with para-toluenesulphonyl chloride in pyridine to yield a pyrrolidine of formula (VI):

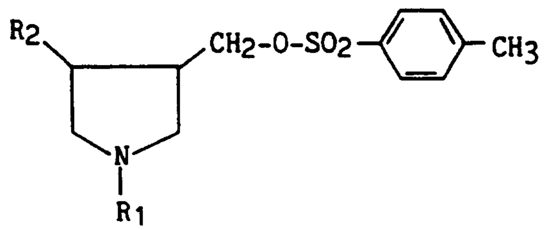

(VI)

in which $R_1$ and $R_2$ are as defined in formula (I),
which is converted into a pyrrolidine of formula (V) described above in the presence of potassium cyanide in dimethyl sulphoxide,
which compound of formula (V) is reduced by means of diisobutylaluminium hydride to yield an aldehyde of formula (VII):

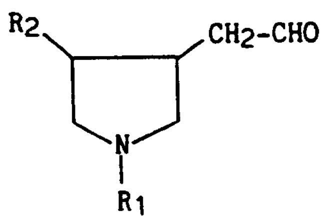

(VII)

in which $R_1$ and $R_2$ are as defined in formula (I),

2) **or** with diisobutylaluminium hydride to yield an aldehyde of formula (VIII):

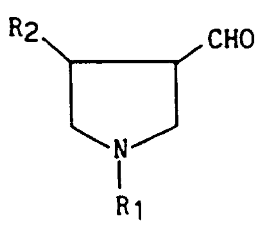

(VIII)

in which $R_1$ and $R_2$ are as defined in formula (I),
which is, if desired, reacted with the phosphorus ylid of formula (IX), prepared by reaction of the corresponding phosphonium salt in the presence of potassium tert.-butanolate in tetrahydrofuran,

$$(C_6H_5)_3P{=}CHOCH_3 \qquad (IX)$$

to yield a compound of formula (X):

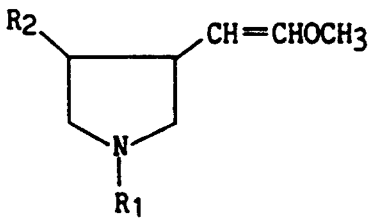

(X)

in which $R_1$ and $R_2$ are as defined in formula (I), which is treated with trifluoroacetic acid to yield an aldehyde of formula (VII) described above, which compound of formula (VII) or (VIII) is reacted with a phosphorus ylid of formula (XI), prepared by reaction of the corresponding phosphonium salt in the presence of potassium tert.-butanolate in tetrahydrofuran,

$$(C_6H_5)_3P{=}CH\text{-}(CH_2)_m\text{-}CO_2H \qquad (XI)$$

in which m is as defined in formula (I), to yield a compound of formula (I/a) or (I/b), respectively, which are a particular case of the compounds of formula (I):

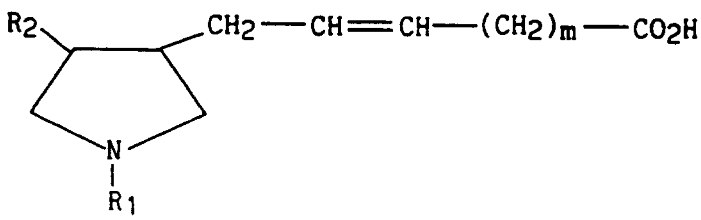

(I/a)

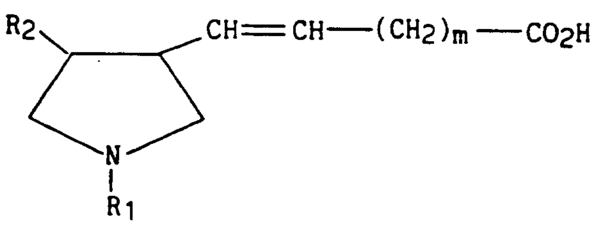

(I/b)

in which $R_1$, $R_2$ and m are as defined in formula (I), which compounds of formulae (I/a) and (I/b) are esterified, if desired, to yield the corresponding compounds of formulae ($I/a_1$) and ($I/b_1$):

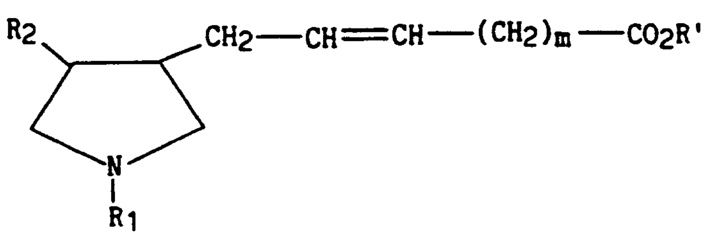

($I/a_1$)

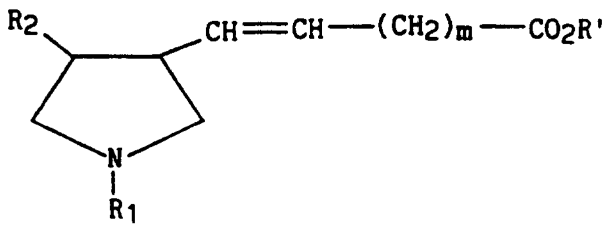

(I/$b_1$)

in which $R_1$ and $R_2$ are as defined in formula (I) and R' represents a linear or branched ($C_1$-$C_6$)alkyl group, the double bond of which is optionally reduced by catalytic hydrogenation to yield a compound of formula (I/c), which is a particular case of the compounds of formula (I):

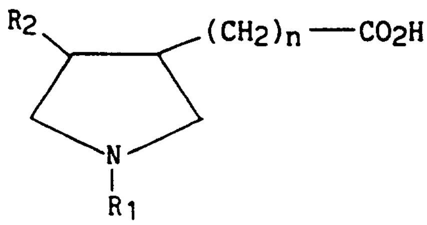

(I/c)

in which $R_1$, $R_2$ and n are as defined in formula (I),
which compound of formula (I/c) is converted, if desired, into the corresponding ester of formula (I/$c_1$):

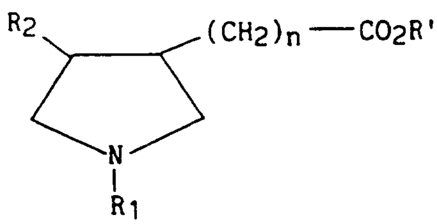

(I/$c_1$)

in which $R_1$ and $R_2$ are as defined in formula (I) and R' represents a linear or branched ($C_1$-$C_6$)alkyl group, which compounds of formulae (I/a), (I/$a_1$), (I/b), (I/$b_1$), (I/c) and (I/$c_1$) form the totality of the compounds of formula (I),

- which, when $R_2$ represents a phenyl ring substituted by a linear or branched ($C_1$-$C_6$)alkoxy group, may be converted, if desired, into compounds of formula (I) in which $R_2$ represents a phenyl ring substituted by a hydroxy group,
- which are purified, where appropriate, by a customary purification method,
- the isomers of which are separated, where appropriate, by a customary purification method,
- and which are converted, if desired, into their addition salts with a pharmaceutically acceptable acid or base.

**10.** Process for the preparation of compounds of formula (I'), which are a particular case of the compounds of formula (I) according to claim 1:

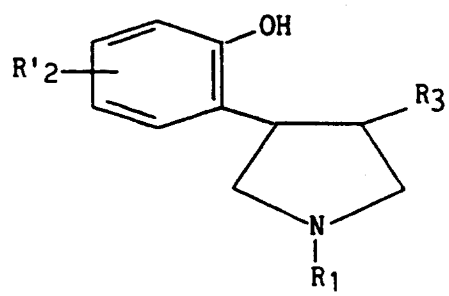

(I')

in which $R_1$ and $R_3$ are as defined in formula (I) and $R'_2$ represents a hydrogen atom, a halogen atom or a group linear or branched ($C_1$-$C_6$)alkyl, linear or branched ($C_1$-$C_6$)alkoxy or trihalomethyl,
characterised in that there is used as starting material a compound of formula (XII):

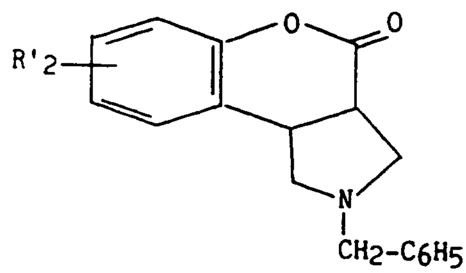

(XII)

in which $R'_2$ is as defined in formula (I'),
which is reduced by means of diisobutylaluminium hydride to yield a compound of formula (XIII):

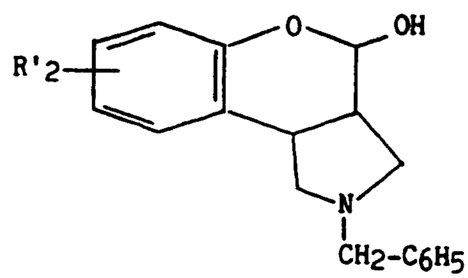

(XIII)

in which $R'_2$ is as defined above,
which is:

**a either**

- either converted by catalytic hydrogenation (Pd/C) in dioxane into a compound of formula (XIV):

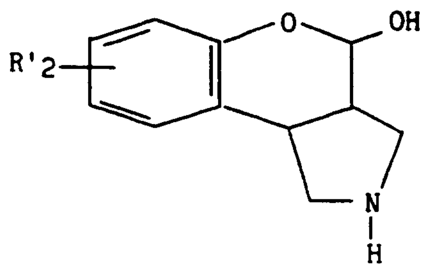

(XIV),

which is treated with a halogenated compound of the formula $R_1X$, in which $R_1$ is as defined in formula (I) and X represents a halogen atom, in chloroform in the presence of triethylamine to yield a compound of formula (XV):

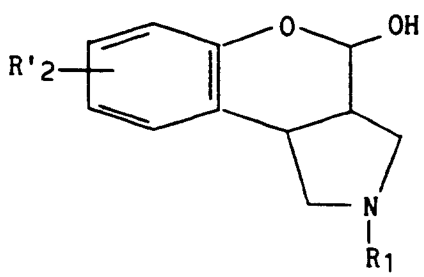

(XV)

in which $R'_2$ and $R_1$ are as defined above,

- or converted by catalytic hydrogenation (Pd/C) in a hydrochloric acid/ethanol medium into a compound of formula (XVI):

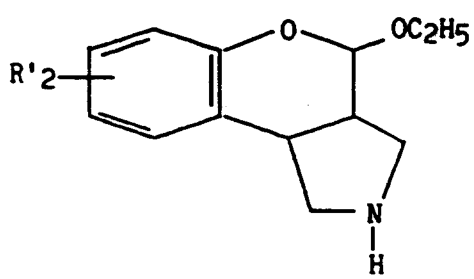

(XVI)

in which $R'_2$ is as defined above,
which is treated with a halogenated compound of the formula $R_1X$, described above, to yield a compound of formula (XVII):

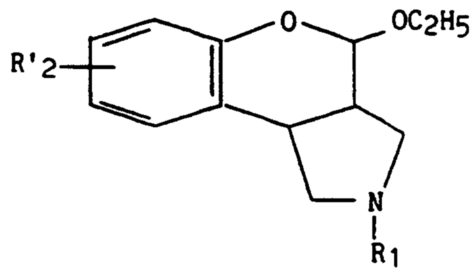

(XVII)

in which R'$_2$ and $R_1$ are as defined above,
which is treated with trifluoroacetic acid to yield a compound of formula (XV) described above,
which compound of formula (XV) is then reacted with the phosphorus ylid of formula (IX), prepared by reaction of the corresponding phosphonium salt in the presence of potassium tert.-butanolate in tetrahydrofuran,

$$(C_6H_5)_3P{=}CHOCH_3 \qquad (IX)$$

to yield a compound of formula (XVIII):

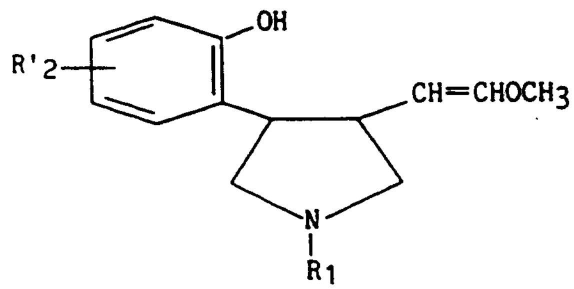

(XVIII)

in which $R_1$ and R'$_2$ are as defined above,
the diastereoisomers of which are separated, where appropriate, by a customary separation method,
which is treated with trifluoroacetic acid to yield an aldehyde of formula (XIX):

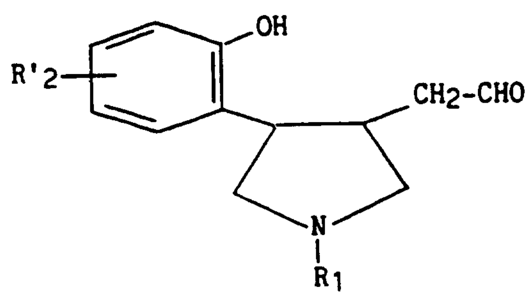

(XIX)

in which $R_1$ and R'$_2$ are as defined above,
the diastereoisomers of which are separated, where appropriate, by a customary separation method,

**b or** reacted with the phosphorus ylid of formula (IX) described above to yield a compound of formula (XX):

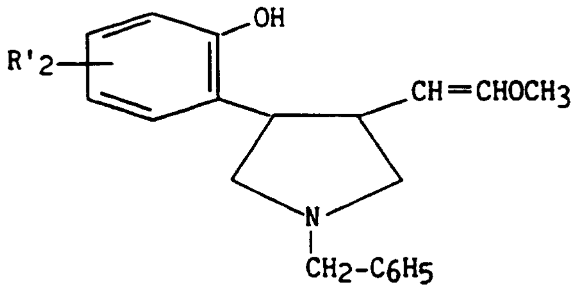

(XX)

in which $R'_2$ is as defined above,
the diastereoisomers of which are separated, where appropriate, by a customary separation method,
which is treated with trifluoroacetic acid to yield an aldehyde of formula (XIX/a), which is a particular case of the compounds of formula (XIX):

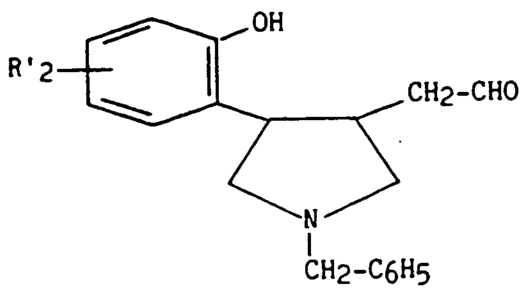

(XIX/a)

in which $R'_2$ is as defined above,
the diastereoisomers of which are separated, where appropriate, by a customary separation method,
which compound of formula (XIII) or (XIX) is reacted with a phosphorus ylid of formula (X), prepared by reaction of the corresponding phosphonium salt in the presence of potassium tert.-butanolate in tetrahydrofuran,

$$(C_6H_5)_3P{=}CH\text{-}(CH_2)_m\text{-}CO_2H \qquad (X)$$

in which m is as defined in formula (I),
to yield a compound of formula (I/d) or (I/e), respectively, which are a particular case of the compounds of formula (I):

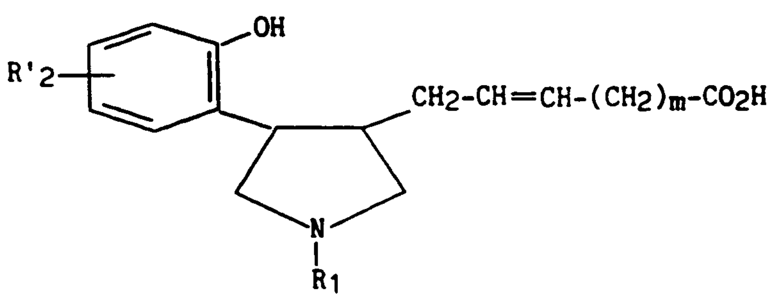

(I/d)

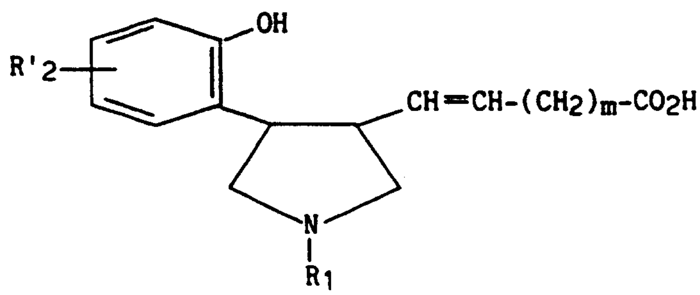

(I/e)

in which $R_1$ and $R'_2$ are as defined above, which compounds of formulae (I/d) and (I/e) are esterified, if desired, to yield the corresponding compounds of formulae (I/$d_1$) and (I/$e_1$):

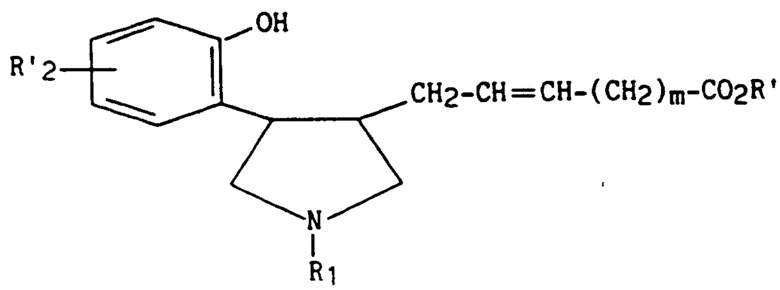

(I/$d_1$)

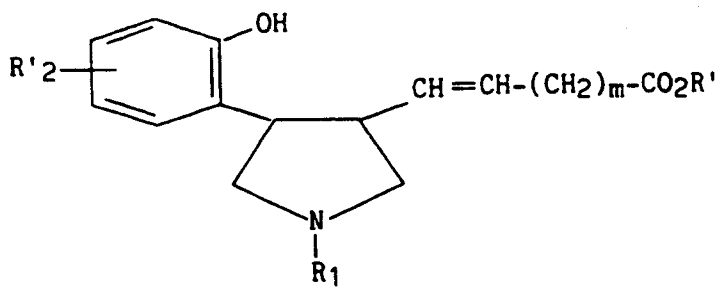

(I/$e_1$)

in which $R_1$ and $R'_2$ are as defined above and R' represents a linear or branched ($C_1$-$C_6$) alkyl group, the double bond of which is optionally reduced by catalytic hydrogenation to yield a compound of formula (I/f), which is a particular case of the compounds of formula (I):

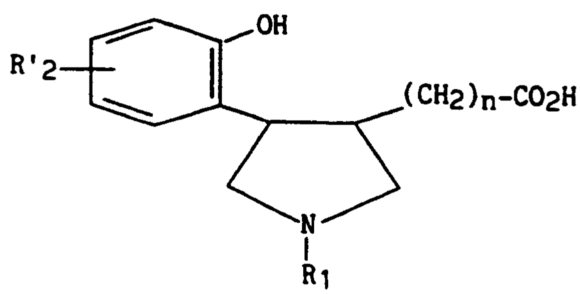

(I/f)

in which $R_1$ and $R'_2$ are as defined above, which is converted, if desired, into the corresponding ester of formula (I/$f_1$):

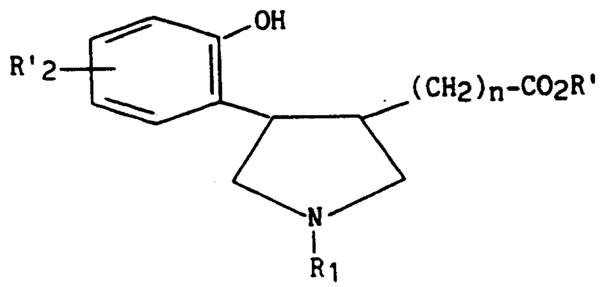

$(I/f_1)$

in which $R_1$, $R'_2$ and R' are as defined above, which compounds of formulae (I/d), $(I/d_1)$, (I/e), $(I/e_1)$, (I/f) and $(I/f_1)$ form the totality of the compounds of formula (I'),

- which, when $R'_2$ represents a linear or branched $(C_1-C_6)$alkoxy group, may be converted, if desired, into compounds of formula (I') in which $R'_2$ represents a hydroxy group,
- which are purified, where appropriate, by a customary purification method,
- the isomers of which are separated, where appropriate, by a customary purification method,
- and which are converted, if desired, into their addition salts with a pharmaceutically acceptable acid or base.

**11.** Pharmaceutical compositions comprising as active ingredient at least one compound according to any one of claims 1 to 3, on its own or in combination with one or more inert, non-toxic, pharmaceutically acceptable carriers.

**12.** Pharmaceutical compositions according to claim 5 comprising at least one active ingredient according to any one of claims 1 to 3, for use as antagonists of thromboxane $A_2$ receptors or as inhibitors of thromboxane $A_2$ synthase.

**Claims for the following Contracting State : ES**

**1.** Process for the preparation of compounds of formula (I):

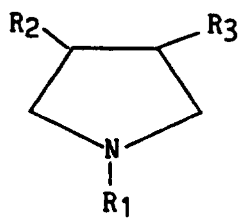

(I)

in which:

$R_1$ represents:

- a linear or branched $(C_1-C_6)$alkyl group that is unsubstituted or substituted by a pyridin-2-yl, pyridin-3-yl or phenyl group (which is itself optionally substituted by one or more halogen atoms or linear or branched $(C_1-C_6)$alkyl, linear or branched $(C_1-C_6)$alkoxy or trihalomethyl groups),
- a phenyl group that is unsubstituted or substituted by one or more halogen atoms or linear or branched $(C_1-C_6)$alkyl, linear or branched $(C_1-C_6)$alkoxy or trihalomethyl groups,
- a pyridyl group,
- a phenylsulphonyl group (that is unsubstituted or substituted on the phenyl ring by one or more halogen atoms or linear or branched $(C_1-C_6)$ alkyl, linear or branched $(C_1-C_6)$alkoxy or trihalomethyl groups), a naphthylsulphonyl group, a quinolylsulphonyl group or an isoquinolylsulphonyl group,
- a linear or branched $(C_1-C_6)$acyl group,

- a benzoyl or pyridylcarbonyl group that is unsubstituted or substituted on the aromatic ring by one or more halogen atoms or linear or branched ($C_1$-$C_6$)alkyl, linear or branched ($C_1$-$C_6$)alkoxy or trihalomethyl groups,
- an alkylaminocarbonyl or phenylaminocarbonyl group, or
- an acylamino or benzoylamino group;

$R_2$ represents:

- a hydrogen atom,
- a phenyl group that is unsubstituted or substituted by one or more halogen atoms or linear or branched ($C_1$-$C_6$)alkyl, linear or branched ($C_1$-$C_6$)alkoxy, hydroxy or trihalomethyl groups, or
- a pyridin-3-yl or pyridin-2-yl group that is unsubstituted or substituted on the pyridine ring by one or more halogen atoms or linear or branched ($C_1$-$C_6$)alkyl, linear or branched ($C_1$-$C_6$)alkoxy or trihalomethyl groups; and

$R_3$ represents any one of the following groups:

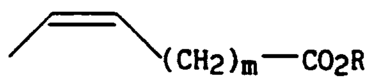

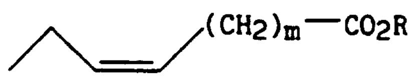

$$-(CH_2)_n-CO_2R$$

in which

m is 2, 3 or 4,
n is 4, 5, 6 or 7, and
R represents a hydrogen atom or a linear or branched ($C_1$-$C_6$)alkyl group;

their enantiomers, diastereoisomers and epimers, as well as addition salts thereof with a pharmaceutically acceptable acid or base,
characterised in that there is used as starting material a pyrrolidine of formula (II):

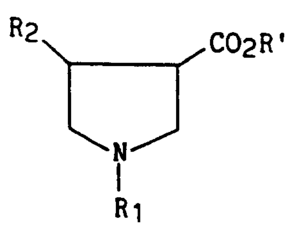

(II),

in which $R_1$ and $R_2$ are as defined in formula (I) and R' represents a linear or branched ($C_1$-$C_6$)alkyl group, which, in racemic or isomeric form, may, when in the form of a pair of enantiomers, be converted into its other pair of enantiomers by epimerisation in a sodium alcoholate,
which compound of formula (II) is reacted:

1) **either** with lithium aluminium hydride in an inert solvent to yield a pyrrolidine of formula (III):

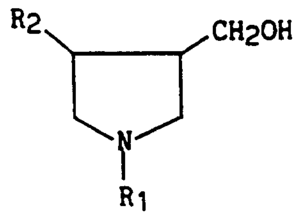

(III)

in which $R_1$ and $R_2$ are as defined in formula (I), which is reacted:

- <u>either</u> with gaseous hydrogen chloride in the presence of thionyl chloride in a chloroform medium to yield a pyrrolidine of formula (IV):

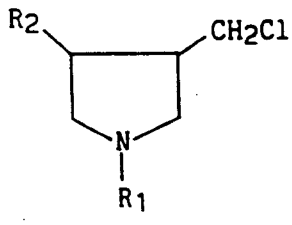

(IV)

in which $R_1$ and $R_2$ are as defined in formula (I), which is converted in the presence of tert.-butylammonium cyanide in dimethylformamide into a pyrrolidine of formula (V):

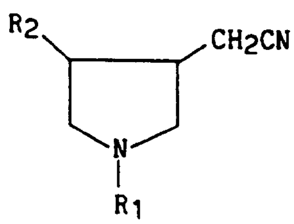

(V)

in which $R_1$ and $R_2$ are as defined in formula (I),

- <u>or</u> with para-toluenesulphonyl chloride in pyridine to yield a pyrrolidine of formula (VI):

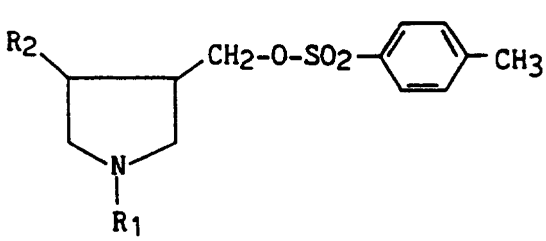

(VI)

in which $R_1$ and $R_2$ are as defined in formula (I),
which is converted into a pyrrolidine of formula (V) described above in the presence of potassium cyanide in dimethyl sulphoxide,
which compound of formula (V) is reduced by means of diisobutylaluminium hydride to yield an aldehyde of formula (VII):

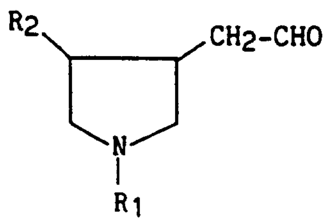

(VII)

in which $R_1$ and $R_2$ are as defined in formula (I),
2) **or** with diisobutylaluminium hydride to yield an aldehyde of formula (VIII):

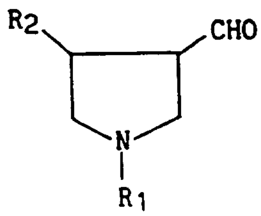

(VIII)

in which $R_1$ and $R_2$ are as defined in formula (I), which is, if desired, reacted with the phosphorus ylid of formula (IX), prepared by reaction of the corresponding phosphonium salt in the presence of potassium tert.-butanolate in tetrahydrofuran,

$$(C_6H_5)_3P{=}CHOCH_3 \qquad (IX)$$

to yield a compound of formula (X):

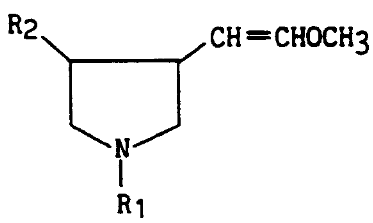

(X)

in which $R_1$ and $R_2$ are as defined in formula (I), which is treated with trifluoroacetic acid to yield an aldehyde of formula (VII) described above, which compound of formula (VII) or (VIII) is reacted with a phosphorus ylid of formula (XI), prepared by reaction of the corresponding phosphonium salt in the presence of potassium tert.-butanolate in tetrahydrofuran,

$$(C_6H_5)_3P{=}CH\text{-}(CH_2)_m\text{-}CO_2H \qquad (XI)$$

in which m is as defined in formula (I), to yield a compound of formula (I/a) or (I/b), respectively, which are a particular case of the compounds of formula (I):

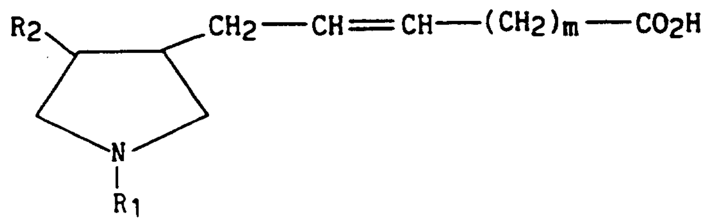

(I/a)

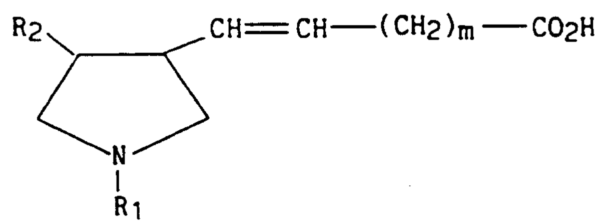

(I/b)

in which $R_1$, $R_2$ and m are as defined in formula (I),
which compounds of formulae (I/a) and (I/b) are esterified, if desired, to yield the corresponding compounds of formulae ($I/a_1$) and ($I/b_1$):

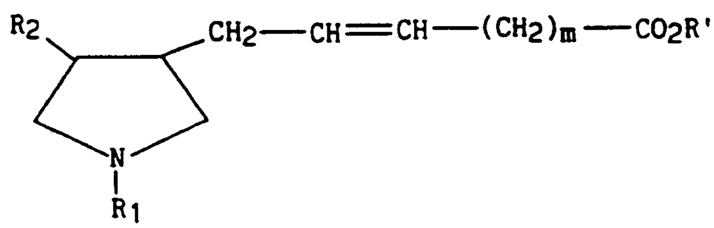

($I/a_1$)

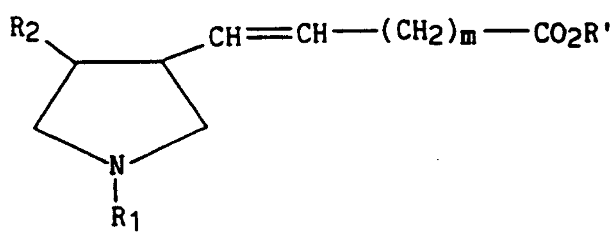

($I/b_1$)

in which $R_1$ and $R_2$ are as defined in formula (I) and R' represents a linear or branched ($C_1$-$C_6$)alkyl group, the double bond of which is optionally reduced by catalytic hydrogenation to yield a compound of formula (I/c), which is a particular case of the compounds of formula (I):

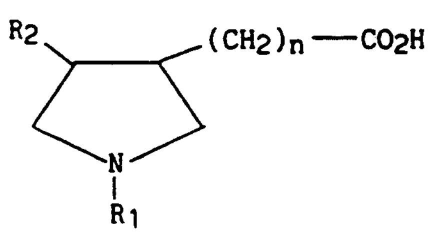

(I/c)

in which $R_1$, $R_2$ and n are as defined in formula (I),
which compound of formula (I/c) is converted, if desired, into the corresponding ester of formula ($I/c_1$):

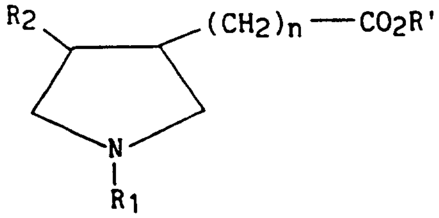

(I/c1)

in which $R_1$ and $R_2$ are as defined in formula (I) and R' represents a linear or branched ($C_1$-$C_6$)alkyl group, which compounds of formulae (I/a), (I/$a_1$), (I/b), (I/$b_1$), (I/c) and (I/$c_1$) form the totality of the compounds of formula (I),

- which, when $R_2$ represents a phenyl ring substituted by a linear or branched ($C_1$-$C_6$)alkoxy group, may be converted, if desired, into compounds of formula (I) in which $R_2$ represents a phenyl ring substituted by a hydroxy group,
- which are purified, where appropriate, by a customary purification method,
- the isomers of which are separated, where appropriate, by a customary purification method,
- and which are converted, if desired, into their addition salts with a pharmaceutically acceptable acid or base.

**2.** Process for the preparation of compounds of formula (I) according to claim 1 in which $R_1$ represents an unsubstituted or substituted phenylsulphonyl group, their enantiomers, diastereoisomers and epimers, as well as addition salts thereof with a pharmaceutically acceptable acid or base.

**3.** Process for the preparation of compounds of formula (I) according to claim 1 in which $R_2$ represents a hydroxyphenyl group, their enantiomers, diastereoisomers and epimers, as well as addition salts thereof with a pharmaceutically acceptable acid or base.

**4.** Process for the preparation of compounds of formula (I) according to claim 1 in which $R_1$ represents a naphthylsulphonyl group, their enantiomers, diastereoisomers and epimers, as well as addition salts thereof with a pharmaceutically acceptable acid or base.

**5.** Process for the preparation of compounds of formula (I) according to claim 1 in which $R_1$ represents an isoquinolylsulphonyl group, their enantiomers, diastereoisomers and epimers, as well as addition salts thereof with a pharmaceutically acceptable acid or base.

**6.** Process for the preparation of the compound of formula (I) according to claim 1 that is (4Z)-6-[(3α,4α)-1-(4-chlorophenylsulphonyl)-4-(2-hydroxyphenyl)pyrrolidin-3-yl]hex-4-enoic acid, as well as its addition salts with a pharmaceutically acceptable base.

**7.** Process for the preparation of the compound of formula (I) according to claim 1 that is (4Z)-6-[(3α,4α)-1-[(isoquinol-5-yl)sulphonyl]-4-(2-hydroxyphenyl)pyrrolidin-3-yl]hex-4-enoic acid, as well as its addition salts with a pharmaceutically acceptable base.

**8.** Process for the preparation of the compound of formula (I) according to claim 1 that is (4Z)-6-[(3α,4α)-1-(naphth-1-ylsulphonyl)-4-(2-hydroxyphenyl)pyrrolidin-3-yl]hex-4-enoic acid, as well as its addition salts with a pharmaceutically acceptable base.

**9.** Process for the preparation of compounds of formula (I'), which are a particular case of the compounds of formula (I) according to claim 1:

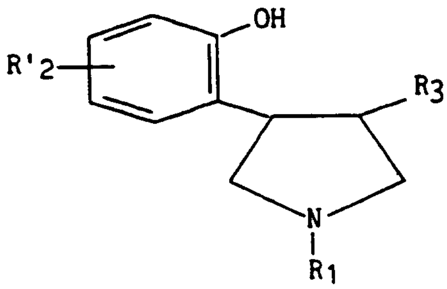

(I')

in which $R_1$ and $R_3$ are as defined in formula (I) and $R'_2$ represents a hydrogen atom, a halogen atom or a group linear or branched $(C_1-C_6)$alkyl, linear or branched $(C_1-C_6)$alkoxy or trihalomethyl, characterised in that there is used as starting material a compound of formula (XII):

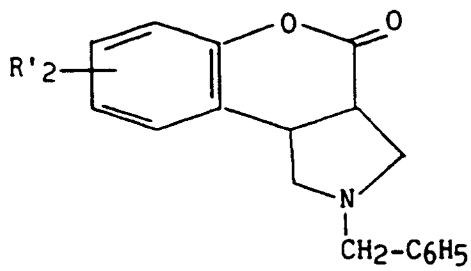

(XII)

in which $R'_2$ is as defined in formula (I'), which is reduced by means of diisobutylaluminium hydride to yield a compound of formula (XIII):

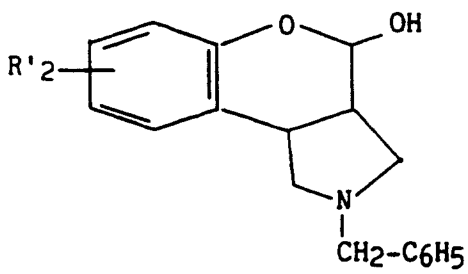

(XIII)

in which $R'_2$ is as defined above, which is:

**a either**

- either converted by catalytic hydrogenation (Pd/C) in dioxane into a compound of formula (XIV):

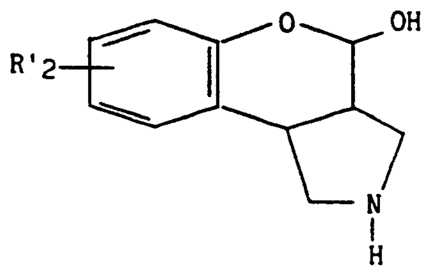

(XIV),

which is treated with a halogenated compound of the formula $R_1X$, in which $R_1$ is as defined in formula (I) and X represents a halogen atom, in chloroform in the presence of triethylamine to yield a compound of formula (XV):

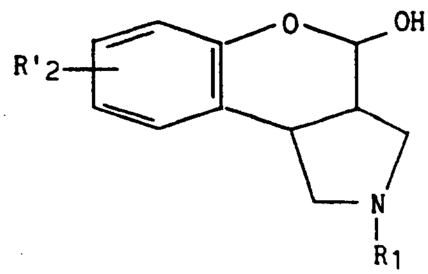

(XV)

in which $R'_2$ and $R_1$ are as defined above,

- or converted by catalytic hydrogenation (Pd/C) in a hydrochloric acid/ethanol medium into a compound of formula (XVI):

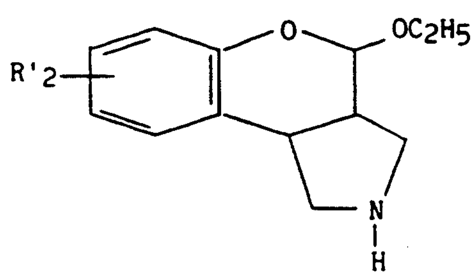

(XVI)

in which $R'_2$ is as defined above,
which is treated with a halogenated compound of the formula $R_1X$, described above, to yield a compound of formula (XVII):

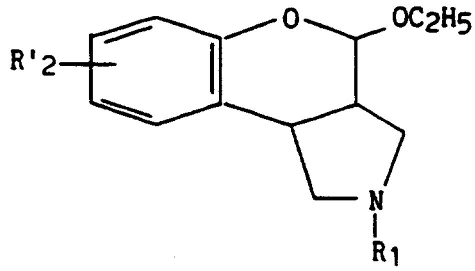

(XVII)

in which $R'_2$ and $R_1$ are as defined above,
which is treated with trifluoroacetic acid to yield a compound of formula (XV) described above,
which compound of formula (XV) is then reacted with the phosphorus ylid of formula (IX), prepared by reaction of the corresponding phosphonium salt in the presence of potassium tert.-butanolate in tetrahydrofuran,

$$(C_6H_5)_3P{=}CHOCH_3 \qquad (IX)$$

to yield a compound of formula (XVIII):

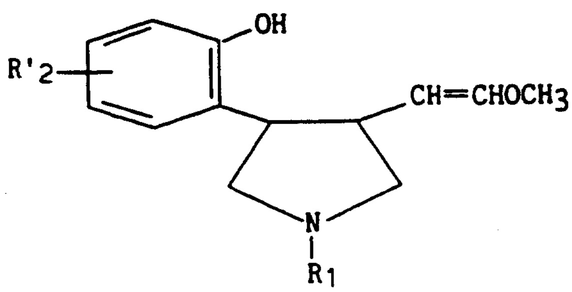

(XVIII)

in which $R_1$ and $R'_2$ are as defined above,
the diastereoisomers of which are separated, where appropriate, by a customary separation method,
which is treated with trifluoroacetic acid to yield an aldehyde of formula (XIX):

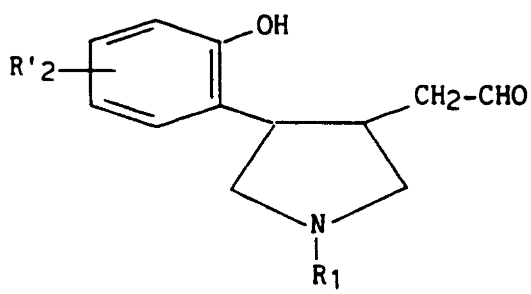

(XIX)

in which $R_1$ and $R'_2$ are as defined above,
the diastereoisomers of which are separated, where appropriate, by a customary separation method,

**b or** reacted with the phosphorus ylid of formula (IX) described above to yield a compound of formula (XX):

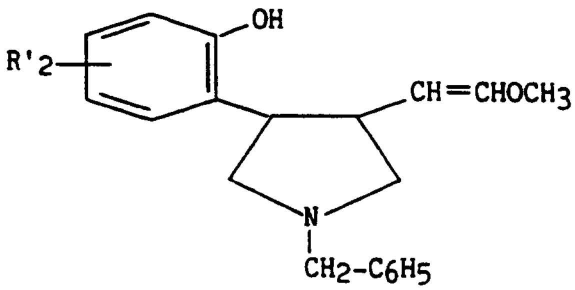

(XX)

in which $R'_2$ is as defined above,

the diastereoisomers of which are separated, where appropriate, by a customary separation method,

which is treated with trifluoroacetic acid to yield an aldehyde of formula (XIX/a), which is a particular case of the compounds of formula (XIX):

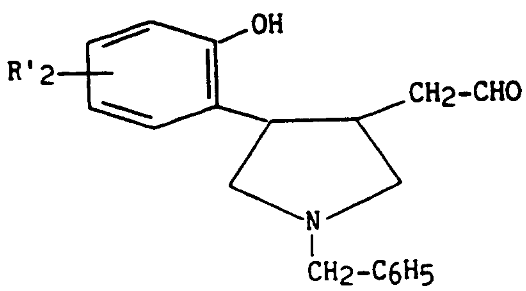

(XIX/a)

in which $R'_2$ is as defined above,

the diastereoisomers of which are separated, where appropriate, by a customary separation method,

which compound of formula (XIII) or (XIX) is reacted with a phosphorus ylid of formula (X), prepared by reaction of the corresponding phosphonium salt in the presence of potassium tert.-butanolate in tetrahydrofuran,

$$(C_6H_5)_3P=CH-(CH_2)_m-CO_2H \qquad (X)$$

in which m is as defined in formula (I),

to yield a compound of formula (I/d) or (I/e), respectively, which are a particular case of the compounds of formula (I):

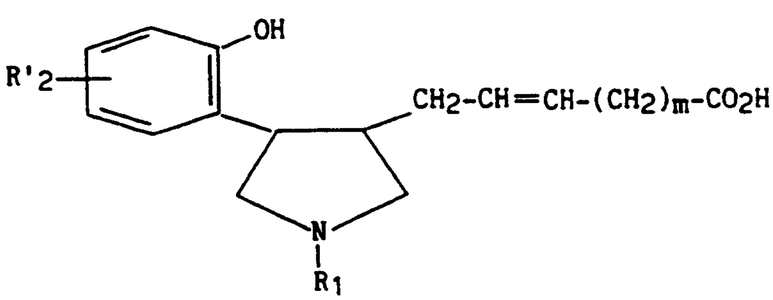

(I/d)

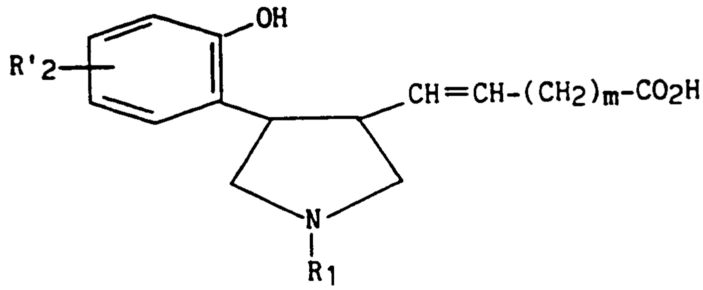

(I/e)

in which $R_1$ and $R'_2$ are as defined above, which compounds of formulae (I/d) and (I/e) are esterified, if desired, to yield the corresponding compounds of formulae (I/$d_1$) and (I/$e_1$):

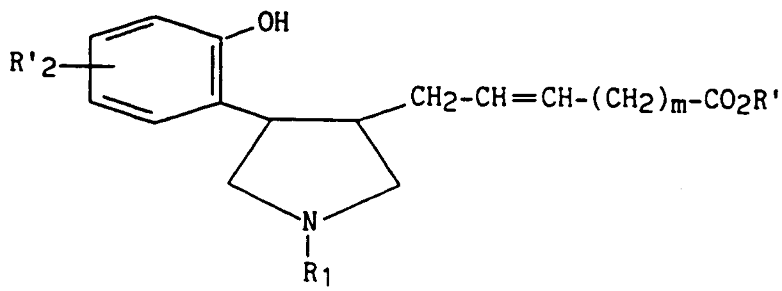

(I/$d_1$)

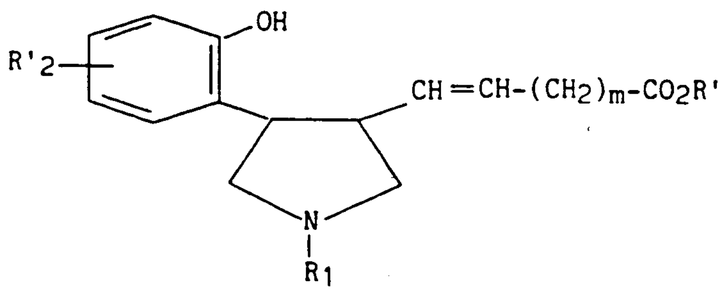

(I/$e_1$)

in which $R_1$ and $R'_2$ are as defined above and R' represents a linear or branched ($C_1$-$C_6$)alkyl group, the double bond of which is optionally reduced by catalytic hydrogenation to yield a compound of formula (I/f), which is a particular case of the compounds of formula (I):

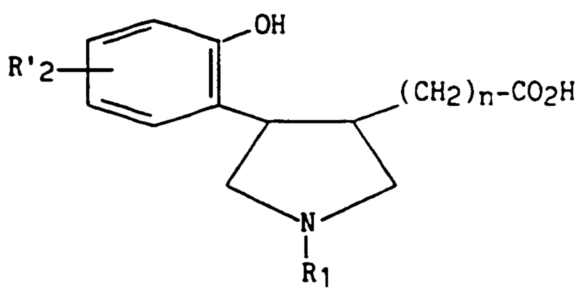

(I/f)

in which $R_1$ and $R'_2$ are as defined above, which is converted, if desired, into the corresponding ester of formula (I/$f_1$):

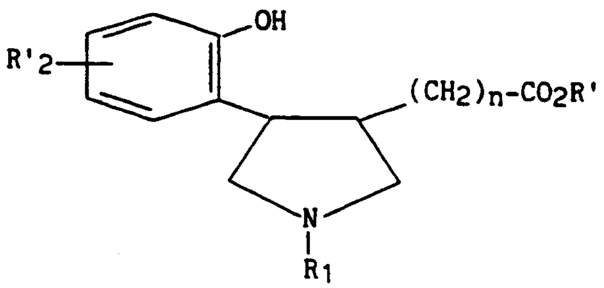

$(I/f_1)$

in which $R_1$, $R'_2$ and R' are as defined above, which compounds of formulae (I/d), $(I/d_1)$, (I/e), $(I/e_1)$, (I/f) and $(I/f_1)$ form the totality of the compounds of formula (I'),

- which, when $R'_2$ represents a linear or branched $(C_1$-$C_6)$alkoxy group, may be converted, if desired, into compounds of formula (I') in which $R'_2$ represents a hydroxy group,
- which are purified, where appropriate, by a customary purification method,
- the isomers of which are separated, where appropriate, by a customary purification method,
- and which are converted, if desired, into their addition salts with a pharmaceutically acceptable acid or base.

**Patentansprüche**

**Patentansprüche für folgende Vertragsstaaten : AT, BE, CH, DE, DK, FR, GB, GR, IE, IT, LI, LU, NL, PT, SE**

1. Verbindungen der Formel (I):

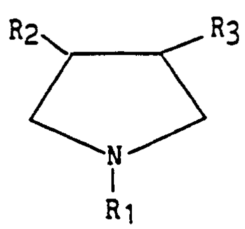

(I)

in der:

$R_1$:

- eine geradkettige oder verzweigte $(C_1$-$C_6)$-Alkylgruppe, die gegebenenfalls durch eine Pyridin-2-yl-gruppe, Pyridin-3-yl-gruppe oder Phenylgruppe (die ihrerseits gegebenenfalls substituiert ist durch eines oder mehrere Halogenatome oder geradkettige oder verzweigte $(C_1$-$C_6)$-Alkylgruppen, geradkettige oder verzweigte $(C_1$-$C_6)$-Alkoxygruppen oder Trihalogenmethylgruppen) substituiert ist,
- eine Phenylgruppe, die gegebenenfalls durch eines oder mehrere Halogenatome, geradkettige oder verzweigte $(C_1$-$C_6)$-Alkylgruppen, geradkettige oder verzweigte $(C_1$-$C_6)$-Alkoxygruppen oder Trihalogenmethylgruppen substituiert ist,
- eine Pyridylgruppe,
- eine Phenylsulfonylgruppe (die gegebenenfalls am Phenylkern durch eines oder mehrere Halogenatome oder geradkettige oder verzweigte $(C_1$-$C_6)$-Alkylgruppen, geradkettige oder verzweigte $(C_1$-$C_6)$-Alkoxygruppen oder Trihalogenmethylgruppen substituiert ist), eine Naphthylsulfonylgruppe, eine Chinolylsulfonylgruppe oder eine Isochinolylsulfonylgruppe,
- eine geradkettige oder verzweigte $(C_1$-$C_6)$-Acylgruppe,
- eine Benzoyl- oder Pyridylcarbonylgruppe, die gegebenenfalls am aromatischen Kern durch eines oder mehrere Halogenatome oder geradkettige oder verzweigte $(C_1$-$C_6)$-Alkylgruppen, geradkettige oder verzweigte $(C_1$-$C_6)$-Alkoxygruppen oder Trihalogenmethylgruppen substituiert ist,
- eine Alkylaminocarbonylgruppe oder Phenylaminocarbonylgruppe, oder

- eine Acylaminogruppe oder Benzoylaminogruppe,

$R_2$:

- ein Wasserstoffatom,
- eine Phenylgruppe, die gegebenenfalls durch eines oder mehrere Halogenatome, geradkettige oder verzweigte ($C_1$-$C_6$)-Alkylgruppen, geradkettige oder verzweigte ($C_1$-$C_6$)-Alkoxygruppen, Hydroxylgruppen oder Trihalogenmethylgruppen substituiert ist,
- eine Pyridin-3-yl-gruppe oder Pyridin-2-yl-gruppe, die gegebenenfalls am Pyridinkern durch eines oder mehrere Halogenatome oder geradkettige oder verzweigte ($C_1$-$C_6$)-Alkylgruppen, geradkettige oder verzweigte ($C_1$-$C_6$)-Alkoxygruppen oder Trihalogenmethylgruppen substituiert ist, und

$R_3$ eine der folgenden Gruppen:

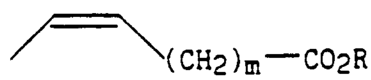

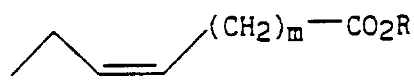

$$-(CH_2)_n-CO_2R$$

in denen

m 2, 3 oder 4,
n 4, 5, 6 oder 7 und
R ein Wasserstoffatom oder eine geradkettige oder verzweigte ($C_1$-$C_6$)-Alkylgruppe darstellen,

bedeuten,

deren Enantiomere, Diastereoisomere und Epimere sowie deren Additionssalze mit einer pharmazeutisch annehmbaren Säure oder Base.

2. Verbindungen der Formel (I) nach Anspruch 1, worin $R_1$ eine gegebenenfalls substituierte Phenylsulfonylgruppe darstellt, deren Enantiomere, Diastereoisomere und Epimere sowie deren Additionssalze mit einer pharmazeutisch annehmbaren Säure oder Base.

3. Verbindungen der Formel (I) nach Anspruch 1, in der $R_2$ eine Hydroxyphenylgruppe darstellt, deren Enantiomere, Diastereoisomere und Epimere sowie deren Additionssalze mit einer pharmazeutisch annehmbaren Säure oder Base.

4. Verbindungen der Formel (I) nach Anspruch 1, in der $R_1$ eine Naphthylsulfonylgruppe bedeutet, deren Enantiomere, Diastereoisomere und Epimere sowie deren Additionssalze mit einer pharmazeutisch annehmbaren Säure oder Base.

5. Verbindungen der Formel (I) nach Anspruch 1, in der $R_1$ eine Isochinolylsulfonylgruppe darstellt, deren Enantiomere, Diastereoisomere und Epimere sowie deren Additionssalze mit einer pharmazeutisch annehmbaren Säure oder Base.

6. Verbindung der Formel (I) nach Anspruch 1, nämlich (4Z)-6-[(3α,4α)-1-(4-Chlorphenylsulfonyl)-4-(2-hydroxyphenyl)-pyrrolidin-3-yl]-hex-4-ensäure, sowie deren Additionssalze mit einer pharmazeutisch annehmbaren Base.

7. Verbindung der Formel (I) nach Anspruch 1, nämlich (4Z)-6-[(3α,4α)-1-[(Isochinol-5-yl)-sulfonyl]-4-(2-hydroxyphenyl)-pyrrolidin-3-yl]-hex-4-ensäure, sowie deren Additionssalze mit einer pharmazeutisch annehmbaren Base.

**8.** Verbindung der Formel (I) nach Anspruch 1, nämlich (4Z)-6-[(3α,4α)-1-(Naphth-1-yl-sulfonyl)-4-(2-hydroxyphenyl)-pyrrolidin-3-yl]-hex-4-ensäure, sowie deren Additionssalze mit einer pharmazeutisch annehmbaren Base.

**9.** Verfahren zur Herstellung der Verbindungen der Formel (I) nach Anspruch 1, **dadurch gekennzeichnet**, daß man als Ausgangsprodukt ein Pyrrolidin der Formel (II) verwendet:

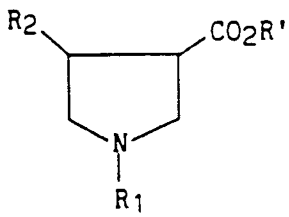

(II)

in der $R_1$ und $R_2$ die bezüglich der Formel (I) angegebenen Bedeutungen besitzen und R' eine geradkettige oder verzweigte ($C_1$-$C_6$)-Alkylgruppe darstellt, welche Verbindung der Formel (II), die in Form des Racemats oder der Isomeren vorliegen kann, und die dann, wenn sie in Form eines Enantiomerenpaars vorliegt, durch Epimerisierung in einem Natriumalkoholat in das andere Enantiomerenpaar umgewandelt werden kann,
welche man:

**1** **<u>entweder</u>** mit Lithiumaluminiumhydrid in einem inerten Lösungsmittel umsetzt zur Bildung des Pyrrolidins der Formel (III):

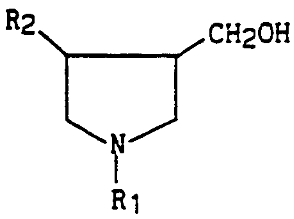

(III)

in der $R_1$ und $R_2$ die bezuglich der Formel (I) angegebenen Bedeutungen besitzen, welches man:

- <u>entweder</u> mit gasförmiger Chlorwasserstoffsäure in Gegenwart von Thionylchlorid in Chloroformmedium umsetzt zur Bildung des Pyrrolidins der Formel (IV):

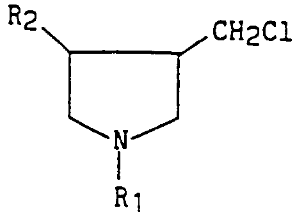

(IV)

in der $R_1$ und $R_2$ die bezüglich der Formel (I) angegebenen Bedeutungen besitzen,

welches man in Gegenwart von tert.-Butylammoniumcyanid in Dimethylformamid in das Pyrrolidin der Formel (V) umwandelt:

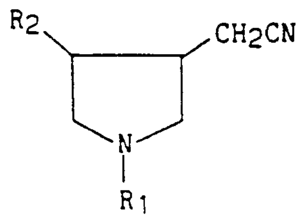

(V)

in der $R_1$ und $R_2$ die bezüglich der Formel (I) angegebenen Bedeutungen besitzen,

- oder mit p-Toluolsulfonylchlorid in Pyridin umsetzt zur Bildung des Pyrrolidins der Formel (VI):

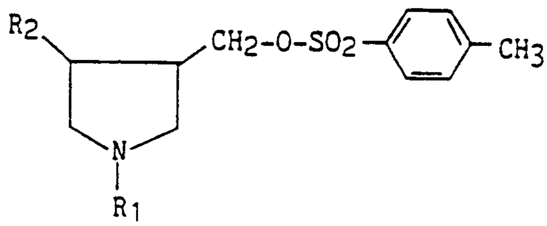

(VI)

in der $R_1$ und $R_2$ die bezuglich der Formel (I) angegebenen Bedeutungen besitzen,

welches man in Gegenwart von Kaliumcyanid in Dimethylsulfoxid in das oben beschriebene Pyrrolidin der Formel (V) umwandelt,
welches Derivat der Formel (V) man mit Diisobutylaluminiumhydrid reduziert zur Bildung des Aldehyds der Formel (VII):

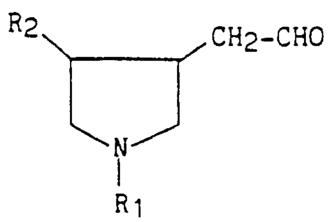

(VII)

in der $R_1$ und $R_2$ die bezuglich der Formel (I) angegebenen Bedeutungen besitzen,
**2 oder** mit Diisobutylaluminiumhydrid umsetzt zur Bildung des Aldehyds der Formel (VIII):

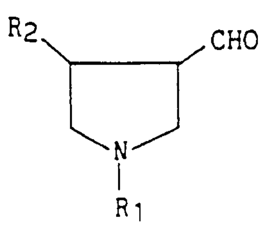

(VIII)

in der $R_1$ und $R_2$ die bezuglich der Formel (I) angegebenen Bedeutungen besitzen, welchen man gewünschtenfalls mit dem Phosphorylid der Formel (IX)

$$(C_6H_5)_3P = CHOCH_3 \qquad (IX)$$

das man durch Reaktion des entsprechenden Phosphoniumsalzes hergestellt hat, in Gegenwart von Kalium-tert.-butanolat in Tetrahydrofuran umsetzt zur Bildung des Verbindung der Formel (X):

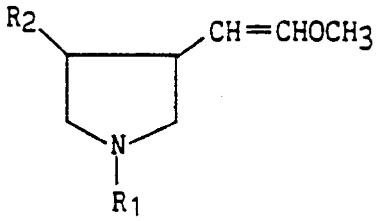

(X)

in der $R_1$ und $R_2$ die bezuglich der Formel (I) angegebenen Bedeutungen besitzen, welche man mit Trifluoressigsäure behandelt zur Bildung des oben beschriebenen Aldehyds der Formel (VII),
welche Verbindungen der Formel (VII) oder (VIII) man mit einem Phosphorylid der Formel (XI)

$$(C_6H_5)_3P = CH—(CH_2)_m—CO_2H \qquad \text{(XI)}$$

in der m die bezuglich der Formel (I) angegebenen Bedeutungen besitzt, das man durch Reaktion des entsprechenden Phosphoniumsalzes hergestellt hat, in Gegenwart von Kalium-tert.-butanolat in Tetrahydrofuran umsetzt, so daß man die Verbindungen der Formel (I/a) bzw. (I/b) erhält, einem Sonderfall der Verbindungen der Formel (I):

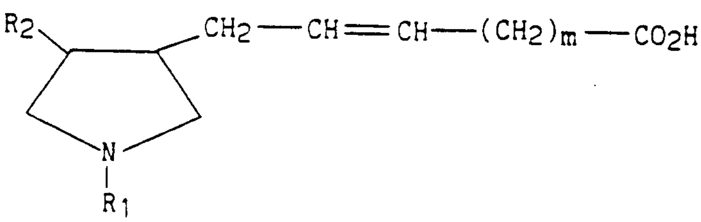

(I/a)

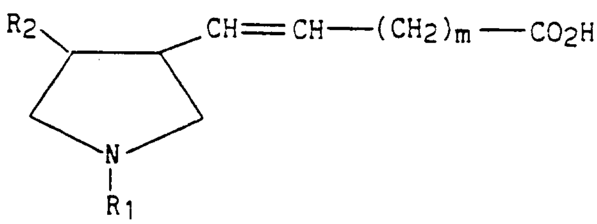

(I/b)

worin $R_1$, $R_2$ und m die bezuglich der Formel (I) angegebenen Bedeutungen besitzen,
welche Verbindungen der Formeln (I/a) oder (I/b)

- man gewünschtenfalls verestert zur Bildung der entsprechenden Verbindungen der Formel ($I/a_1$) und ($I/b_1$):

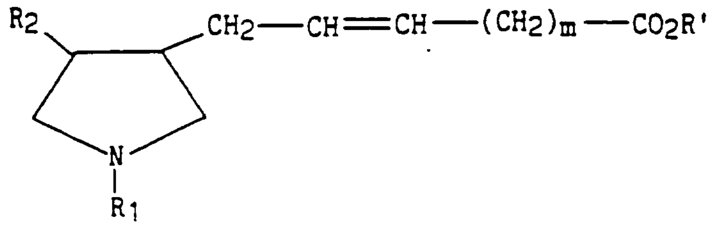

(I/a1)

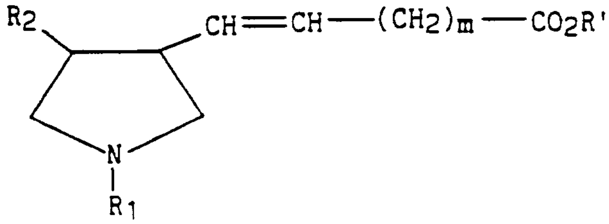

(I/b1)

worin $R_1$ und $R_2$ die bezuglich der Formel (I) angegebenen Bedeutungen besitzen und R' eine geradkettige oder verzweigte ($C_1$-$C_6$)-Alkylgruppe darstellt,

- bei denen man gegebenenfalls die Doppelbindung durch katalytische Hydrierung reduziert zur Bildung der Verbindung der Formel (I/c), einem Sonderfall der Verbindungen der Formel (I):

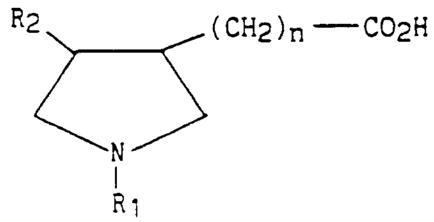

(I/c)

in der $R_1$, $R_2$ und n die bezuglich der Formel (I) angegebenen Bedeutungen besitzen, welche man gewünschtenfalls in den entsprechenden Ester der Formel (I/$c_1$) umwandelt:

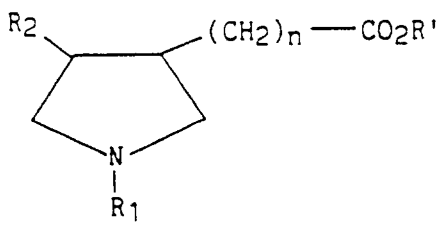

(I/c1)

in der $R_1$ und $R_2$ die bezüglich der Formel (I) angegebenen Bedeutungen besitzen und R' eine geradkettige oder verzweigte ($C_1$-$C_6$)-Alkylgruppe darstellt, welche Verbindungen der Formeln (I/a), (I/$a_1$), (I/b), (I/$b_1$) (I/c) und (I/$c_1$) gemeinsam die Verbindungen der Formel (I) bilden,

- welche, wenn $R_2$ einen Phenylkern bedeutet, der durch eine geradkettige oder verzweigte ($C_1$-$C_6$)-Alkoxygruppe substituiert ist, gewünschtenfalls in die Verbindungen der Formel (I) umgewandelt werden können, in denen $R_2$ einen durch eine Hydroxylgruppe substituierten Phenylkern darstellt,
- welche man gegebenenfalls mit Hilfe einer klassischen Reinigungsmethode reinigt,
- welche man gegebenenfalls mit Hilfe einer klassischen Reinigungsmethode in ihre Isomeren auftrennt und
- welche man gewünschtenfalls mit einer pharmazeutisch annehmbaren Säure oder Base in ihre Additionssalze umwandelt.

**10.** Verfahren zur Herstellung der Verbindungen der Formel (I'), einem Sonderfalls der Verbindungen der Formel (I) nach Anspruch 1:

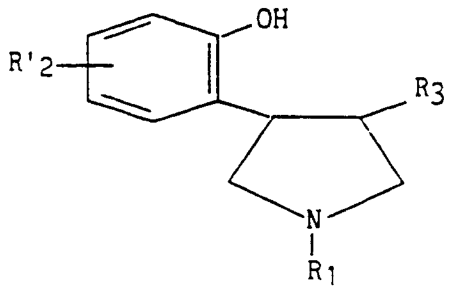

(I')

in der $R_1$ und $R_3$ die bezüglich der Formel (I) angegebenen Bedeutungen besitzen und $R'_2$ ein Wasserstoffatom, ein Halogenatom, eine geradkettige oder verzweigte ($C_1$-$C_6$)-Alkylgruppe, eine geradkettige oder verzweigte ($C_1$-$C_6$)-Alkoxygruppe oder eine Trihalogenmethylgruppe bedeutet,
welche man ebenfalls unter Anwendung eines Verfahrens erhalt, das **dadurch gekennzeichnet** ist, daß man als Ausgangsprodukt die Verbindung der Formel (XII) verwendet:

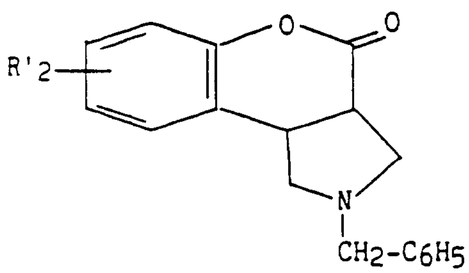

(XII)

in der $R'_2$ die bezüglich der Formel (I') angegebenen Bedeutungen besitzt, welche man mit Diisobutylaluminiumhydrid reduziert zur Bildung der Verbindung der Formel (XIII):

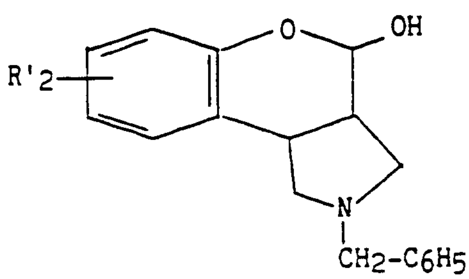

(XIII)

in der $R'_2$ die oben angegebenen Bedeutungen besitzt, welche man:

**a entweder:**

- entweder durch katalytische Hydrierung (Pd/C) in Dioxan in die Verbindung der Formel (XIV) umwandelt:

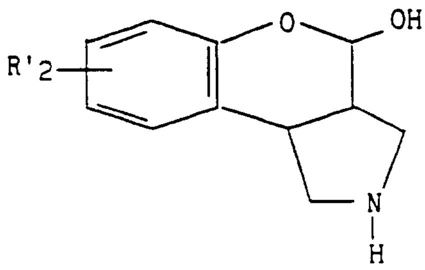

(XIV)

welche man mit einem Halogenderivat der Formel $R_1X$, in der $R_1$ die bezüglich der Formel (I) angegebenen Bedeutungen besitzt und X ein Halogenatom darstellt, in Chloroform in Gegenwart von Triethylamin behandelt, zur Bildung der Verbindung der Formel (XV):

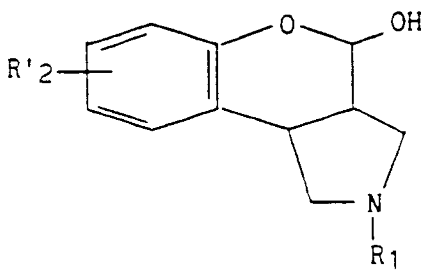

(XV)

in der $R'_2$ und $R_1$ die oben angegebenen Bedeutungen besitzen,

- oder durch katalytische Hydrierung (Pd/C) in einem Chlorwasserstoffsäure/Ethanol-Medium in die Verbindung der Formel (XVI) umwandelt:

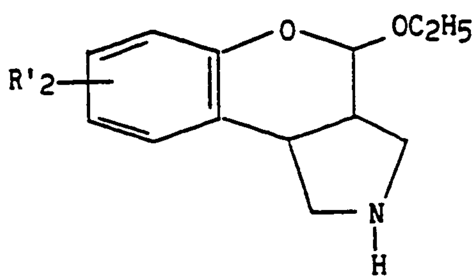

(XVI)

In der $R'_2$ die oben angegebenen Bedeutungen besitzt,
welche man mit einem Halogenderivat der oben beschriebenen Formel $R_1X$ behandelt zur Bildung der Verbindung der Formel (XVII):

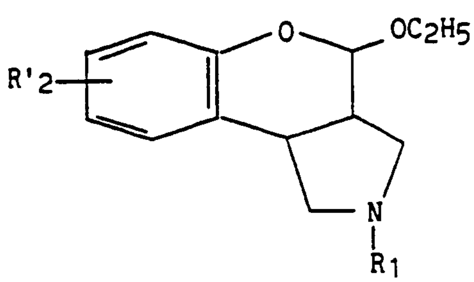

(XVII)

in der $R'_2$ und $R_1$ die oben angegebenen Bedeutungen besitzen,
welche man mit Trifluoressigsäure behandelt zur Bildung der oben beschriebenen Verbindung der Formel (XV),
welches Derivat der Formel (XV) man anschließend mit dem durch Reaktion des entsprechenden Phosphoniumsalzes gebildeten Phosphorylid der Formel (IX):

$$(C_6H_5)_3P = CHOCH_3 \qquad (IX)$$

in Gegenwart von Kalium-tert.-butanolat in Tetrahydrofuran umsetzt zur Bildung der Verbindung der Formel (XVIII):

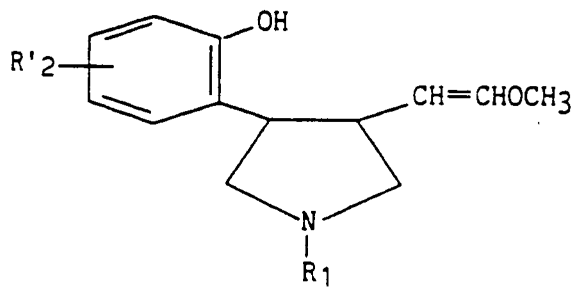

(XVIII)

in der $R_1$ und $R'_2$ die oben angegebenen Bedeutungen besitzen,
welche man gegebenenfalls mit Hilfe einer klassischen Trennungsmethode in ihre Diastereoisomeren auftrennt,
welche man mit Trifluoressigsäure behandelt zur Bildung des Aldehyds der Formel (XIX):

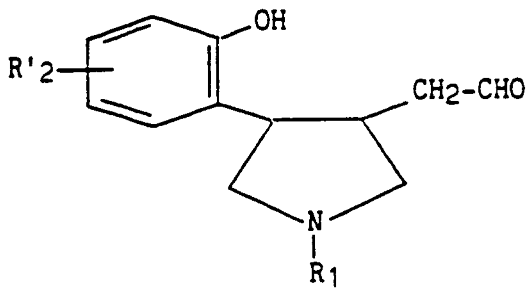

(XIX)

in der $R_1$ und $R'_2$ die oben angegebenen Bedeutungen besitzen,
welche man gegebenenfalls mit Hilfe einer klassischen Trennungsmethode in die Diastereoisomeren auftrennt,
**b** **oder** mit dem oben beschriebenen Phosphorylid der Formel (IX) umsetzt zur Bildung der Verbindung der Formel (XX):

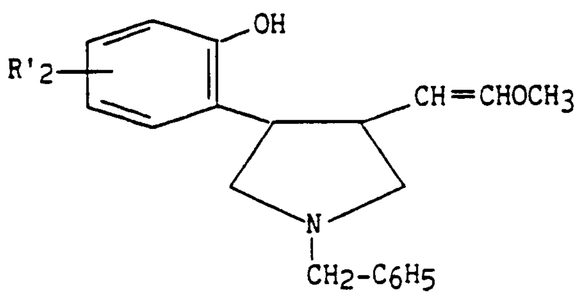

(XX)

in der $R'_2$ die oben angegebenen Bedeutungen besitzt,
welche man gegebenenfalls mit Hilfe einer klassischen Trennungsmethode in die Diastereoisomeren auftrennt,

und mit Trifluoressigsäure behandelt zur Bildung des Aldehyds der Formel (XIX/a), einem Sonderfall der Verbindungen der Formel (XIX):

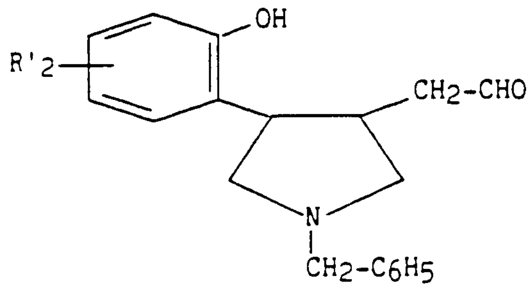

(XIX/a)

in der $R'_2$ die oben angegebenen Bedeutungen besitzt,
welchen man gegebenenfalls mit Hilfe einer klassischen Trennungsmethode in die Diastereoisomeren auftrennt,
welche Verbindungen der Formel (XIII) oder (XIX) man mit einem Phosphorylid der Formel (X):

$$(C_6H_5)_3P = CH\text{-}(CH_2)_m\text{-}CO_2H \qquad (X)$$

in der m die bezüglich der Formel (I) angegebenen Bedeutungen besitzt, das durch Reaktion des entsprechenden Phosphoniumsalzes hergestellt wordenist, in Gegenwart von Kalium-tert.-butanolat in Tetrahydrofuran umsetzt, zur Bildung der Verbindungen der Formeln (I/d) bzw. (I/e), einem Sonderfall der Verbindungen der Formel (I):

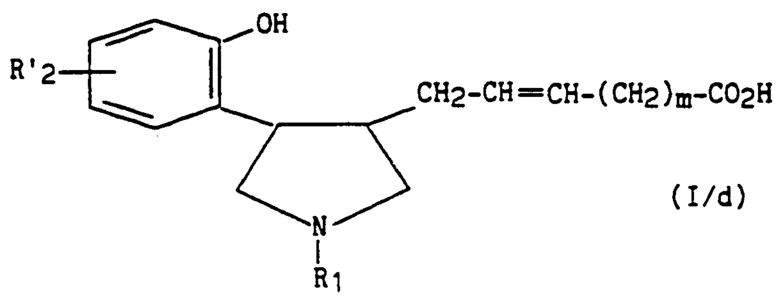

(I/d)

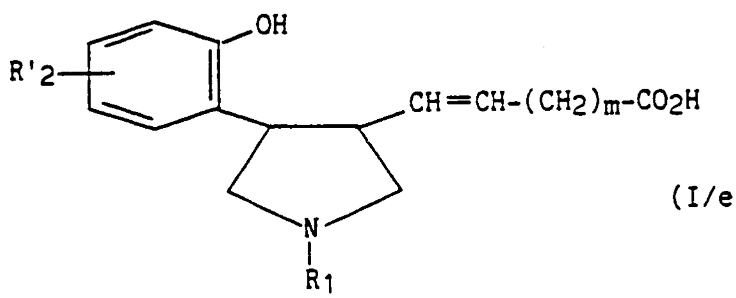

(I/e)

in denen $R_1$ und $R'_2$ die oben angegebenen Bedeutungen besitzen,

- welche man gewünschtenfalls verestert zur Bildung der entsprechenden Verbindungen der Formeln ($I/d_1$) und ($I/e_1$):

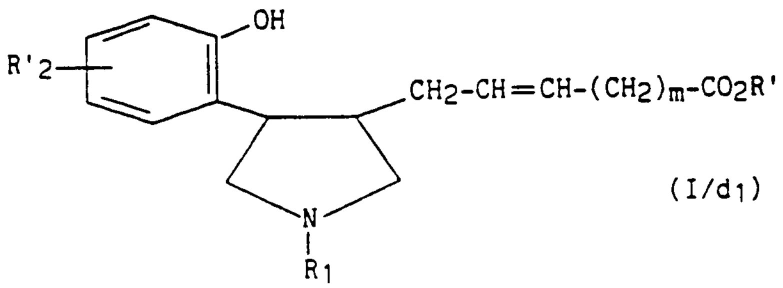

(I/d1)

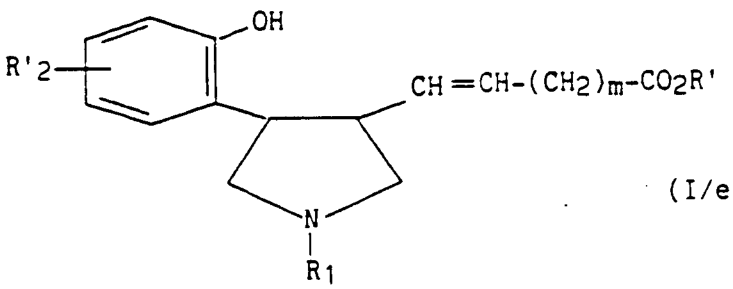

(I/e1)

in denen $R_1$ und $R'_2$ die oben angegebenen Bedeutungen besitzen und R' eine geradkettige oder verzweigte ($C_1$-$C_6$)-Alkylgruppe darstellt,

- dessen Doppelbindung man gegebenenfalls durch katalytische Hydrierung reduziert zur Bildung der Verbindung der Formel (I/f), einem Sonderfall der Verbindungen der Formel (I):

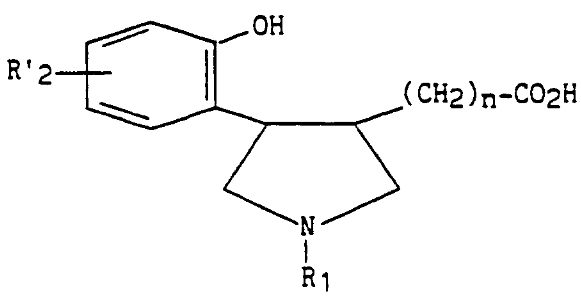

(I/f)

in der $R_1$ und $R'_2$ die oben angegebenen Bedeutungen besitzen,
welche man gewünschtenfalls in die entsprechenden Ester der Formel (I/$f_1$) umwandelt:

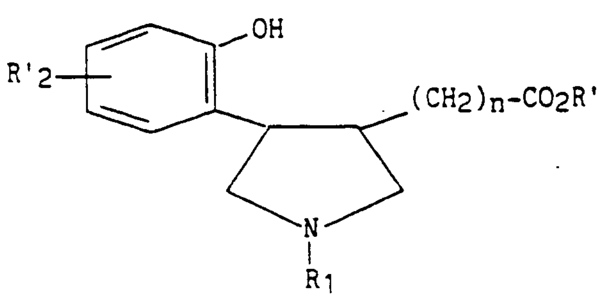

(I/$f_1$)

in der $R_1$, $R'_2$ und R' die oben angegebenen Bedeutungen besitzen,

welche Verbindungen der Formeln (I/d), (I/$d_1$), (I/e), (I/$e_1$), (I/f) und (I/$f_1$) gemeinsam die Verbindungen der Formel (I') darstellen,

- welche, wenn $R'_2$ eine geradkettige oder verzweigte ($C_1$-$C_6$)-Alkoxygruppe darstellt, gewünschtenfalls in die Verbindungen der Formel (I') umgewandelt werden können, in der $R'_2$ eine Hydroxylgruppe darstellt,
- welche man gegebenenfalls mit Hilfe einer klassischen Reinigungsmethode reinigt,
- welche man gegebenenfalls mit Hilfe einer klassischen Reinigungsmethode in die Isomeren auftrennt und
- welche man gewünschtenfalls mit einer pharmazeutisch annehmbaren Säure oder Base in ihre Additionssalze umwandelt.

**11.** Pharmazeutische Zubereitungen enthaltend als Wirkstoff mindestens eine Verbindung nach irgendeinem der Ansprüche 1 bis 3 allein oder in Kombination mit einem oder mehreren inerten, nichttoxischen, pharmazeutisch annehmbaren Trägermaterialien oder Bindemitteln.

**12.** Pharmazeutische Zubereitung nach Anspruch 5 enthaltend mindestens einen Wirkstoff nach einem der Ansprüche 1 bis 3 zur Verwendung als Antagonisten der Rezeptoren des Thromboxans $A_2$ oder als Inhibitoren der Thromboxan-$A_2$-Synthase.

**Patentansprüche für folgenden Vertragsstaat : ES**

**1.** Verfahren zur Herstellung der Verbindungen der Formel (I):

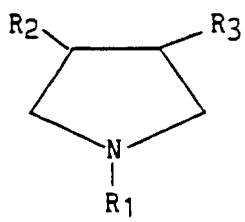

(I)

in der:

$R_1$:

- eine geradkettige oder verzweigte ($C_1$-$C_6$)-Alkylgruppe, die gegebenenfalls durch eine Pyridin-2-yl-gruppe, Pyridin-3-yl-gruppe oder Phenylgruppe (die ihrerseits gegebenenfalls substituiert ist durch eines oder mehrere Halogenatome oder geradkettige oder verzweigte ($C_1$-$C_6$)-Alkylgruppen, geradkettige oder verzweigte ($C_1$-$C_6$)-Alkoxygruppen oder Trihalogenmethylgruppen) substituiert ist,
- eine Phenylgruppe, die gegebenenfalls durch eines oder mehrere Halogenatome, geradkettige oder verzweigte ($C_1$-$C_6$)-Alkylgruppen, geradkettige oder verzweigte ($C_1$-$C_6$)-Alkoxygruppen oder Trihalogenmethylgruppen substituiert ist,
- eine Pyridylgruppe,
- eine Phenylsulfonylgruppe (die gegebenenfalls am Phenylkern durch eines oder mehrere Halogenatome oder geradkettige oder verzweigte ($C_1$-$C_6$)-Alkylgruppen, geradkettige oder verzweigte ($C_1$-$C_6$)-Alkoxygruppen oder Trihalogenmethylgruppen substituiert ist), eine Naphthylsulfonylgruppe, eine Chinolylsulfonylgruppe oder eine Isochinolylsulfonylgruppe,
- eine geradkettige oder verzweigte ($C_1$-$C_6$)-Acylgruppe,
- eine Benzoyl- oder Pyridylcarbonylgruppe, die gegebenenfalls am aromatischen Kern durch eines oder mehrere Halogenatome oder geradkettige oder verzweigte ($C_1$-$C_6$)-Alkylgruppen, geradkettige oder verzweigte ($C_1$-$C_6$)-Alkoxygruppen oder Trihalogenmethylgruppen substituiert ist,
- eine Alkylaminocarbonylgruppe oder Phenylaminocarbonylgruppe, oder
- eine Acylaminogruppe oder Benzoylaminogruppe,

$R_2$:

- ein Wasserstoffatom,
- eine Phenylgruppe, die gegebenenfalls durch eines oder mehrere Halogenatome, geradkettige oder verzweigte ($C_1$-$C_6$)-Alkylgruppen, geradkettige oder verzweigte ($C_1$-$C_6$)-Alkoxygruppen, Hydroxylgruppen

oder Trihalogenmethylgruppen substituiert ist,

- eine Pyridin-3-yl-gruppe oder Pyridin-2-yl-gruppe, die gegebenenfalls am Pyridinkern durch eines oder mehrere Halogenatome oder geradkettige oder verzweigte ($C_1$-$C_6$)-Alkylgruppen, geradkettige oder verzweigte ($C_1$-$C_6$)-Alkoxygruppen oder Trihalogenmethylgruppen substituiert ist, und

$R_3$ eine der folgenden Gruppen:

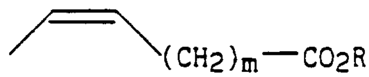

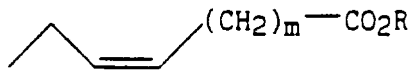

$-(CH_2)_n-CO_2R$

in denen

m 2, 3 oder 4,
n 4, 5, 6 oder 7 und
R ein Wasserstoffatom oder eine geradkettige oder verzweigte ($C_1$-$C_6$)-Alkylgruppe darstellen,

bedeuten,

und von deren Enantiomeren, Diastereoisomeren und Epimeren sowie von deren Additionssalzen mit einer pharmazeutisch annehmbaren Säure oder Base, **dadurch gekennzeichnet**, daß man als Ausgangsprodukt ein Pyrrolidin der Formel (II) verwendet:

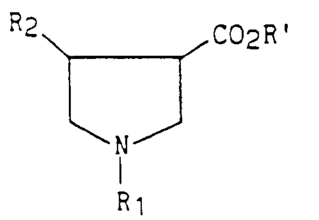

(II)

in der $R_1$ und $R_2$ die bezüglich der Formel (I) angegebenen Bedeutungen besitzen und R' eine geradkettige oder verzweigte ($C_1$-$C_6$)-Alkylgruppe darstellt, welche Verbindung der Formel (II), die in Form des Racemats oder der Isomeren vorliegen kann, und die dann, wenn sie in Form eines Enantiomerenpaars vorliegt, durch Epimerisierung in einem Natriumalkoholat in das andere Enantiomerenpaar umgewandelt werden kann, welche man:

**1** **entweder** mit Lithiumaluminiumhydrid in einem inerten Lösungsmittel umsetzt zur Bildung des Pyrrolidins der Formel (III):

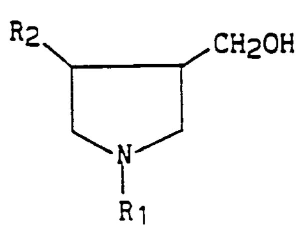

(III)

in der $R_1$ und $R_2$ die bezüglich der Formel (I) angegebenen Bedeutungen besitzen, welches man:

- entweder mit gasförmiger Chlorwasserstoffsäure in Gegenwart von Thionylchlorid in Chloroformmedium

umsetzt zur Bildung des Pyrrolidins der Formel (IV):

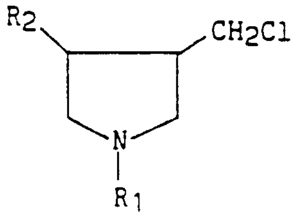

(IV)

in der $R_1$ und $R_2$ die bezüglich der Formel (I) angegebenen Bedeutungen besitzen,

welches man in Gegenwart von tert.-Butylammoniumcyanid in Dimethylformamid in das Pyrrolidin der Formel (V) umwandelt:

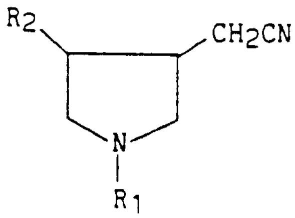

(V)

in der $R_1$ und $R_2$ die bezüglich der Formel (I) angegebenen Bedeutungen besitzen.

- oder mit p-Toluolsulfonylchlorid in Pyridin umsetzt zur Bildung des Pyrrolidins der Formel (VI):

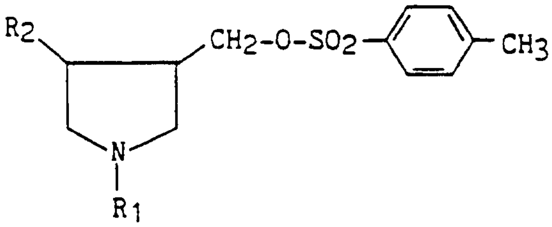

(VI)

in der $R_1$ und $R_2$ die bezüglich der Formel (I) angegebenen Bedeutungen besitzen,

welches man in Gegenwart von Kaliumcyanid in Dimethylsulfoxid in das oben beschriebene Pyrrolidin der Formel (V) umwandelt,
welches Derivat der Formel (V) man mit Diisobutylaluminiumhydrid reduziert zur Bildung des Aldehyds der Formel (VII):

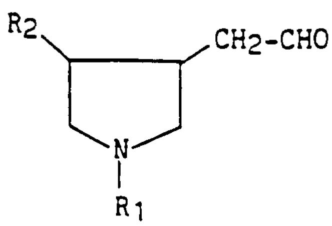

(VII)

in der $R_1$ und $R_2$ die bezüglich der Formel (I) angegebenen Bedeutungen besitzen,
**2 oder** mit Diisobutylaluminiumhydrid umsetzt zur Bildung des Aldehyds der Formel (VIII):

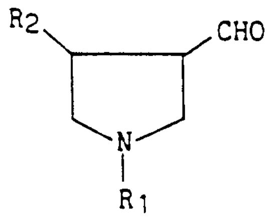

(VIII)

in der $R_1$ und $R_2$ die bezüglich der Formel (I) angegebenen Bedeutungen besitzen, welchen man gewünschtenfalls mit dem Phosphorylid der Formel (IX)

$$(C_6H_5)_3P = CHOCH_3 \qquad (IX)$$

das man durch Reaktion des entsprechenden Phosphoniumsalzes hergestellt hat, in Gegenwart von Kalium-tert.-butanolat in Tetrahydrofuran umsetzt zur Bildung des Verbindung der Formel (X):

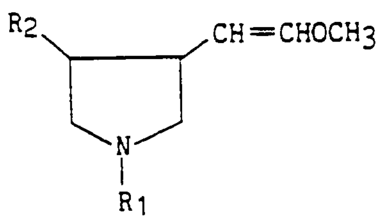

(X)

in der $R_1$ und $R_2$ die bezüglich der Formel (I) angegebenen Bedeutungen besitzen, welche man mit Trifluoressigsäure behandelt zur Bildung des oben beschriebenen Aldehyds der Formel (VII),
welche Verbindungen der Formel (VII) oder (VIII) man mit einem Phosphorylid der Formel (XI)

$$(C_6H_5)_3P = CH-(CH_2)_m-CO_2H \qquad (XI)$$

in der m die bezüglich der Formel (I) angegebenen Bedeutungen besitzt, das man durch Reaktion des entsprechenden Phosphoniumsalzes hergestellt hat, in Gegenwart von Kalium-tert.-butanolat in Tetrahydrofuran umsetzt, so daß man die Verbindungen der Formel (I/a) bzw. (I/b) erhält, einem Sonderfall der Verbindungen der Formel (I):

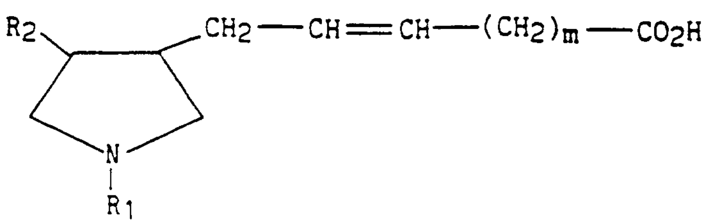

(I/a)

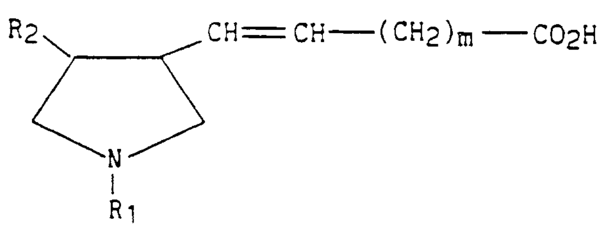

(I/b)

worin $R_1$, $R_2$ und m die bezüglich der Formel (I) angegebenen Bedeutungen besitzen,
welche Verbindungen der Formeln (I/a) oder (I/b)

- man gewünschtenfalls verestert zur Bildung der entsprechenden Verbindungen der Formel ($I/a_1$) und ($I/b_1$):

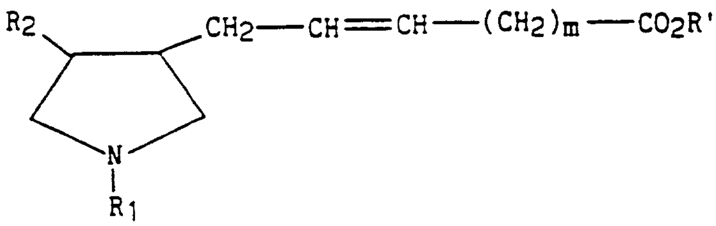

($I/a_1$)

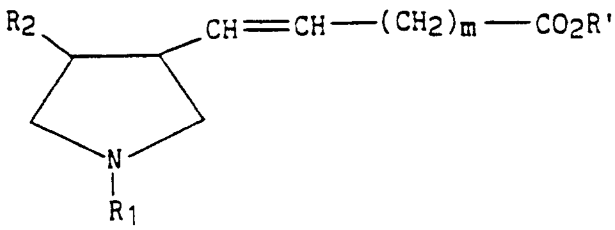

($I/b_1$)

worin $R_1$ und $R_2$ die bezüglich der Formel (I) angegebenen Bedeutungen besitzen und R' eine geradkettige oder verzweigte ($C_1$-$C_6$)-Alkylgruppe darstellt,

- bei denen man gegebenenfalls die Doppelbindung durch katalytische Hydrierung reduziert zur Bildung der Verbindung der Formel (I/c), einem Sonderfall der Verbindungen der Formel (I):

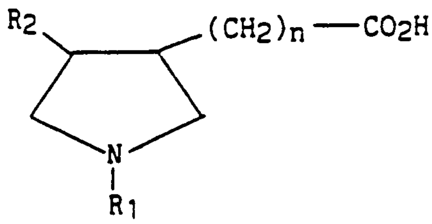

(I/c)

In der $R_1$, $R_2$ und n die bezuglich der Formel (I) angegebenen Bedeutungen besitzen, welche man gewünschtenfalls in den entsprechenden Ester der Formel ($I/c_1$) umwandelt:

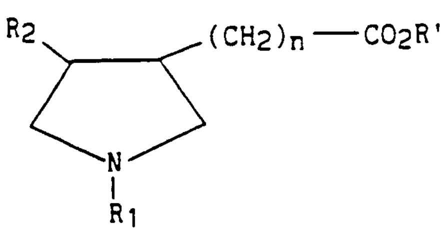

($I/c_1$)

in der $R_1$ und $R_2$ die bezüglich der Formel (I) angegebenen Bedeutungen besitzen und R' eine geradkettige oder verzweigte ($C_1$-$C_6$)-Alkylgruppe darstellt,
welche Verbindungen der Formeln (I/a), ($I/a_1$), (I/b), ($I/b_1$) (I/c) und ($I/c_1$) gemeinsam die Verbindungen der Formel (I) bilden,

- welche, wenn $R_2$ einen Phenylkern bedeutet, der durch eine geradkettige oder verzweigte ($C_1$-$C_6$)-Alkoxygruppe substituiert ist, gewünschtenfalls in die Verbindungen der Formel (I) umgewandelt werden können, in denen $R_2$ einen durch eine Hydroxylgruppe substituierten Phenylkern darstellt,
- welche man gegebenenfalls mit Hilfe einer klassischen Reinigungsmethode reinigt,
- welche man gegebenenfalls mit Hilfe einer klassischen Reinigungsmethode in ihre Isomeren auftrennt und
- welche man gewünschtenfalls mit einer pharmazeutisch annehmbaren Säure oder Base in ihre Additionssalze umwandelt.

2. Verfahren zur Herstellung der Verbindungen der Formel (I) nach Anspruch 1, in der $R_1$ eine gegebenenfalls substituierte Phenylsulfonylgruppe darstellt, von deren Enantiomeren, Diastereoisomeren und Epimeren sowie von deren Additionssalzen mit einer pharmazeutisch annehmbaren Säure oder Base.

3. Verfahren zur Herstellung der Verbindungen der Formel (I) nach Anspruch 1, worin $R_2$ eine Hydroxyphenylgruppe darstellt, von deren Enantiomeren, Diastereoisomeren und Epimeren sowie von deren Additionssalzen mit einer pharmazeutisch annehmbaren Säure oder Base.

4. Verfahren zur Herstellung der Verbindungen der Formel (I) nach Anspruch 1, in der $R_1$ eine Naphthylsulfonylgruppe darstellt, von deren Enantiomeren, Diastereoisomeren und Epimeren sowie von deren Additionssalzen mit einer pharmazeutisch annehmbaren Säure oder Base.

5. Verfahren zur Herstellung der Verbindungen der Formel (I) nach Anspruch 1, worin $R_1$ eine Isochinolylsulfonylgruppe darstellt, von deren Enantiomeren, Diastereoisomeren und Epimeren sowie von deren Additionssalzen mit einer pharmazeutisch annehmbaren Säure oder Base.

6. Verfahren zur Herstellung der Verbindung der Formel (I) nach Anspruch 1, nämlich (4Z)-6-[(3α,4α)-1-(4-Chlorphenylsulfonyl)-4-(2-hydroxyphenyl)-pyrrolidin-3-yl]-hex-4-ensäure, sowie von deren Additionssalzen mit einer pharmazeutisch annehmbaren Base.

7. Verfahren zur Herstellung der Verbindung der Formel (I) nach Anspruch 1, nämlich (4Z)-6-[(3α,4α)-1-[(Isochinol-5-yl)-sulfonyl]-4-(2-hydroxyphenyl)-pyrrolidin-3-yl]-hex-4-ensäure, sowie von deren Additionssalzen mit einer pharmazeutisch annehmbaren Base.

8. Verfahren zur Herstellung der Verbindung der Formel (I) nach Anspruch 1, nämlich (4Z)-6-[(3α,4α)-1-(Naphth-1-yl-sulfonyl)-4-(2-hydroxyphenyl)-pyrrolidin-3-yl]-hex-4-ensäure, sowie von deren Additionssalzen mit einer pharmazeutisch annehmbaren Base.

9. Verfahren zur Herstellung der Verbindungen der Formel (I'), einem Sonderfalls der Verbindungen der Formel (I) nach Anspruch 1:

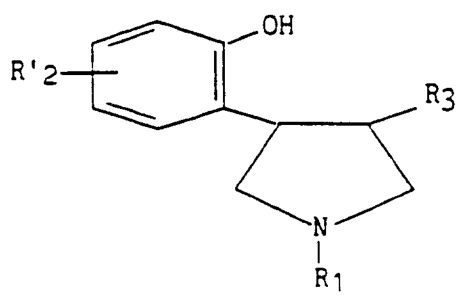

(I')

in der $R_1$ und $R_3$ die bezüglich der Formel (I) angegebenen Bedeutungen besitzen und $R'_2$ ein Wasserstoffatom, ein Halogenatom, eine geradkettige oder verzweigte ($C_1$-$C_6$)-Alkylgruppe, eine geradkettige oder verzweigte ($C_1$-$C_6$)-Alkoxygruppe oder eine Trihalogenmethylgruppe bedeutet, welche man ebenfalls unter Anwendung eines Verfahrens erhält, das **dadurch gekennzeichnet** ist, daß man als Ausgangsprodukt die Verbindung der Formel (XII) verwendet:

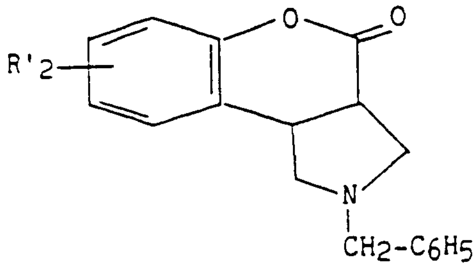

(XII)

in der $R'_2$ die bezüglich der Formel (I') angegebenen Bedeutungen besitzt, welche man mit Diisobutylaluminiumhydrid reduziert zur Bildung der Verbindung der Formel (XIII):

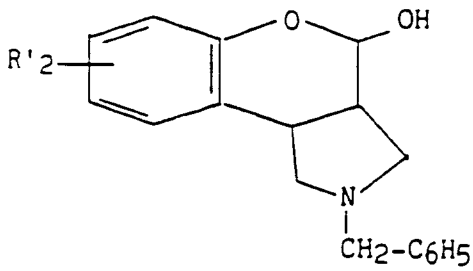

(XIII)

in der $R'_2$ die oben angegebenen Bedeutungen besitzt, welche man:

**a entweder:**

- entweder durch katalytische Hydrierung (Pd/C) in Dioxan in die Verbindung der Formel (XIV) umwandelt:

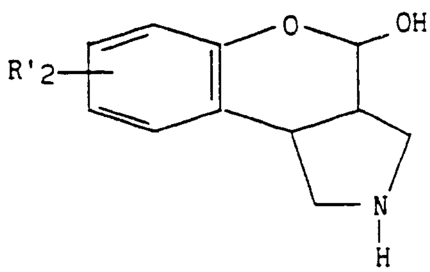

(XIV)

welche man mit einem Halogenderivat der Formel $R_1X$, In der $R_1$ die bezüglich der Formel (I) angegebenen Bedeutungen besitzt und X ein Halogenatom darstellt, in Chloroform in Gegenwart von Triethylamin behandelt, zur Bildung der Verbindung der Formel (XV):

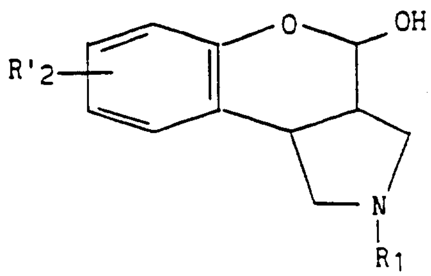

(XV)

in der $R'_2$ und $R_1$ die oben angegebenen Bedeutungen besitzen,

- oder durch katalytische Hydrierung (Pd/C) in einem Chlorwasserstoffsäure/Ethanol-Medium in die Verbindung der Formel (XVI) umwandelt:

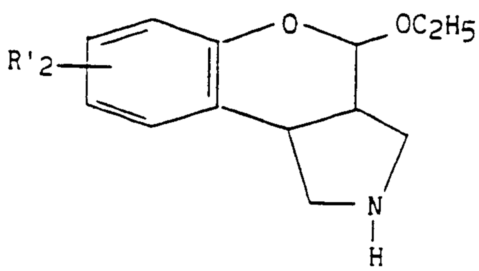

(XVI)

in der $R'_2$ die oben angegebenen Bedeutungen besitzt,
welche man mit einem Halogenderivat der oben beschriebenen Formel $R_1X$ behandelt zur Bildung der Verbindung der Formel (XVII):

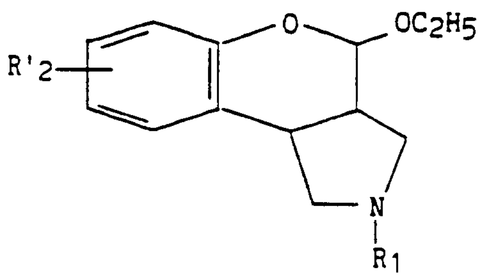

(XVII)

in der $R'_2$ und $R_1$ die oben angegebenen Bedeutungen besitzen,
welche man mit Trifluoressigsäure behandelt zur Bildung der oben beschriebenen Verbindung der Formel (XV),
welches Derivat der Formel (XV) man anschließend mit dem durch Reaktion des entsprechenden Phosphoniumsalzes gebildeten Phosphorylid der Formel (IX):

$$(C_6H_5)_3P = CHOCH_3 \qquad (IX)$$

in Gegenwart von Kalium-tert.-butanolat in Tetrahydrofuran umsetzt zur Bildung der Verbindung der Formel (XVIII):

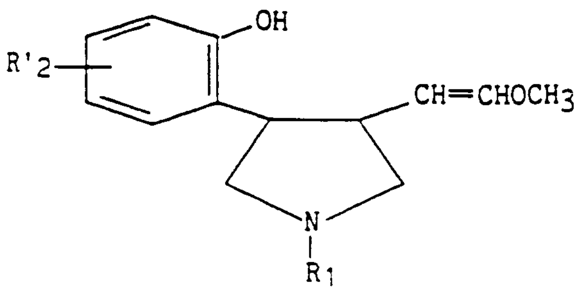

(XVIII)

in der $R_1$ und $R'_2$ die oben angegebenen Bedeutungen besitzen,
welche man gegebenenfalls mit Hilfe einer klassischen Trennungsmethode in ihre Diastereoisomeren auftrennt,
welche man mit Trifluoressigsäure behandelt zur Bildung des Aldehyds der Formel (XIX):

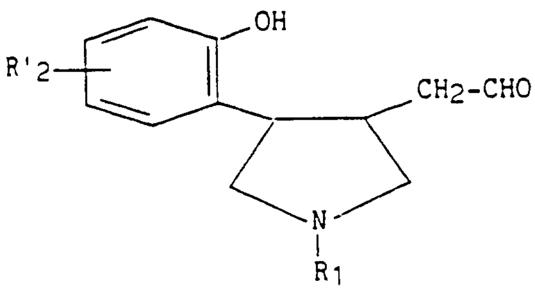

(XIX)

in der $R_1$ und $R'_2$ die oben angegebenen Bedeutungen besitzen,
welche man gegebenenfalls mit Hilfe einer klassischen Trennungsmethode in die Diastereoisomeren auftrennt,
**b oder** mit dem oben beschriebenen Phosphorylid der Formel (IX) umsetzt zur Bildung der Verbindung der Formel (XX):

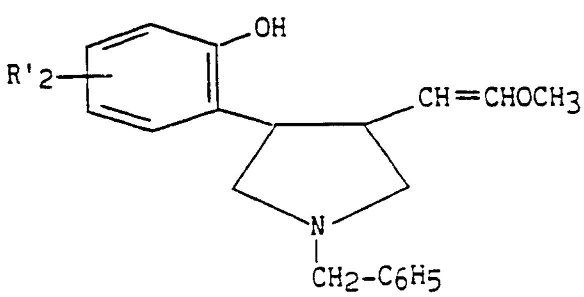

(XX)

in der $R'_2$ die oben angegebenen Bedeutungen besitzt,
welche man gegebenenfalls mit Hilfe einer klassischen Trennungsmethode in die Diastereoisomeren auftrennt,
und mit Trifluoressigsäure behandelt zur Bildung des Aldehyds der Formel (XIX/a), einem Sonderfall der Verbindungen der Formel (XIX):

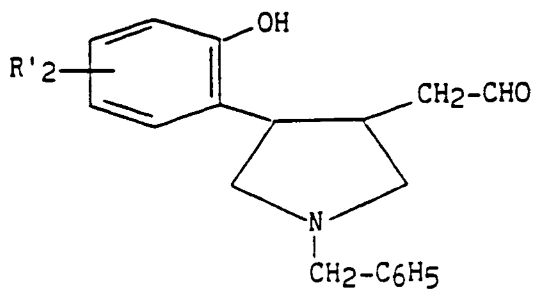

(XIX/a)

in der R'$_2$ die oben angegebenen Bedeutungen besitzt,
welchen man gegebenenfalls mit Hilfe einer klassischen Trennungsmethode in die Diastereoisomeren auftrennt,
welche Verbindungen der Formel (XIII) oder (XIX) man mit einem Phosphorylid der Formel (X):

$$(C_6H_5)_3P = CH\text{-}(CH_2)_m\text{-}CO_2H \qquad (X)$$

in der m die bezüglich der Formel (I) angegebenen Bedeutungen besitzt,
das durch Reaktion des entsprechenden Phosphoniumsalzes hergestellt wordenist, in Gegenwart von Kalium-tert.-butanolat in Tetrahydrofuran umsetzt, zur Bildung der Verbindungen der Formeln (I/d) bzw. (I/e), einem Sonderfall der Verbindungen der Formel (I):

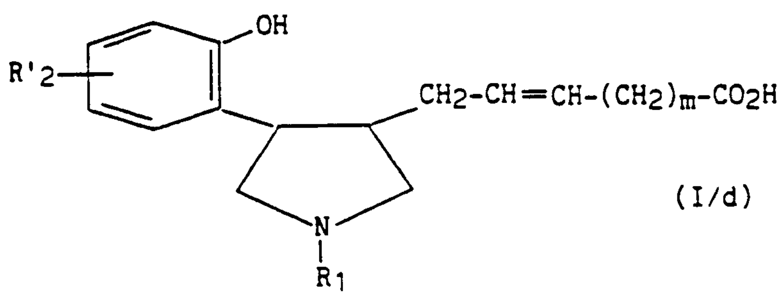

(I/d)

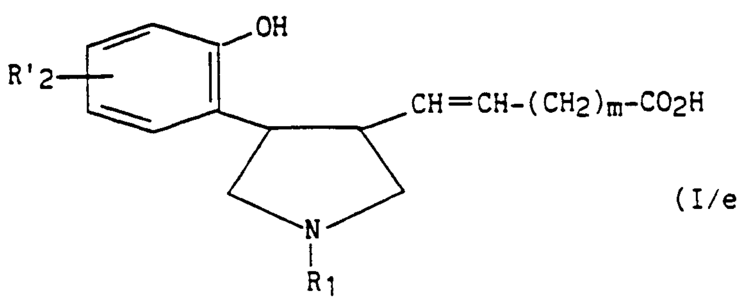

(I/e)

in denen R$_1$ und R'$_2$ die oben angegebenen Bedeutungen besitzen,

- welche man gewünschtenfalls verestert zur Bildung der entsprechenden Verbindungen der Formeln (I/d$_1$) und (I/e$_1$):

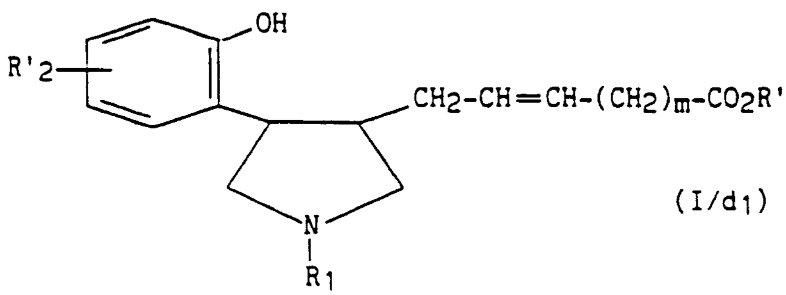

(I/d$_1$)

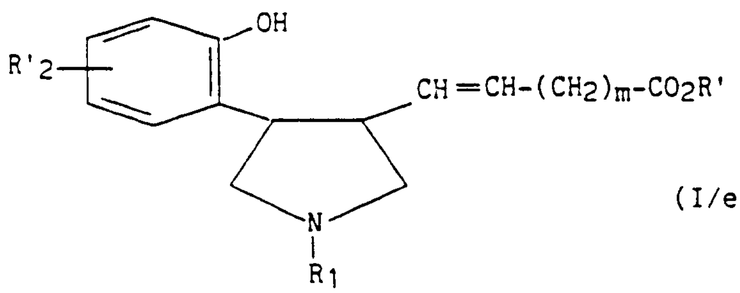

(I/e$_1$)

in denen $R_1$ und $R'_2$ die oben angegebenen Bedeutungen besitzen und R' eine geradkettige oder verzweigte ($C_1$-$C_6$)-Alkylgruppe darstellt,

- dessen Doppelbindung man gegebenenfalls durch katalytische Hydrierung reduziert zur Bildung der Verbindung der Formel (I/f), einem Sonderfall der Verbindungen der Formel (I):

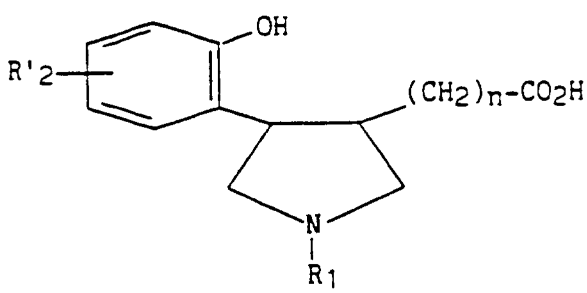

(I/f)

in der $R_1$ und $R'_2$ die oben angegebenen Bedeutungen besitzen,
welche man gewünschtenfalls in die entsprechenden Ester der Formel (I/f$_1$) umwandelt:

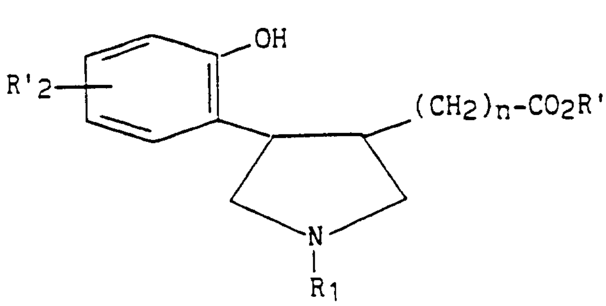

(I/f$_1$)

in der $R_1$, $R'_2$ und R' die oben angegebenen Bedeutungen besitzen,
welche Verbindungen der Formeln (I/d), (I/d$_1$), (I/e), (I/e$_1$), (I/f) und (I/f$_1$) gemeinsam die Verbindungen der Formel (I') darstellen,

- welche, wenn $R'_2$ eine geradkettige oder verzweigte ($C_1$-$C_6$)-Alkoxygruppe darstellt, gewünschtenfalls in die Verbindungen der Formel (I') umgewandelt werden können, in der $R'_2$ eine Hydroxylgruppe darstellt,
- welche man gegebenenfalls mit Hilfe einer klassischen Reinigungsmethode reinigt,
- welche man gegebenenfalls mit Hilfe einer klassischen Reinigungsmethode in die Isomeren auftrennt und
- welche man gewünschtenfalls mit einer pharmazeutisch annehmbaren Säure oder Base in ihre Additionssalze umwandelt.